# EUROPEAN PATENT SPECIFICATION

(11) **EP 1 692 139 B1**
(45) Date of publication and mention of the grant of the patent: **13.02.2013**
(21) Application number: 04811142.1
(22) Date of filing: 15.11.2004
(51) Int. Cl.: C07D 491/18

(54) **5,6 -DIHYDROXY-ISOINDOLE DERIVATIVES AS LINKERS FOR OLIGOMER SOLID PHASE SYNTHESIS**
5,6-DIHYDROXY-ISOINDOLDERIVATE ALS LINKER FÜR DIE FESTPHASENSYNTHESE VON OLIGOMEREN
DERIVES DE 5, 6-DIHYDROXY-ISOINDOLE EN TANT QUE LIEURS POUR LA SYNTHESE D'OLIGOMERES EN PHASE SOLIDE

(30) Priority: 13.11.2003 US 520179 P; 16.12.2003 US 530477 P; 21.04.2004 US 564649 P
(43) Date of publication of application: 23.08.2006
(60) Divisional application: 12199177.2
(73) Proprietor: Isis Pharmaceuticals, Inc., Carlsbad, CA 92010 (US)
(72) Inventor: VASULINGA, Ravikumar, Carlsbad, CA 92009 (US); ZHIWEI, Wang, Carlsbad, CA 92009 (US); Kumar, Raju Krishna., Carlsbad, CA 92009 (US)
(74) Representative: Hallybone, Huw George
(86) International application number: PCT/US2004/038325
(87) International publication number: WO 2005/049621

(56) References cited:
- WO-A-97/23497
- WO-A-03/062241
- JP-A- 7 096 677
- US-A1- 2004 152 905
- DATABASE CAPLUS CHEMICAL ABSTRACTS SERVICE, COLUMBUS, OHIO, US; XP002323601 retrieved from STN accession no. 1405 Database accession no. DN 138:137436 & LIU, FU-LONG ET ALL.: "Synthesis of 5,6-dehydronorcantharidin derivatives of substituted aromatic amine and their anti-tumor activities" YOUJI HUAXUE, vol. 22, no. 10, 2002, pages 761-767,
- NEUNG-JU LEE ET ALL.: "Synrhesis and biological activity of medium range molecular weight polymers containing exo-3,6-epoxy-1,2,3,6-tetrahydrophthalimid o-caproic acid" POLYMER INTERNATIONAL, vol. 50, 2001, pages 1010-1015, XP002323597
- HEATHER D. MAYNARD ET ALL.: "Synthesis of norbornenyl polymers with bioactive oligopeptides by ring-opening metathesis" MACROMOLECULES, vol. 33, 2000, pages 6239-6248, XP001050858
- DONG-YEOL LEE: "synthesis and biological activities of polymers containing exo-3,6-epoxy-tetrahydophthalic glycinylimide" JOURNAL OF APPLIED POLYMER SCIENCE, vol. 62, no. 3, 1996, pages 557-565, XP002323598
- STEFANO C.G. BIAGINI ET ALL.: "The synthesis of N-norbornenyl-amino acids and esters: monomers for the preparation of well defined polymers" TETRAHEDRON, vol. 51, no. 26, 1995, pages 7247-7262, XP002323599
- ANDREI P. GUZAEV AND MUTHIAH MANOHARAN: "A conformationally preorganized universal solid suppport for efficient oligonucleotide synthesis" J. AM. CHEM. SOC, vol. 125, no. 9, 5 March 2003 (2003-03-05), pages 2380-2381, XP002323600
- MAXIM ANTOPOLSKY, ELENA AZHAYEVA, UNNI TENGVALL AND ALEX AZHAYEV: "Towards a general method for the stepwise solid-phase synthesis of peptide-oligonucleotide conjugates" TETRAHEDRON LETTERS, vol. 43, 2002, pages 527-530, XP004329420

## Description

### FIELD OF THE INVENTION

The disclosure herein provides teaching of compounds, compositions and methods of use relating to oligomer synthesis. For example, the disclosure provides supports for synthesis of oligonucleotides and modified oligonucleotides, compositions comprising such supports and methods of using such supports in the synthesis of oligonucleotides.

### BACKGROUND OF THE INVENTION

Oligonucleotides have been used in various biological and biochemical applications. They have been used as primers and probes for the polymerase chain reaction (PCR), as antisense agents used in target validation, drug discovery and development, as ribozymes, as aptamers, and as general stimulators of the immune system. As the popularity of oligonucleotides has increased, the need for producing greater sized batches, and greater numbers of small-sized batches, has increased at pace. Additionally, there has been an increasing emphasis on reducing the costs of oligonucleotide synthesis, and on improving the purity and increasing the yield of oligonucleotide products.

A number of innovations have been introduced to the art of oligonucleotide synthesis. Amongst these innovations have been the development of excellent orthogonal protecting groups, activators, reagents, and synthetic conditions. The oligonucleotides themselves have been subject to a variety of modifications and improvements. Amongst these are chemistries that improve the affinity of an oligonucleotide for a specific target, that improve the stability of an oligonucleotide *in vivo,* that enhance the pharmacokinetic (PK) and toxicological (Tox) properties of an oligonucleotide, etc. These novel chemistries generally involve a chemical modification to one or more of the constituent parts of the oligonucleotide.

The term "oligonucleotide" thus embraces a class of compounds that include naturally-occurring, as well as modified, oligonucleotides. Both naturally-occurring and modified oligonucleotides have proven useful in a variety of settings, and both may be made by similar processes, with appropriate modifications made to account for the specific modifications adopted. A naturally occurring oligonucleotide, i.e. a short strand of DNA or RNA may be envisioned as being a member of the following generic formulas, denominated oligo-RNA and oligo-DNA, respectively, below: 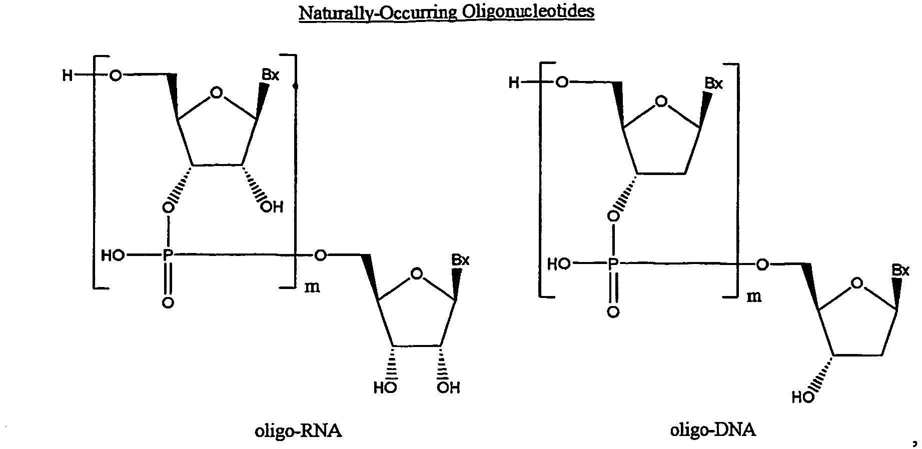 wherein m is an integer of from 1 to about 100, and Bx is one of the naturally occurring nucleobases.

Physiologic pH, an oligonucleotide occurs as the anion, as the phosphate easily dissociates at neutral pH, and an oligonucleotide will generally occur in solid phase, whether amorphous or crystalline, as a salt. Thus, unless otherwise modified, the term "oligonucleotide" encompasses each of the anionic, salt and free acid forms above.

In essence, a naturally occurring oligonucleotide may be thought of as being an oligomer of m monomeric subunits represented by the following nucleotides: 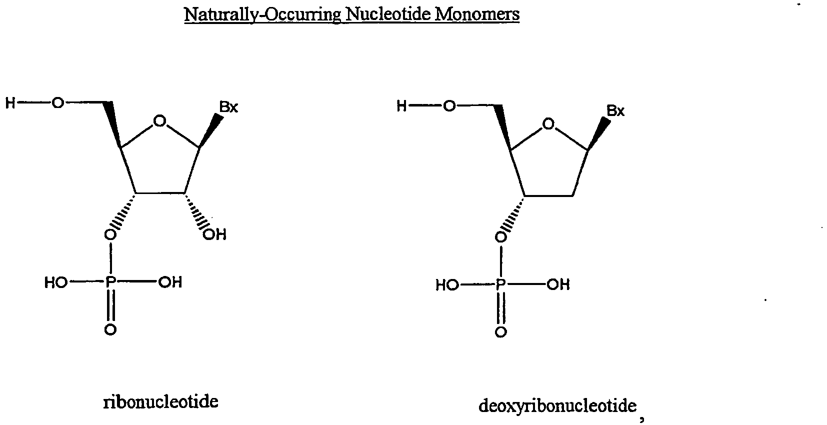 wherein each Bx is a nucleobase, wherein the last residue is a nucleoside (i.e. a nucleotide without the 3'-phosphate group).

As mentioned above, various chemistry modifications have been made to oligonucleotides, in order to improve their affinity, stability, PK, Tox, and other properties. In general, the term oligonucleotide, as now used in the art, encompasses *inter alia* compounds of the formula: 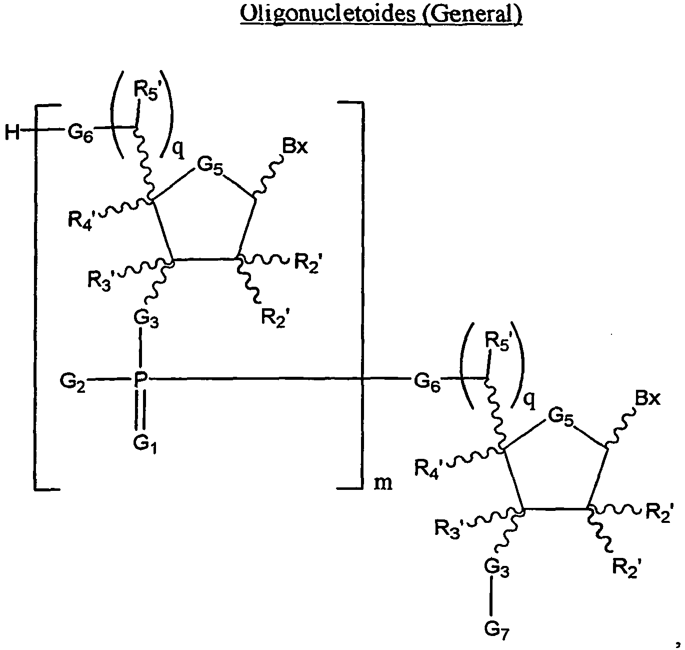 wherein m is an integer from 1 to about 100, each G₁ is O or S, each G₂ is OH or SH, each G₃ is O, S, CH₂, or NH, each G₅ is a divalent moiety such as O, S, CH₂, CFH, CF₂, -CH=H-, etc., each R₂' is H, OH, O-rg, wherein rg is a removable protecting group, a 2'-substituent, or together with R₄' forms a bridge, each R₃' is H, a substituent, or together with R₄' forms a bridge, each R₄' is H, a substituent, together with R₂' forms a bridge, together with R_{3'} forms a bridge, or together with R₅' forms a bridge, each q is 0 or 1, each R₅' is H, a substituent, or together with R₄' forms a bridge, each G₆ is O, S, CH₂ or NH, and each G₇ is H, PO₃H₂, or a conjugate group, and each Bx is a nucleobase, as described herein (i.e. naturally occurring or modified).

The standard synthetic methods for oligonucleotides include the solid phase methods first described by Caruthers et al. (See, for example, US Patent No. 5,750,666, incorporated herein by reference, especially columns 3-58, wherein starting materials and general methods of making oligonucleotides, and especially phosphorothioate oligonucleotides, are disclosed, which parts are specifically incorporated herein by reference.) These methods were later improved upon by Köster et al. (See, for example, US Patent No. RE 34,069, which is incorporated herein by reference, especially columns , wherein are disclosed, which parts are specifically incorporated herein by reference.) These methods have further been improved upon by various inventors, as discussed in more detail below. Methods of synthesizing RNA are disclosed in, *inter alia,* US Patent Nos. 6,111,086, 6,008,400, and 5,889,136, each of which is incorporated herein in its entirety. Especially relevant are columns 7-20 of US 6,008,400, which are expressly incorporated herein by reference.

The general process for manufacture of an oligonucleotide by the Köster et al. method may be described as follows:

First, a synthesis primer is prepared by covalently linking a suitable nucleoside to a solid support medium (SS) through a linker. Such a synthesis primer is as follows: 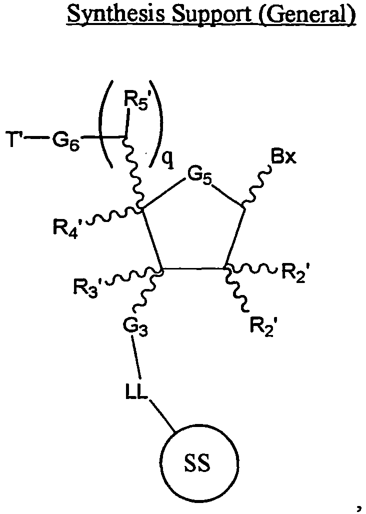 wherein SS is the solid support medium, LL is a linking group that links the nucleoside to the solid support medium via G₃. The linking group is generally a di-functional group, covalently binds the ultimate 3'-nucleoside (and thus the nascent oligonucleotide) to the solid support medium during synthesis, but which is cleaved under conditions orthogonal to the conditions under which the 5'-protecting group, and if applicable any 2'-protecting group, are removed. T' is a removable protecting group, and the remaining variables have already been defined, and are described in more detail herein. Suitable synthesis primers may be acquired from Amersham Biosciences under the brand name Primer Support 200™. The support medium having the synthesis primer bound thereto may then be swelled in a suitable solvent, e.g. acetonitrile, and introduced into a column of a suitable solid phase synthesis instrument, such as one of the synthesizers available form Amersham Biosciences, such as an AKTA oligopilot™, or OligoProcess™ brand DNA/RNA synthesizer.

Synthesis is carried out from 3'- to 5'-end of the oligomer. In each cycle, the following steps are carried out: (1) removal of T', (2) coupling, (3) oxidation, (4) capping. Each of the steps (1)-(4) may be, and generally is, followed by one or more wash steps, whereby a clean solvent is introduced to the column to wash soluble materials from the column, push reagents and/or activators through the column, or both. The steps (1)-(4) are depicted below:

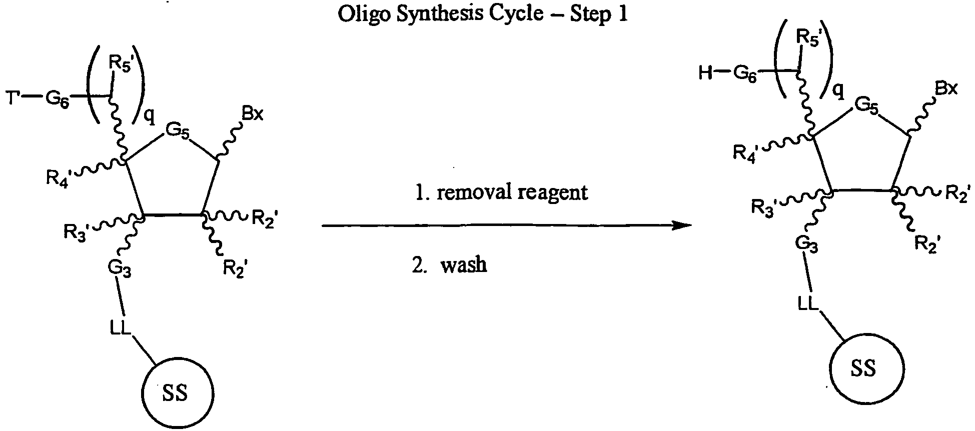

In general, T' is selected to be removable under conditions orthogonal to those used to cleave the oligonucleotide from the solid support medium at the end of synthesis, as well as those used to remove other protecting groups used during synthesis. An art-recognized protecting group for oligonucleotide synthesis is DMT (4,4'-dimethoxytrityl). The DMT group is especially useful as it is removable under weakly acid conditions. Thus, an acceptable removal reagent is 3% DCA in a suitable solvent, such as acetonitrile. The wash solvent, if used, may conveniently be acetonitrile.

The solid support medium may be controlled pore glass or a polymeric bead support medium. Some polymeric supports are disclosed in the following patents: US 6,016,895; US 6,043,353; US 5,391,667 and US 6,300,486, each of which is specifically incorporated herein by reference. 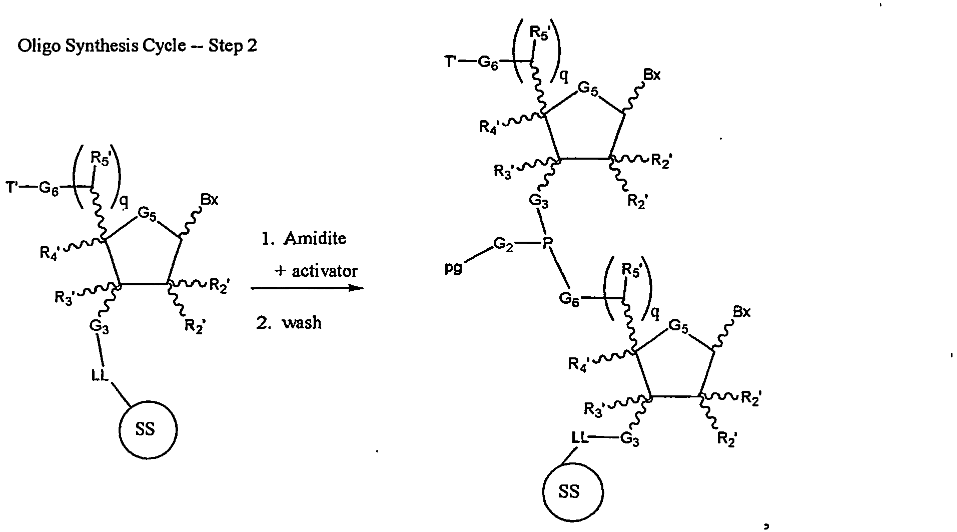 wherein pg is a phosphorus protecting group, such as a cyanoethyl group. See, Koster et al., supra, for information on manufacturing of the amidite: 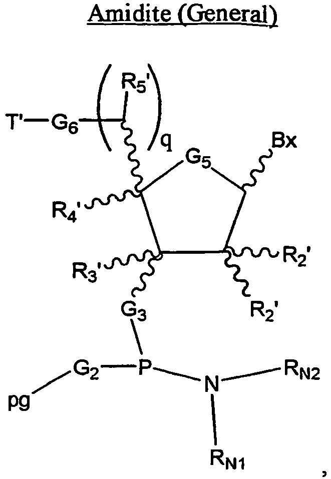 wherein NR_{N1}R_{N2} is an amine leaving group, such as diisopropyl amino, and for teaching of suitable activator (e.g. tetrazole). Other suitable amidites, and methods of manufacturing amidites, are set forth in the following patents: US 6,133,438; US 5,646,265; US 6,124,450; US 5,847,106; US 6,001,982; US 5,705,621; US 5,955,600; US 6,160,152; US 6,335,439; US 6,274,725; US 6,329,519, each of which is specifically incorporated herein by reference, especially as they relate to manufacture of amidites. Suitable activators are set forth in the Caruther et al. patent and in the Köster et al. patent. Especially suitable activators are set forth in the following patents: US 6,031,092 and US 6,476,216, each of which is expressly incorporated herein by reference.

The next step of the synthesis cycle is oxidation, which indicates that the P(III) species is oxidized to a P(V) oxidation state with a suitable oxidant: 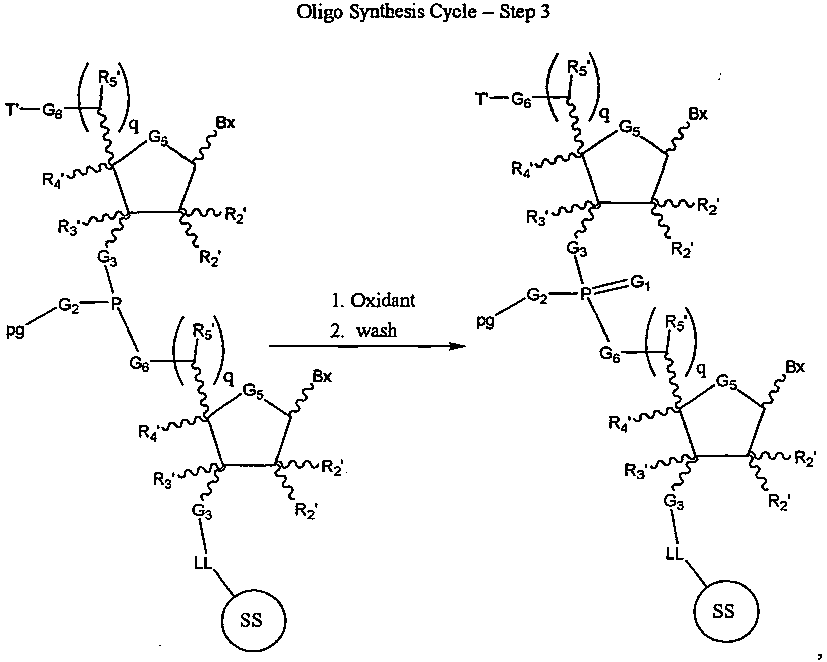 wherein G₁ is O or S.

The oxidant is an oxidizing agent suitable for introducing G₁. In the case where G₁ is oxygen, a suitable oxidant is set forth in the Caruthers et al. patent, above. In cases where G₂ is sulfur, the oxidant may also be referred to as a thiation agent or a sulfur-transfer reagent. Suitable thiation agents include the so-called Beaucage reagent, 3H-1,2-benzothiol, phenylacetyl disulfide (also referred to as PADS; see, for example the patents: US 6,114,519 and 6,242,591, each of which is incorporated herein by reference) and thiouram disulfides (e.g. N,N,N',N'-tetramethylthiouram disulfide, disclosed by US patent No. 5,166,387). The wash may be a suitable solvent, such as acetonitrile.

The oxidation step is followed by a capping step, which although not illustrated herein, is an important step for synthesis, as it causes free 5'-OH groups, which did not undergo coupling in step 1, to be blocked from being coupled in subsequent synthetic cycles. Suitable capping reagents are set forth in Caruthers et al., Köster et al., and other patents described herein. Suitable capping reagents include a combination of acetic anhydride and N-methylimidazole.

Synthetic cycle steps (1)-(4) are repeated (if so desired) n-1 times to produce a solid support-bound oligonucleotide: 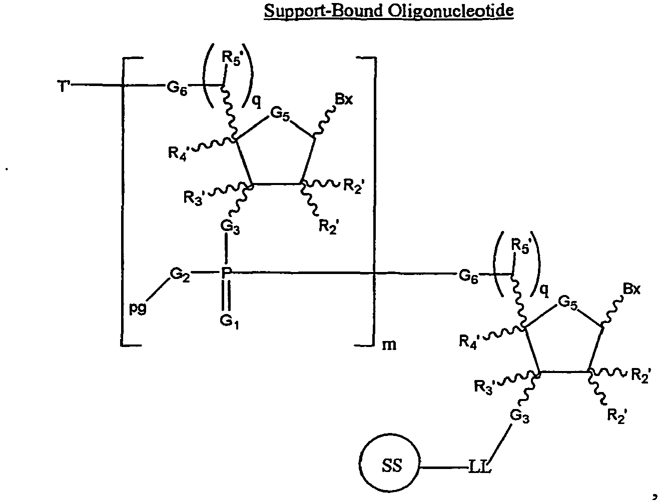 wherein each of the variables is as herein defined.

In general, the protecting group pg may be removed by a method as described by Caruthers et al. or Köster et al., supra. Where pg is a cyanoethyl group, the methodology of Köster et al., e.g. reaction with a basic solution, is generally suitable for removal of the phosphorus protecting group. In some cases it is desirable to avoid formation of adducts such as the N1-cyanoethyl thymidine group. In these cases, it is desirable to include in the reagent a tertiary amine, such as triethylamine (TEA) as taught in US Patent No. US 6,465,628, which is expressly incorporated herein by reference. In general, where the nucleobases are protected, they are deprotected under basic conditions. The deprotected oligonucleotides is cleaved from the solid support medium to give the following 5'-protested oligonucleotide: 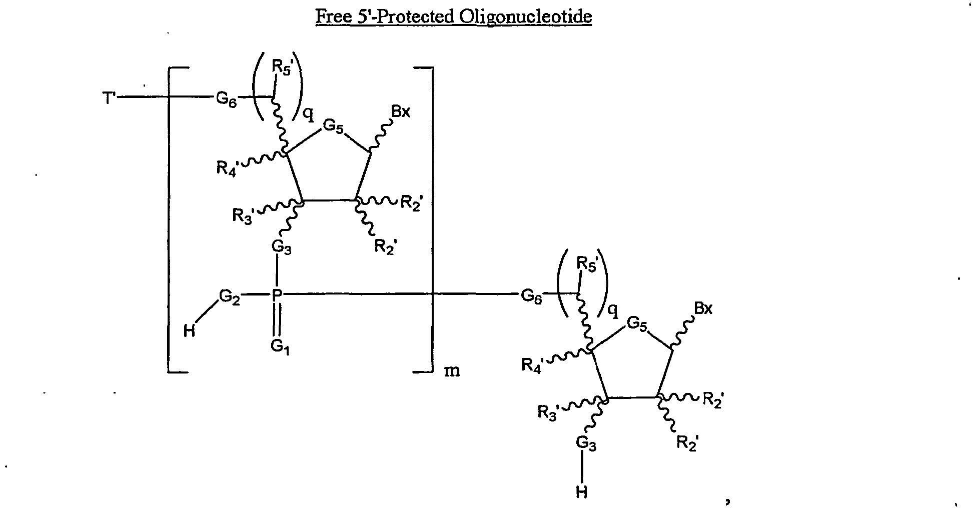 which may then be purified by reverse phase liquid chromatography, deprotected at the 5'-end in acetic acid, desalted, lyophilized or otherwise dried, and stored in an inert atmosphere until needed. Optionally, the G₃H group may be derivatized with a conjugate group. The resulting oligonucleotide may be visualized as having the formula:

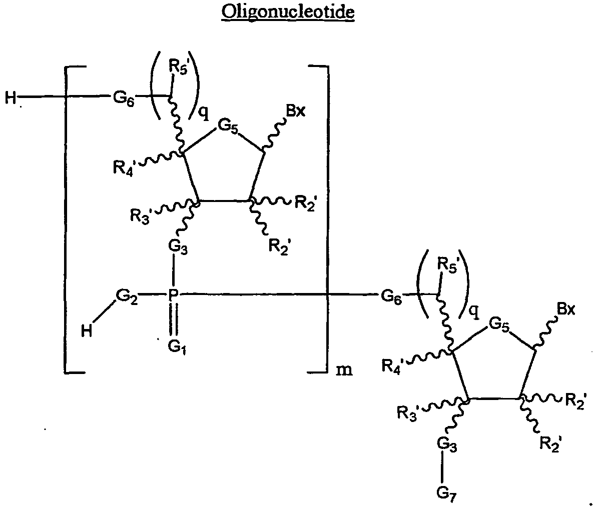

While many improvements have been made in the quality and costs of oligonucleotide synthesis, there still remain a number of improvements to be made. For example, impurities often arise in the synthesis of oligonucleotides. While the quantities of these impurities are generally small, it is desirable, where possible, to eliminate even trace amounts of impurities, especially when the oligonucleotides are intended for pharmaceutical use, including pharmaceutical testing and therapeutic use.

Standard methods of preparing succinyl-linked solid synthesis supports require relatively complex processes that are protected as proprietary knowledge by vendors of synthetic supports. The logistics of ordering and supply dictate that synthesis supports must generally be ordered months in advance of the time when they will be used, and may sit unused for days, weeks or even months after they are synthesized but before they are used. It has been discovered that certain synthesis supports can, on standing for periods of time, degrade, e.g. by losing protecting groups from protected nucleobases. It has been shown that loss of these protecting groups can give rise to high molecular weight species, e.g. branchmers, which occur when an oligonucleotide building block couples to an exocyclic OH or NH₂ of a nucleobase, thereby giving rise to a branched species that can itself be extended. For example, it has been shown that the standard benzoyl protecting group for 5-methyl-2'-*O*-methoxyethyl cytosine (5-MeMOE C) is relatively rapidly lost from solid support medium-bound 5-MeMOE C, thereby providing an exocyclic primary nitrogen as a potential branching point during the following synthesis.

There is thus a need for a synthesis support suitable for oligomer synthesis that could be used in conjunction with a variety of support media. There is further a need for a synthesis support that can be prepared without resorting to complex or proprietary methods for its synthesis. Advantageously, such a synthesis support would be capable of being coupled to a support medium on-site or on-demand, and/or that would not be subject to degradation, such as loss of exocyclic functional group protecting groups, prior to and/or during synthesis. It would also be desirable that such a synthesis support would be readily available from commercial sources.

### SUMMARY OF THE INVENTION

The present invention describes compounds of Formula I suitable for use as traceless linking moieties for solid phase chemical synthesis: 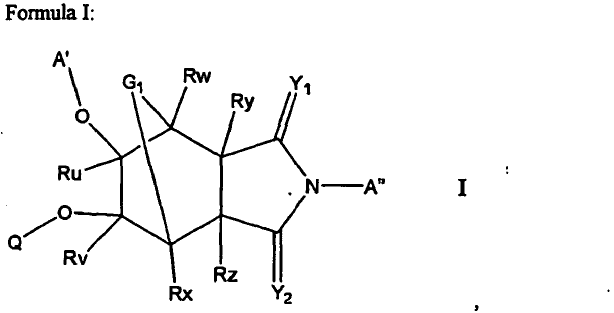 wherein one of A' and A" is SM or L-SM, wherein SM is a support medium, and L is a linking moiety, the other of A' and A" being H, a blocking group or a substituent; each of Ru, Rv, Rw, Rx, Ry and Rz is H, alkyl, substituted alkyl, alkenyl, substituted alkenyl, alkynyl or substituted alkynyl; Y₁ and Y₂ are each independently of one another O, S, NR₁ CH₂ or CR₁R₁, wherein R₁ is H, alkyl, substituted alkyl, alkenyl, substituted alkenyl, alkynyl or substituted alkynyl; G₁ is O, S or NR', wherein R' is H, a substituent or a blocking group; Q is H, a nucleosidyl moiety, a protected nucleosidyl moiety, a nucleosidyl moiety linked through a phosphorus linker (a phosphitidyl triester, phosphodiester, phosphorothioate diester, or phosphotriester moiety), a protected oligonucleotidyl or an oligonucleotidyl moiety, or Q is T, wherein T is a protecting group.

The present invention also provides processes for the synthesis of the compounds of Formula I and processes of linking said compounds of Formula I to a solid support media. Further embodiments provide methods of synthesis on solid supports, e.g. processes of making oligomers such as oligonucleotides (single stranded (ss) or double stranded (ds)), peptide nucleic acids (PNAs), peptides, combinatorial small molecule libraries, etc.

The compounds of the present invention may be used in the solid phase synthesis of oligomers, e.g. oligonucleotides, by solid phase synthetic methods. In some embodiments, the compounds evince unprecedented convenience as traceless linking moieties for solid phase synthesis using a variety of synthetic support media. In some embodiments, the compounds of the present evince facility of manufacture, as they may be made from commonly available starting materials, in high yields and/or in exceptional purity. In some embodiments, the compounds of the present invention provide exceptional performance as solid phase traceless linking moieties for oligonucleotide synthesis on solid support medium, as their use results in fewer side-products, especially so-called branchmers, which are commonly experienced with previously known linking moieties.

Compounds of formula (I) can be manufactured by reacting a compound of formula II with a compound of formula III:

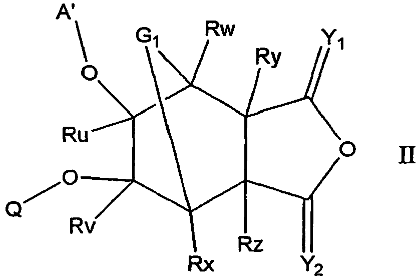

NH₂A" III,

wherein each of the variables is described above.

Compounds of formula II can be synthesized by Diels-Alder reaction from dienophile IV and diene V: 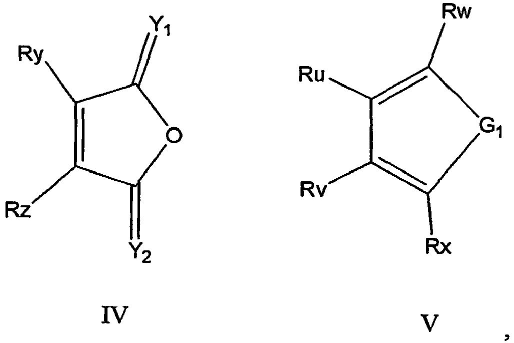 which together form intermediate A:

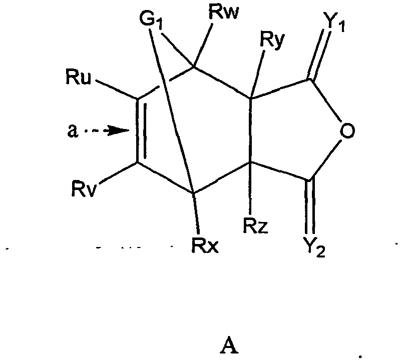

In each of IV, V and A, the variables are as described above.

Addition of hydroxyl groups across the double bond a results in intermediate B: 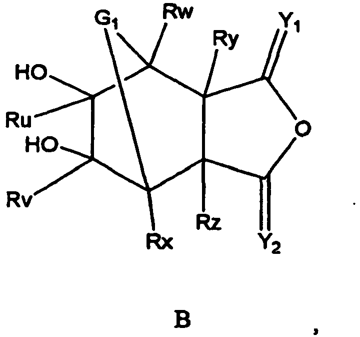 which may be derivatized to form a compound of formula II, above, as explained in more detail below. Of course, in formula B, the variables are as described above.

Compounds of the present invention provide facile linking of nascent chemical moieties, such as nucleosides, nucleotides, other oligomers and combinatorial chemicals, to a support medium such as a solid or semi-soluble support. Thus it is unnecessary to have a different species of linking moiety for commonly used nucleoside amidites, such as protected dA amidite, protected dG amidite, T amidite, protected dC amidite, etc. protected 5-methyl dC, protected 2'-O-methoxyethyl A amidite, protected 2'-O-methoxyethyl G amidites, protected 2'-O-methoxyethyl T amidite, protected 2'-O-methoxyethyl C amidite, protected 2'-O-methoxyethyl 5-methyl C amidite, protected 5-propynyl dC amidite, protected 5-propynyl dU amidite, (wherein, in each case, "protected" refers to protection of exocyclic amine with, e.g. isobutyryl, benzoyl), etc.

Other aspects and advantages of the invention will become apparent to the person skilled in the art upon consideration of the specification and claims.

### DETAILED DESCRIPTION OF THE INVENTION

The present invention is described in the appended claims

In some embodiments, the invention provides compounds of the formula I: 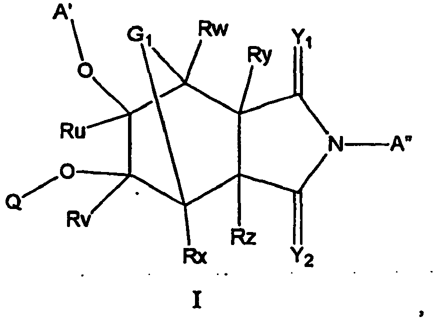 wherein one of A' and A" is SM or L-SM, wherein SM is a support medium, and L is a linking moiety, the other of A' and A" being H or a blocking group; each of Ru, Rv, Rw, Rx, Ry and Rz is H, alkyl, substituted alkyl, alkenyl, substituted alkenyl, alkynyl or substituted alkynyl; Y₁ and Y₂ are each independently of one another O, S, NR₁ CH₂ or CR₁R₁, wherein 12 R₁ is H, alkyl, substituted alkyl, alkenyl, substituted alkenyl, alkynyl or substituted alkynyl; G₁ is O, S or NR', wherein R' is H or a blocking group; T is H, a labile group, a nucleosidyl moiety, a protected nucleosidyl moiety, a nucleosidyl moiety linked through a phosphorus linker (e.g. phosphitidyl triester, phosphodiester, phosphorothioate diester, or phosphotriester moiety), a protected oligonucleotidyl or an oligonucleotidyl moiety.

In some preferred embodiments, compounds of formula I are support-bound linkers of formula I(a): , 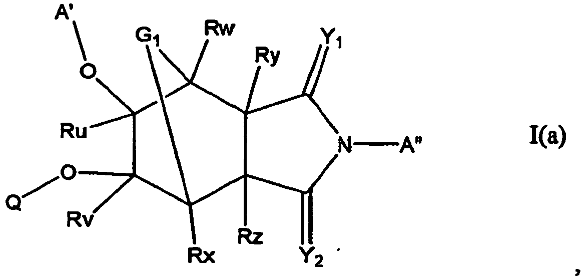 wherein one of A' and A" is SM or L-SM, wherein SM is a support medium, and L is a linking moiety, the other of A' and A" being H or a blocking group; each of Ru, Rv, Rw, Rx, Ry and Rz is H, alkyl, substituted alkyl, alkenyl, substituted alkenyl, alkynyl or substituted alkynyl; Y₁ and Y₂ are each independently of one another O, S, NR₁ CH₂ or CR₁R₁, wherein R₁ is H, alkyl, substituted alkyl, alkenyl, substituted alkenyl, alkynyl or substituted alkynyl; G₁ is O, S or NR', wherein R' is H or a blocking group; T is H, a labile group, a nucleosidyl moiety, a protected nucleosidyl moiety, a nucleosidyl moiety linked through a phosphorus linker (e.g. phosphitidyl triester, phosphodiester, phosphorothioate diester, or phosphotriester moiety), a protected oligonucleotidyl or an oligonucleotidyl moiety.

In some preferred embodiments of formula I(a), A' is SM or L-SM, wherein L and SM are defined above, and A" is bg, wherein bg is a blocking group as described in more detail herein. In particularly preferred embodiments, A' is L-SM.

In other preferred embodiments of formula I(a), A' is bg and A" is SM or L-SM, wherein bg, SM and L-SM are defined above.

In some preferred embodiments of formula I(a), T' is an acid-labile group. In particularly preferred embodiments of formula I(a), T is an acid labile group selected from optionally substituted triphenylmethyl or optionally substituted pixyl groups. In such compounds, the optional substituents are on the aryl groups of the respective triphenylmethyl or pixyl groups. Particularly preferred labile groups include MMT (4-methoxytriphenylmethyl), DMT (4,4'-dimethoxytriphenylmethyl), pixyl (9-phenylxanthenyl), or substituted pixyl (9-phenylxanthenyl having one or more substituents on the aryl rings of the xanthenyl moiety; suitable substituents being halogen, C₁-C₁₂ alkyl, preferably C₁-C₄ alkyl and C₁-C₁₂, preferably C₁-C₄ alkoxy). Selection of the appropriate labile group is possible based, *inter alia* upon the stability of the support medium (SM) and the linking group (L) to acid. In general, triphenylmethyl (trityl) and its derivatives (DMT and MMT being commonly referred to as "trityl groups") will require lower pH for removal than will 9-phenylxanthenyl and substituted variants of 9-phenylxanthenyl (referred to herein as "pixyl groups). The person skilled in the art should choose the labile group that is nearly fully removed under conditions that are compatible with the support medium and linker, taking into account other factors such as relative cost of labile group, cost of removal reagents, availability and disposability of reagents and solvents, etc. In general, pixyl groups are favored for their greater lability in relatively high pKa acids such as acetic acid, whereas trityl groups are favored for their relative abundance in the economy, but generally require lower pKa acids, such as chlorinated acetic acids (e.g. dichloroacetic acid (DCA)), which are less-favored due to ecological concerns, for their removal.

When T is a labile group, e.g. an acid-labile group, it may be removed to produce embodiments in which T is H. As such embodiments are generally produced in the process of manufacturing an oligonucleotide, which is a preferred use for compounds of formula I(a), such embodiments in which T is H, are also preferred.

When T is H, the free OH group formed by T and the O to which it is attached may be reacted with a species capable of linking an optionally protected nucleosidyl moiety to the O. Such species would include amidites, H-phosphonates and phosphotriesters, especially amidites and H-phosphonates.

In some preferred embodiments, G₁ is O, S or NH or N-bg, wherein bg is as defined above.

In some preferred embodiments, G₁ is O, S or N-bg. In particular preferred embodiments, G₁ is O or S, O being particularly preferred. In other particular preferred embodiments, G₁ is N-bg, wherein bg is either a non-labile group or a base-labile group. Specific non-labile groups that may be mentioned in this context are C₁-C₁₂ alkyl (preferably C₁-C₄ alkyl), substituted C₁-C₁₂ (preferably C₁-C₄) alkyl, phenyl, napthyl, anthracenyl, norbonyl, C₃-C₁₂ (preferably C₅-C₈) cycloalkyl, and substituted phenyl, napthyl, anthracenyl, norbonyl, C₃-C₁₂ (preferably C₅-C₈) cycloalkyl. Suitable substituents on the alkyl, phenyl, napthyl, anthracenyl, norbornyl and cycloalkyl groups are F, Cl, Br, I, NO₂, dialkylamino, alkoxy, alkylthio, etc. Additional suitable substituents on phenyl, napthyl, anthracenyl, norbornyl and cycloalkyl groups include at least one C₁-C₄ alkyl group.

In some preferred embodiments, Y₁ and Y₂ are independently O, S, CH₂ or C(alkyl)₂, wherein alkyl is C₁-C₁₂, preferably C₁-C₄ alkyl. In particularly preferred embodiments, Y₁ and Y₂ are independently O, S, C(CH₃)₂, CH(CH₃) or CH₂.

In particular preferred embodiments G₁ is O, Y, is O and Y₂ is O.

In other preferred embodiments G₁ is S, Y₁ is O and Y₂ is O.

In other preferred embodiments G₁ is O, Y₁ is S and Y₂ is S.

In other preferred embodiments, G₁ is S, Y₁ is S and Y₂ is S.

In some preferred embodiments, each of Ru, Rv, Rw, Rx, Ry, and Rz is independently selected from the group consisting of H and C₁-C₁₂ alkyl, preferably C₁-C₄ alkyl.

In some preferred embodiments, at least one of Ru, Rv, Rw, Rx, Ry, and Rz is a substituent. In other preferred embodiments, at least one pair of variables, Ru and Rv, Rx and Rw, or Ry and Rz, is a substituent. In particularly preferred embodiments, at least one pair of variables, Ru and Rv, Rx and Rw, or Ry and Rz, is a substituent selected from C₁-C₁₂, even more preferably C₁-C₄ alkyl. In other preferred embodiments, each or Ru, Rv, Rw, Rx, Ry, and Rz is H.

In some preferred embodiments, G₁, Y₁ and Y₂ are each O and each of Ru, Rv, Rw, Rx, Ry, and Rz are each independently H or C₁-C₄ alkyl. In particularly preferred embodiments, G₁, Y₁ and Y₂ are each O and Ru, Rv, Rw, Rx, Ry, and Rz are each H or methyl. In exemplary embodiments, G₁, Y₁ and Y₂ are each O and Ru, Rv, Rw, Rx, Ry, and Rz, are each H.

A suitable support medium may be any medium that facilitates separation of the compound of formula I(a) and derivatives thereof as described in detail herein, from a solution comprising reagent, reactant or wash solvent. In some cases, the support medium will be a solid phase support medium, such as a polymeric bead or controlled pore glass. In other cases, the support medium will be a semi-soluble support, which permits the compound of formula I(a) and derivatives thereof to be soluble in a solution having a certain characteristic polarity, but insoluble in solution having a different characteristic polarity. Suitable semi-soluble supports include polyethylene glycol polymer supports, which are generally soluble in less polar organic solutions, but which can be rendered insoluble upon addition of a more polar solvent, such as an alcohol (e.g. methanol or ethanol). Other suitable semi-solid supports include chitosan supports, which are similarly relatively soluble in less polar solutions. The person of ordinary skill in the art should recognize that it is necessary to choose higher molecular weight polymers (whether polyethyleneglycol (PEG), chitosan or some other semi-soluble support) to solubilize not only the compounds of formula I(a), but also derivatives thereof.

In some embodiments, there are provided compositions of formula I(b): 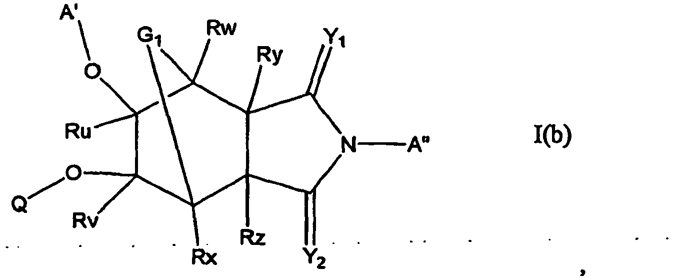 wherein one of A' and A" is SM or L-SM, wherein SM is a support medium, and L is a linking moiety, the other of A' and A" being H or a blocking group; each of Ru, Rv, Rw, Rx, Ry and Rz is H, alkyl, substituted alkyl, alkenyl, substituted alkenyl, alkynyl or substituted alkynyl; Y₁ and Y₂ are each independently of one another O, S, NR₁ CH₂ or CR₁R₁, wherein R₁ is H, alkyl, substituted alkyl, alkenyl, substituted alkenyl, alkynyl or substituted alkynyl; G₁ is O, S or NR', wherein R' is H or a blocking group; T is H, a labile group, a nucleosidyl moiety, a protected nucleosidyl moiety, a nucleosidyl moiety linked through a phosphorus linker (e.g. phosphitidyl triester, phosphodiester, phosphorothioate diester, or phosphotriester moiety), a protected oligonucleotidyl or an oligonucleotidyl moiety.

Some embodiments disclosed herein of the compound of formula I(b) are those in which each of A' and A" are H. Such compounds are useful as intermediates in the manufacture of support-bound linkers of formula I(a). Other embodiments disclosed herein of the compound of formula I(b) are those in which one of A' is H and A" is a blocking group, as described above. Such compounds are useful as intermediates in the manufacture of support-bound linkers of formula I(a). Other embodiments disclosed herein of the compound of formula I(b) are those in which A' is a blocking group, as described above, and A" is H_{L} Such compounds are useful as intermediates in the manufactured of support-bound linkers of formula I(a).

In some embodiments, there are provided synthesis supports of the formula: 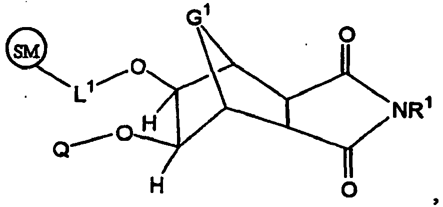 wherein SM is a support medium, e.g. a solid or a semi-soluble support medium, L¹ is a linking moiety G¹ is O, S, NR², wherein R² is other than H; Q is H or pg, wherein pg is a protecting group, and R¹ is a blocking group.

In particular embodiments, SM is a solid support, G¹ is O, and R¹ is an optionally further substituted alkyl, aryl, acyl, aralkyl, arylacyl, cycloalkyl (which may be partially dehydrogenated) heteroaryl or heteroarylacyl group. In some preferred embodiments, R¹ is optionally further substituted aryl, such as phenyl, naphthyl, or anthracenyl. In some preferred embodiments, R¹ is optionally substituted heteroaryl, such as pyridyl (e.g. pyrid-2-yl, pyrid-3-yl, pyrid-4-yl, or further substituted variants thereof), pyrimidyl (e.g. pyrimid-2-yl, pyrimid-4-yl, pyrimid-5-yl, or further substituted variants thereof), thiophenyl (e.g. thiophen-2-yl, thiophen-3-yl, or further substituted variants thereof), furanyl (furan-2-yl, furan-3-yl, or further substituted variants thereof), quinolinyl (e.g. quinolin-2-yl, quinolin-3-yl, quinolin-4-yl, quinolin-6-yl, quinolin-7-yl, or further substituted variants thereof). In some other preferred embodiments, R¹ is optionally substituted alkyl, such as methyl, ethyl, n-propyl, isopropyl, n-butyl, s-butyl, t-butyl, i-butyl, n-pentanyl, isooctanyl or dodecanyl, each of which is optionally substituted with one or more substituents. In some preferred embodiments, R¹ is optionally further substituted acyl, preferably C₁-C₁₂ acyl, and more preferably C₁-C₈ acyl, such as formyl, acetyl, propanoyl, butanoyl, octanoyl, etc.

Further substituents for groups defined above for R¹, when R¹ is alkyl, acyl, aryl, or heteroaryl, include F, Cl, Br, I, O-C₁-C₁₂ alkyl (e.g. O-methyl, O-ethyl and O-isopropyl), O-C₁-C₁₂ acyl, NO₂, aryl or heteroaryl. When R¹ is aryl or heteroaryl, the R¹ substituents further include alkyl and/or acyl.

In some embodiments of the invention, there are provided synthesis supports of the formula: 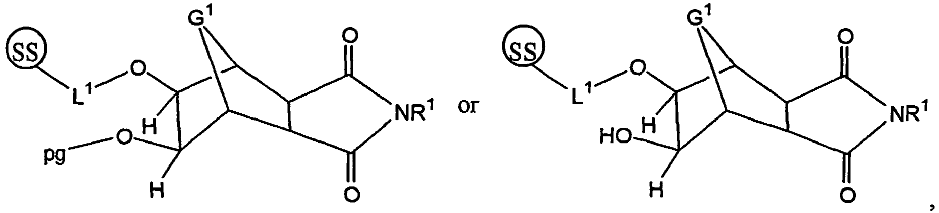 wherein SS is a solid support medium, L¹ is a linker, G¹ is O, S or NR², R² is a substituent other than H, pg is a protecting group and R¹ is a substituent other than H.

In some embodiments of the invention, there are provided synthesis supports of the formula: 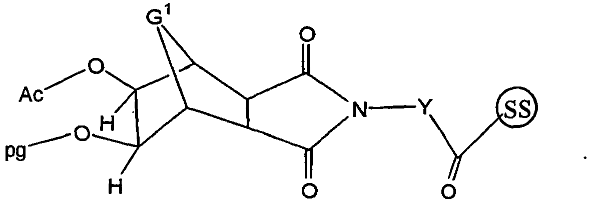 or 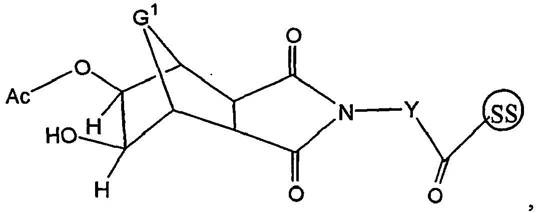 wherein SS is a solid support medium, Y is a divalent group, G¹ is O, S or NR², R² is a substituent other than H, and Ac and pg are mutually orthogonal protecting groups.

In some embodiments, there are provided compounds of the formula: 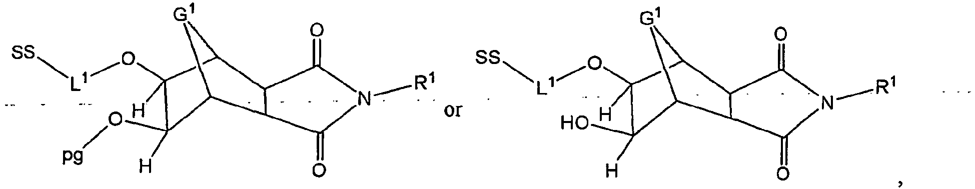 wherein SS is a solid support medium, L¹ is a divalent linker joining the solid support to O, pg is a protecting group, G¹ is O, S, NR², R² is a substituent other than H, and R¹ is a substituent other than H.

In other embodiments, there are provides compounds of the formula: 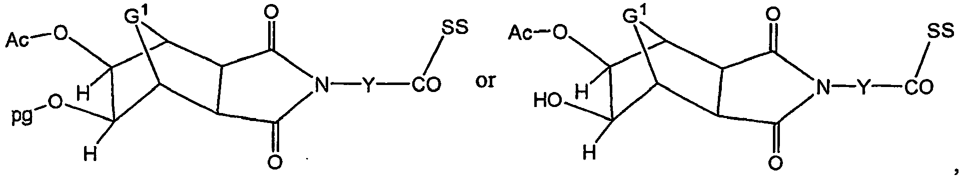 wherein SS is a solid support medium, Y is a divalent group, G¹ is O, S, NR², R² is a substituent other than H, Ac is an acetyl blocking group and pg is a protecting group. In some embodiments of the invention, the acetyl blocking group Ac and the protecting group pg are orthogonal to one another. In some embodiments, Ac is base-labile and pg is acid-labile.

In other embodiments, there are provided compounds of the formula: 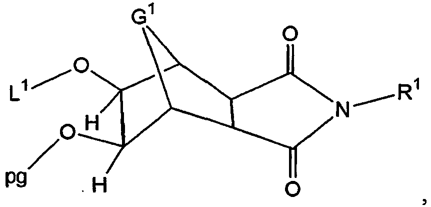 wherein the variables are defined herein, e.g. as above.

In other embodiments, there are provided compounds of the formula: 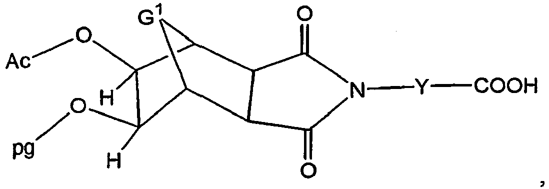 wherein the variables are defined herein, e.g. as above.

In other embodiments, there are provided compounds of the formula: 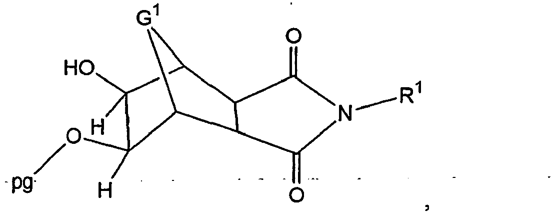 wherein the variables are defined herein, e.g. as above.

In other embodiments, there are provided compounds of the formula: 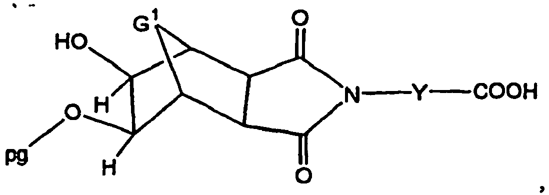 wherein the variables are defined herein, e.g. as above.

In other embodiments, there are provided compounds of the formula: 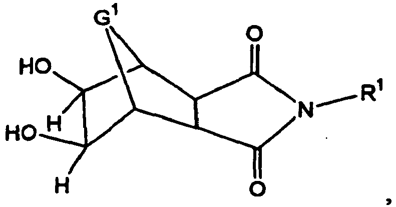 wherein the variables are defined herein, e.g. as above.

In other embodiments, there are provided compounds of the formula: 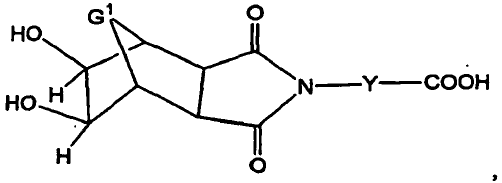 wherein the variables are defined herein, e.g. as above.

In further embodiments of the present disclosure are compounds of the formula: 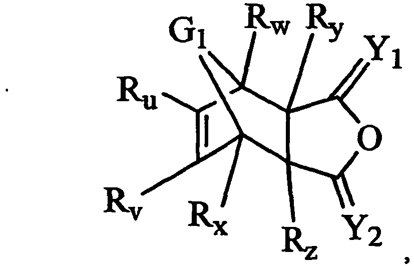 wherein G¹ is O, S or NR¹, R¹ is H or a substituent, each of Rᵤ, Rᵥ, R_{w}, Rₓ, R_{y} and R_{z} is independently H, C₁-C₁₂ alkyl, aryl, heteroalkyl, arylalkyl, Y₁ is O, S, CH₂, or CR_{y'}R_{y-}, wherein each of R_{y1'} and R_{y1}- is independently optionally substituted C₁-C₁₂ alkyl, Y₂ is O, S, CH₂, or CP_{y2}-R_{2"}, wherein each of R_{y2'} and R_{y2"} is optionally substituted C₁-C₁₂ alkyl.

In further embodiments of the present disclosure are compounds of the formula 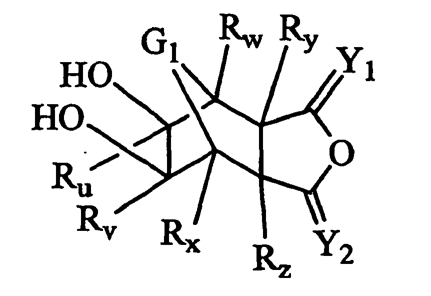 wherein G¹ is O, S or NR¹, R¹ is H or a substituent, each of Rᵤ, Rᵥ, R_{w}, Rₓ, R_{y} and R_{z} is independently H, C₁-C₁₂ alkyl, aryl, heteroalkyl, arylalkyl, Y₁ is O, S, CH₂, or CR_{y'}R_{y"}, wherein each of R_{y1'} and R_{y1"} is independently optionally substituted C₁-C₁₂ alkyle Y₂ is O, S, CH₂, or CR_{y2}·R_{2"}, wherein each of R_{y2'} and R_{y2"} is optionally substituted C₁-C₁₂ alkyl.

In further embodiments of the present disclosure are compounds of the formula 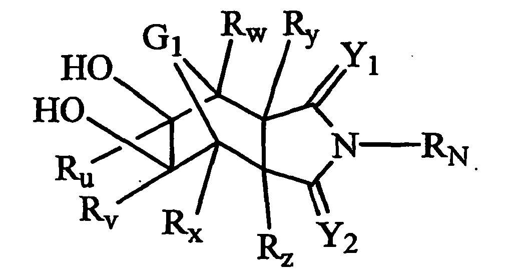 wherein G¹ is O, S or NR¹, R¹ is H or a substituent; each of Rᵤ, Rᵥ, R_{w}, Rₓ, R_{y} and R_{z} is independently H, C₁-C₁₂ alkyl, aryl, heteroalkyl, arylalkyl; Y₁ is O, S, CH₂, or CR_{y'}R_{y"}, wherein each of R_{y1'} and R_{y1"} is independently optionally substituted C₁-C₁₂ alkyl; Y₂ is O, S, CH₂, or CR_{y2'}R_{2"}, wherein each of R_{y2'} and R_{y2"} is optionally substituted C₁-C₁₂ alkyl; and R_{N} is H or a substituent.

In further embodiments of the present disclosure are compounds of the formula: 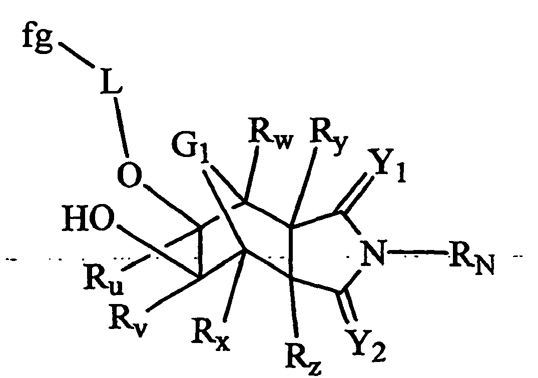 wherein G¹ is O, S or NR¹, R¹ is H or a substituent; each of Rᵤ, Rᵥ, R_{w}, Rₓ, R_{y} and R_{z} is independently H, C₁-C₁₂ alkyl, aryl, heteroalkyl, arylalkyl; Y₁ is O, S, CH₂, or CR_{y'}R_{y"}, wherein each of R_{y1'} and R_{y1"} is independently optionally substituted C₁-C₁₂ alkyl; Y₂ is 0, S, CH₂, or CR_{y2}R_{2"}, wherein each of R_{y2'} and R_{y2"} is optionally substituted C₁-C₁₂ alkyl; R_{N} is H or a substituent; and L is a linking group and fg is a functional group.

In further embodiments of the present invention are compounds of the formula 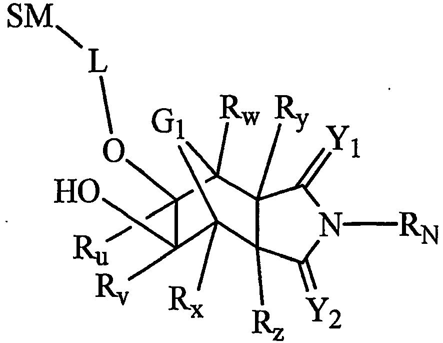 wherein G¹ is O, S or NR¹, R¹ is H or a substituent; each of Rᵤ, Rᵥ, R_{w}, Rₓ, R_{y} and R_{z} is independently H, C₁-C₁₂ alkyl, aryl, heteroalkyl, arylalkyl; Y₁ is 0, S, CH₂, or CR_{y'}R_{y"}, wherein each of R_{y1'} and R_{y1"} is independently optionally substituted C₁-C₁₂ alkyl; Y₂ is 0, S, CH₂, or CR_{y2'}R_{2"}, wherein each of R_{y2'} and R_{y2"} is optionally substituted C₁-C₁₂ alkyl; R_{N} is H or a substituent; L is a linking group and fg is a functional group; and SM is a support medium.

In further embodiments of the present invention are compounds of the formula: 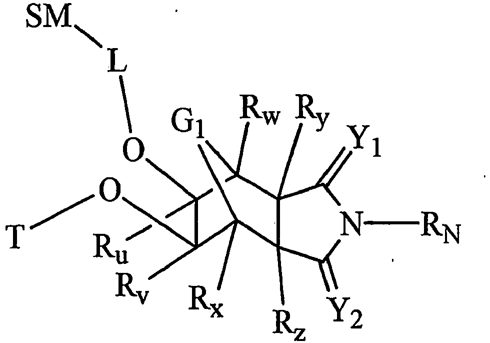 wherein G¹ is 0, S or NR¹, R¹ is H or a substituent; each of R_{u,} Rᵥ, R_{w}, Rₓ, R_{y} and R_{z} is independently H, C₁-C₁₂ alkyl, aryl, heteroalkyl, arylalkyl; Y₁ is O, S, CH₂, or CR_{y'}R_{y"}, wherein each of R_{y1'} and R_{y1"} is independently optionally substituted C₁-C₁₂ alkyl; Y₂ is 0, S, CH₂, or CR_{y2'}R_{2"}, wherein each of R_{y2'} and R_{y2"} is optionally substituted C₁-C₁₂ alkyl; R_{N} is H or a substituent; L is a linking group and fg is a functional group; T is a labile protecting group; SM is a support medium.

In further embodiments of the present invention are compounds of the formula: 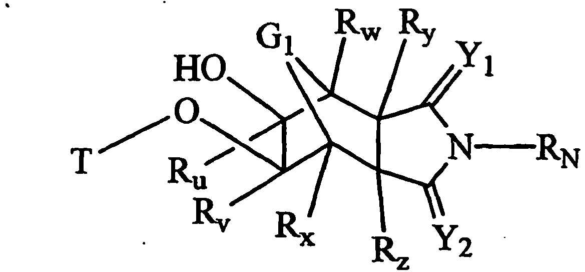 wherein G¹ is O, S or NR¹, R¹ is H or a substituent; each of Rₐ, Rᵥ, R_{w}, Rₓ, R_{y} and R_{z} is independently H, C₁-C₁₂ alkyl, aryl, heteroalkyl, arylalkyl; Y₁ is O, S, CH₂, or CR_{y'}Ry_{"}, wherein each of R_{y1'} and R_{y1"} is independently optionally substituted C₁-C₁₂ alkyl; Y₂ is O, S, CH₂, or CR_{y2'}R_{2"}, wherein each of R_{y2'} and R_{y2"} is optionally substituted C₁-C₁₂ alkyl; R_{N} is H or a substituent; and T is a labile protecting group.

In further embodiments of the present disclosure are compounds of the formula: 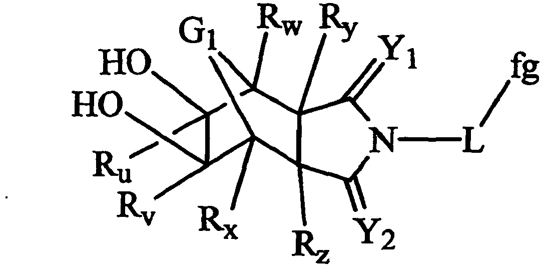 wherein G¹ is **O,** S or NR¹, R¹ is H or a substituent; each of Rᵤ, R_{v,} R_{w}, Rₓ R_{y} and R_{z} is independently H, C₁-C₁₂ alkyl, aryl, heteroalkyl, arylalkyl; Y₁ is **O,** S, CH₂, or CR_{y'}R_{y"}, wherein each of R_{y1'} and R_{y1"} is independently optionally substituted C₁-C₁₂ alkyl; Y₂ is O, S, CH₂, or CR_{y2'}R_{2"}, wherein each of R_{y2'} and R_{y2"} is optionally substituted C₁-C₁₂ alkyl; R_{N} is H or a substituent; L is a linking group; and fg is a functional group.

In further embodiments of the present disclosure are compounds of the formula: 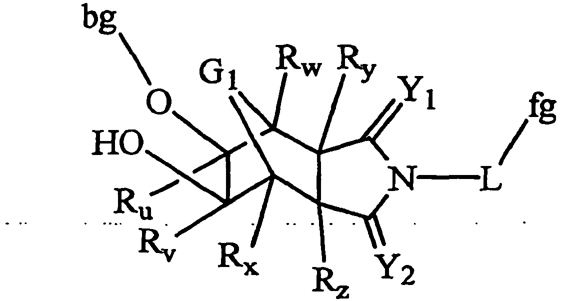 wherein G¹ is O, S or NR¹, R¹ is H or a substituent; each of Rᵤ, Rᵥ R_{w}, Rₓ R_{y} and R_{z} is independently H, C₁-C₁₂ alkyl, aryl, heteroalkyl, arylalkyl; Y₁ is O, S, CH₂, or CR_{y'}R_{y"}, wherein each of R_{y1'} and R_{y1"} is independently optionally substituted C₁-C₁₂ alkyl; Y₂ is **O,** S, CH₂, or CR_{y2'}R_{2"}, wherein each of R_{y2'} and R_{y2"} is optionally substituted C₁-C₁₂ alkyl; R_{N} is H or a substituent; L is a linking group; fg is a functional group; and bg is a blocking group.

In further embodiments of the present invention are compounds of the formula 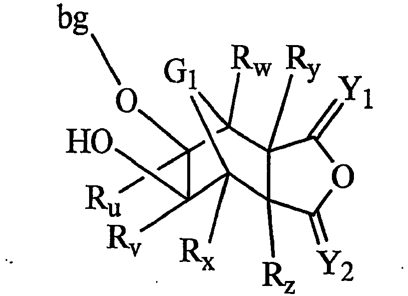 wherein G¹ is O, S or NR¹, R¹ is H or a substituent; each of Rᵤ, Rᵥ, R_{w}, Rₓ, R_{y} and R_{z} is independently H, C₁-C₁₂ alkyl aryl, heteroalkyl, arylalkyl; Y₁ is O, S, CH₂, or CR_{y'}R_{y"}, wherein each of R_{y1'} and R_{y1"} is independently optionally substituted C₁-C₁₂ alkyl; Y₂ is O, S, CH₂, or CR_{y2'}R_{2"}, wherein each of R_{y2'} and R_{y2"} is optionally substituted C₁-C₁₂ alkyl.

In further embodiments of the present invention are compounds of the formula 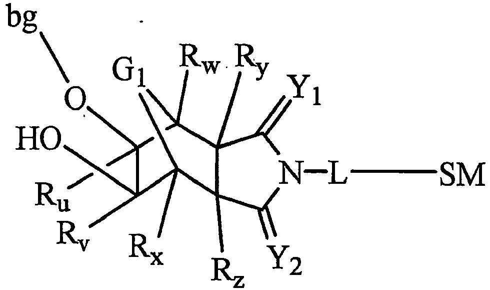 wherein G¹ is O, S or NR¹, R¹ is H or a substituent; each of Rᵤ, Rᵥ, R_{w}, Rₓ, R_{y} and R_{z} is independently H, C₁-C₁₂ alkyl, aryl, heteroalkyl, arylalkyl; Y₁ is O, S, CH₂, or CR_{y'}R_{y"}, wherein each of R_{y1'} and R_{y1"} is independently optionally substituted C₁-C₁₂ alkyl; Y₂ is O; S, CH₂, or CR_{y2'}R_{2"}, wherein each of R_{y2'} and R_{y2"} is optionally substituted C₁-C₁₂ alkyl; L is a linking group and fg is a functional group; bg is a blocking group; and SM is a support medium.

In further embodiments of the present invention are compounds of the formula 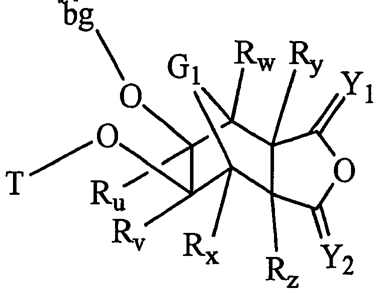 wherein G¹ is 0, S or NR¹, R¹ is H or a substituent; each of Rᵤ, Rᵥ, R_{w}, Rₓ, R_{y} and R_{z} is independently H, C₁-C₁₂ alkyl, aryl, heteroalkyl, arylalkyl; Y₁ is O, S, CH₂, or CR_{y'}R_{y"}, wherein each of R_{y1'} and R_{y1"} is independently optionally substituted C₁-C₁₂ alkyl; Y₂ is O, S, CH₂, or CR_{y2'}R_{2"}, wherein each of R_{y2'} and R_{y2"} is optionally substituted C₁-C₁₂ alkyl; and bg is a blocking group and T is a labile protecting group.

In further embodiments of the present invention are compounds of the formula 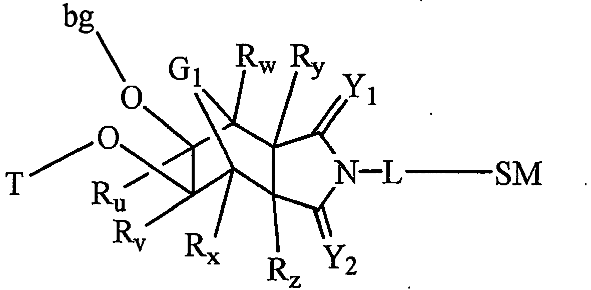 wherein G¹ is 0, S or NR¹, R¹ is H or a substituent; each of Rᵤ, Rᵥ, R_{w}, Rₓ, R_{y} and R_{z} is independently H, C₁-C₁₂ alkyl, aryl, heteroalkyl, arylalkyl; Y₁ is O, S, CH₂, or CR_{y'}R_{y"}, wherein each of R_{y1'} and R_{y1"} is independently optionally substituted C₁-C₁₂ alkyl; Y₂ is O, S, CH₂, or CR_{y2'}R_{2"}, wherein each of R_{y2'} and R_{y2"} is optionally substituted C₁-C₁₂ alkyl; R_{N} is H or a substituent; L is a linking group and fg is a functional group; bg is a blocking group; SM is a support medium; T is a labile protecting group.

In further embodiments of the present invention are compounds of the formula 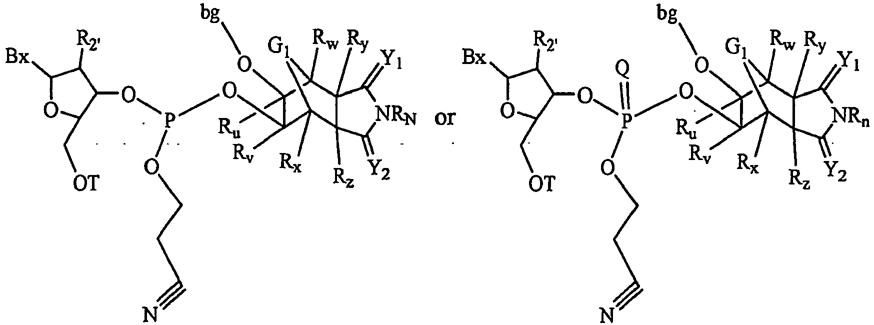 wherein G¹ is O, S or NR¹, R¹ is H or a substituent; each of Rᵤ, Rᵥ, R_{w}, Rₓ, R_{y} and R_{z} is independently H, C₁-C₁₂ alkyl, aryl, heteroalkyl, arylalkyl; Y₁ is O, S, CH₂, or CR_{y'}R_{y"}, wherein each of R_{y1'} and R_{y1"} is independently optionally substituted C₁-C₁₂ alkyl; Y₂ is O, S, CH₂, or CR_{y2'}R_{2"}, wherein each of R_{y2'} and R_{y2"} is optionally substituted C₁-C₁₂ alkyl; R_{N} is H or a substituent; L is a linking group and fg is a functional group; SM is a support medium; Bx is a nucleobase which is optionally substituted; T is a labile protecting group; and Q is O or S.

### PROCESSES OF MAKING INVENTIVE COMPOUNDS

Compounds of formula (I) can be manufactured by reacting a compound of formula II with a compound of formula III:

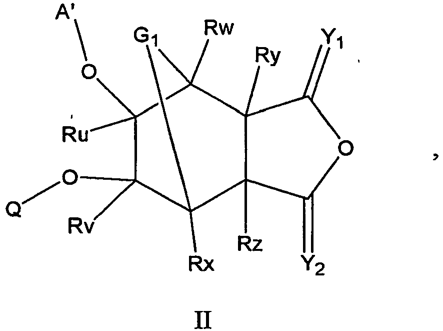

NH₂A" III

wherein each of the variables is described above.

Compounds of formula II can be synthesized by Diels-Alder reaction from dienophile IV and diene V: 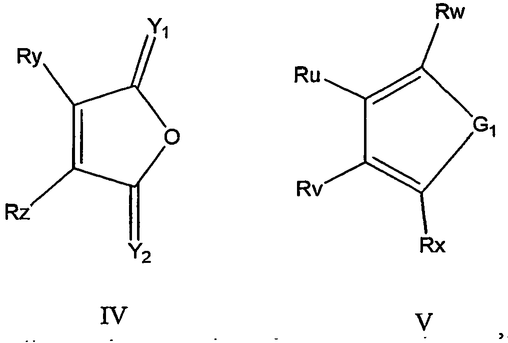 which together form intermediate A:

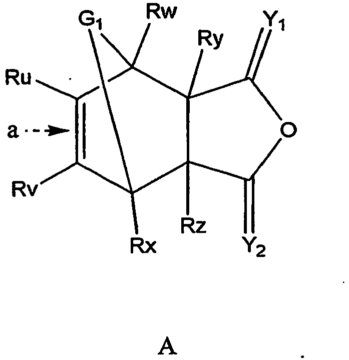

Addition of hydroxyl groups across the double bond a results in intermediate B: 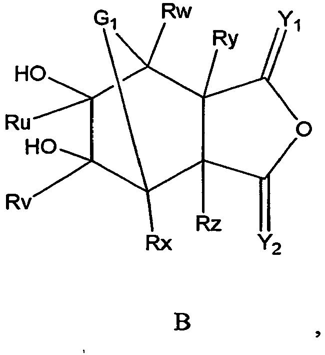 which may be derivatized to form a compound of formula II, above.

In other embodiments, compounds of formula I can be manufactured via the following pathway:

A dienophile IVa is reacted with a diene V to form an intermediate AA:

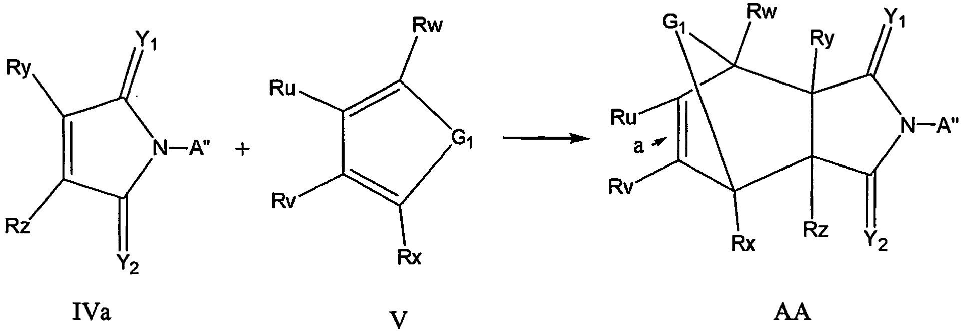

Hydroxyl groups may the be added across double bond a, to form the intermediate BB: 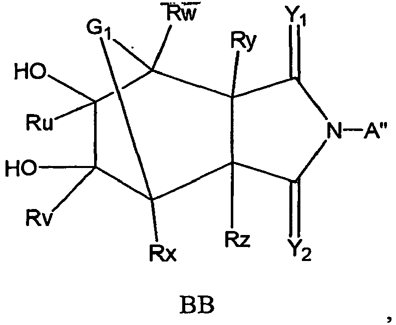 which may then be derivatized to form the compound of formula I, as described above.

In some embodiments of BB, AA" is H. Such embodiments may be derivatized by reacting BB with diacid, diacid chloride, diacid anhydride, etc. to produce a compound of the formula: 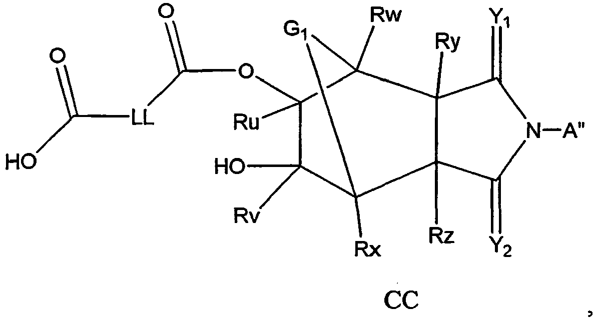 wherein LL is a divalent group such as alkylene, cycloalkylene, arylene, heterocyclyl, heteroarylene, and the other variables are as described above. Exemplary alkylene LL groups include C₁-C₁₂ alkylene (e.g. preferably methylene, ethylene (e.g. ethyl-1,2-ene), propylene (e.g. propyl-1,2-ene, propyl-1,3-ene), butylene, (e.g. butyl-1,4-ene, 2-methylpropyl-1,3-ene), pentylene, hexylene, heptylene, octylene, decylene, dodecylene), etc. Exemplary cycloalkylene groups include C₃-C₁₂ cycloalkylene groups, such as cyclopropylene, cyclobutylene, cyclopentanyl-1,3-ene, cyclohexyl-1,4-ene, etc. Exemplary arylene LL groups include mono- or bicyclic arylene groups having from 6 to about 14 carbon atoms, e.g. phenyl-1,2-ene, naphthyl-1,6-ene, napthyl-2,7-ene, anthracenyl, etc. Exemplary heterocyclyl groups within the scope of the invention include mono- or bicyclic aryl groups having from about 4 to about 12 carbon atoms and about 1 to about 4 hetero atoms, such as N, O and S, where the cyclic moieties may be partially dehydrogenated. Certain heteroaryl groups that may be mentioned as being within the scope of the invention include: pyrrolidinyl, piperidinyl (e.g. 2,5-piperidinyl, 3,5-piperidinyl), piperazinyl, tetrahydrothiophenyl, tetrahydrofuranyl, tetrahydro quinolinyl, tetrahydro isoquinolinyl, tetrahydroquinazolinyl, tetrahydroquinoxalinyl, etc. Exemplary heteroarylene groups include mono- or bicyclic aryl groups having from about 4 to about 12 carbon atoms and about 1 to about 4 hetero atoms, such as N, O and S. Certain heteroaryl groups that may be mentioned as being within the scope of the invention include: pyridylene (e.g. pyridyl-2,5-ene, pyridyl-3,5-ene), pyrimidinyl, thiophenyl, furanyl, quinolinyl, isoquinolinyl, quinazolinyl, quinoxalinyl, etc.

Suitable reagents for introducing the group HOCO-LL-CO above include diacids (HO₂C-LL-CO₂H). Particularly suitable diacids include malonic acid (LL is methylene), succinic acid (LL is 1,2-ethylene), glutaric acid, adipic acid, pimelic acid, and phthalic acid. Other suitable reagents for introducing HOCO-LL-CO above include diacid anhydrides. Particularly suitable diacid anhydrides include malonic anhydride, succinic anhydride, glutaric anhydride, adipic anhydride, pimelic anhydride, and phthalic anhydride. Other suitable reagents for introducing HOCO-LL-CO include diacid esters, diacid halides, etc. One especially preferred reagent for introducing HOCO-LL-CO is succinic anhydride.

The compound of formula may be linked to a support via terminal carboxylic acid of the HOCO-LL-CO group, via a reactive group on the support medium. In some embodiments, the terminal carboxylic acid forms an amide linkage with an amine reagent on the support surface. In other embodiments, the terminal carboxylic acid forms an ester with an OH group on the support medium. In some embodiments, the terminal carboxylic acid may be replaced with a terminal acid halide, acid ester, acid anhydride, etc. Specific acid halides include carboxylic chlorides, bromides and iodides. Specific esters include methyl, ethyl, and other C₁-C₁₀ alkyl esters. Specific anhydrides include formyl, acetyl, propanoyl, and other C₁-C₁₀ alkanoyl esters of the terminal carboxylic acid group of the compound of formula CC.

In particular embodiments of formula I, Ru, Rv, Rw, Rx, Ry, and Rz are independently H or C₁-C₄ alkyl; Y₁ and Y₂ are independently of each other O or S; G₁ is O, S, N-alkyl (wherein alkyl is C₁-C₁₀ alkyl), N-aryl (wherein aryl is C₆-C₁₄ aryl) or N-cycloalkyl (wherein cycloalkyl is C₃-C₁₂ mono- or bicycloalkyl); and A" is H, C₁-C₁₂ alkyl, C₃-C₁₂ cycloalkyl, or C₆-C₁₄ mono-, bi- or tri-cycloaryl.

In particularly preferred compounds of formula I, Ru, Rv, Rw, Rx, Ry, and Rz are independently H or methyl; Y, and Y₂ are both O; G₁ is O, S, N-methyl, N-ethyl, N-n-propyl, N-isopropyl, N-n-butyl, N-isobutyl, N-s-butyl or N-t-butyl, N-phenyl, N-cyclopentyl, N-cyclohexyl or N-cycloheptyl; and A" is H, C₁-C₄ alkyl, C₅-C₁₀ cycloalkyl, or phenyl.

In particularly preferred compounds of formula I, Ru, Rv, Rw, Rx, Ry, and Rz are independently H or methyl, at least one being methyl; Y₁ and Y₂ are both O; G₁ is O, S, N-methyl, N-ethyl, N-n-propyl, N-isopropyl, N-n-butyl, N-isobutyl, N-s-butyl or N-t-butyl, N-phenyl, N-cyclopentyl, N-cyclohexyl or N-cycloheptyl; and A" is H, C₁-C₄ alkyle, C₅-C₁₀ cycloalkyl, or phenyl.

In some preferred embodiments of compounds of formula I, at least two of Ru, Rv, Rw, Rx, Ry, and Rz are H, the remainder being substituents other than H. In particularly preferred embodiments at least two of Ru, Rv, Rw, Rx, Ry, and Rz are H, the remainder being substituents other than H ; and Y₁ and Y₂ are both O. In especially preferred embodiments, embodiments at least two of Ru, Rv, Rw, Rx, Ry, and Rz are H, the remainder being substituents other than H ; Y₁ and Y₂ are both O and G₁ is O or S. In specific particularly preferred embodiments, at least two of Ru, Rv, Rw, Rx, Ry, and Rz are H, the remainder being substituents other than H ; Y₁ and Y₂ are both O; G₁ is O or S; and A" is H, C₁-C₄ alkyl C₅-C₁₀ cycloalkyl, or phenyl. In other specific preferred embodiments, at least two of Ru, Rv, Rw, Rx, Ry, and Rz are H, the remainder being substituents other than H ; Y₁ and Y₂ are both O; G₁ is O; and A" is H, C₁-C₄ alkyl, C₅-C₁₀ cycloalkyl, or phenyl.

In some preferred embodiments of compounds of formula I, each of Ru, Rv, Rw, Rx, Ry, and Rz is H. In particularly preferred embodiments each of Ru, Rv, Rw, Rx, Ry, and Rz is H; and Y₁ and Y₂ are both O. In especially preferred embodiments, each of Ru, Rv, Rw, Rx, Ry, and Rz is H; Y₁ and Y₂ are both O and G₁ is O or S. In specific particularly preferred embodiments, each of Ru, Rv, Rw, Rx, Ry, and Rz is H; Y₁ and Y₂ are both O; G₁ is O or S; and A" is H, C₁-C₄ alkyl, C₅-C₁₀ cycloalkyl, or phenyl. In other specific preferred embodiments, each of Ru, Rv, Rw, Rx, Ry, and Rz is H; Y₁ and Y₂ are both O; G₁ is O; and A" is H, C₁-C₄ alkyl, C₅-C₁₀ cycloalkyl, or phenyl.

In some embodiments of the invention, Y₁ and Y₂ are S.

In some embodiments of the invention, G₁ is O or S.

In some embodiments of the invention, A" is H, C₁-C₄ alkyl or phenyl.

In a further embodiment of the present disclosure is a process of making a product of the formula: 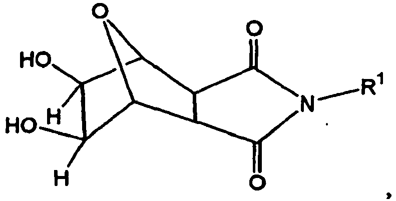 wherein R¹ is aryl, cycloalkyl, unsaturated cycloalkyl, alkyl, unsaturated alkyl, heterocyclyl, unsaturated heterocyclyl, heteroaryl or acyl, wherein R¹ is optionally substituted with one or more substituents, the process comprising reacting an intermediate of formula: 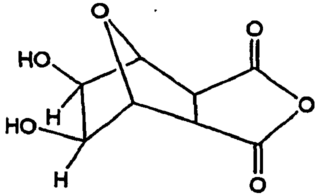 with a compound of formula: NH₂-R¹, wherein aryl, cycloalkyl, unsaturated cycloalkyl, alkyl, unsaturated alkyl, heterocyclyl, unsaturated heterocyclyl, heteroaryl or acyl, wherein R¹ is optionally substituted with one or more substituents, under conditions sufficient to produce the product. In a further embodiment of the present invention is a process of making a product of the formula: 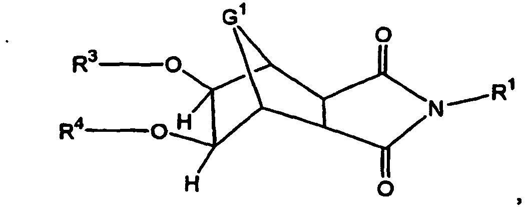 wherein G¹ is O, R³ is H, R⁴ is pg, wherein pg is a protecting group; R¹ is aryl, cycloalkyl, unsaturated cycloalkyl, alkyl, unsaturated alkyl, heterocyclyl, unsaturated heterocyclyl, heteroaryl or acyl, wherein R¹ is optionally substituted with one or more substituents, the process comprising reacting an intermediate of formula: 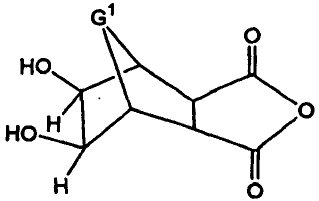 with a reactant of formula: H₂NR¹ under conditions suitable to produce an imide intermediate of formula: 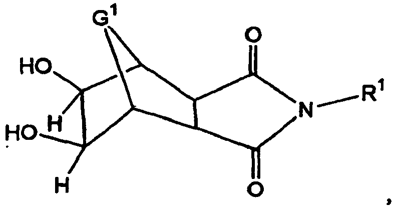 and reacting the imide intermediate with a compound of formula Ig - pg, wherein lg is a leaving group and pg is a protecting group, to form the product.

In a further embodiment of the present is a process of making a product of the formula: 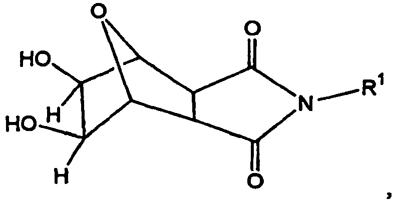 wherein R¹ is phenyl, the process comprising reacting an intermediate of formula: 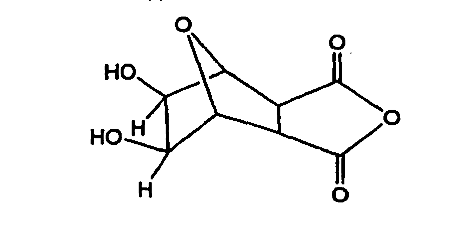 with a compound of formula: NH₂-R¹, wherein R¹ is phenyl, under conditions sufficient to produce the product.

In a further embodiment of the present disclosure is a process of making a product of the formula: 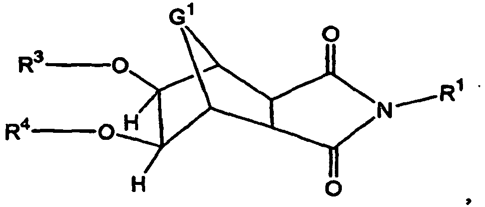 wherein G¹ is O, S or NR², wherein R² is a member of the group consisting of alkyl, cycloalkyl, aryl, heterocyclyl or heteroaryl, wherein R² is optionally further substituted and wherein each R² optionally contains one or more unsaturations; R³ is H, R⁴ is pg, wherein pg is a protecting group; R¹ is aryl, cycloalkyl, unsaturated cycloalkyl, alkyl, unsaturated alkyl, heterocyclyl, unsaturated heterocyclyl, heteroaryl or acyl, wherein R¹ is optionally substituted with one or more substituents, the process comprising reacting an intermediate of formula: 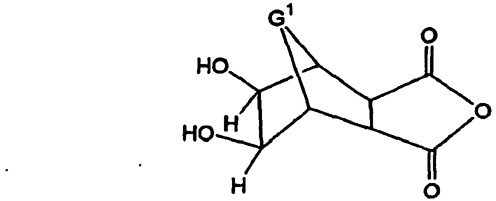 with a reactant of formula: H₂NR¹ under conditions suitable to produce an imide intermediate of formula: 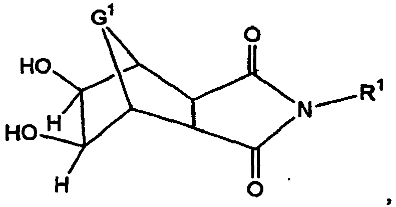 and reacting the imide intermediate with a compound of formula lg - pg, wherein lg is a leaving group and pg is a protecting group, to form the product.

The process of claim 125, wherein R¹ is optionally substituted aryl or alkyl substituted with optionally substituted aryl.

In a further embodiment of the present disclosure is a process of making a product of the formula: 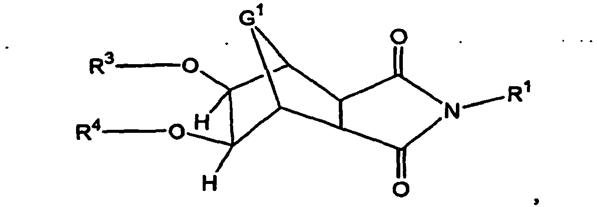 wherein G¹ is O, R³ is H, R⁴ is pg, wherein pg is a protecting group; R¹ is aryl, cycloalkyl, unsaturated cycloalkyl, alkyl, unsaturated alkyl heterocyclyl, unsaturated heterocyclyl, heteroaryl or acyl, wherein R¹ is optionally substituted with one or more substituents, the process comprising reacting an intermediate of formula: 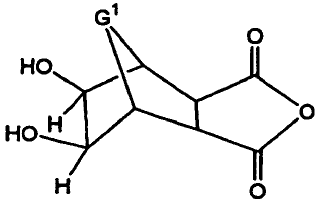 with a reactant of formula: H₂NR¹ under conditions suitable to produce an imide intermediate of formula: 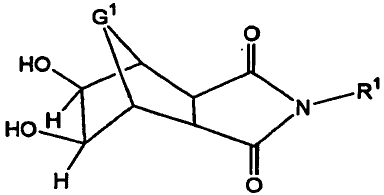 and reacting the imide intermediate with a compound of formula **lg -** pg, wherein lg is a leaving group and pg is a protecting group, to form the product.

In a further embodiment of the present disclosure is a process of making a product of the formula: 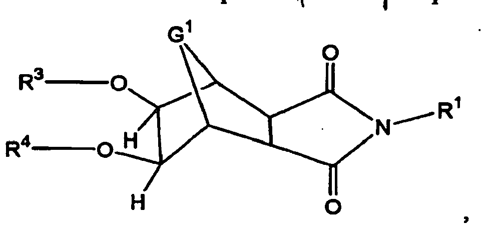 wherein G¹ is O, R³ is H, R⁴ is pg, wherein pg is a protecting group; and R¹ is phenyl, the process comprising reacting an intermediate of formula: 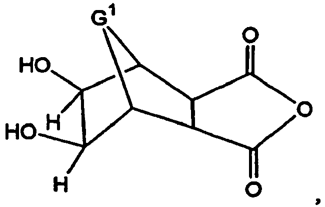 wherein G¹ is O,
with a compound of formula: NH₂-R¹, wherein R¹ is phenyl, under conditions sufficient to produce an imide intermediate of formula: 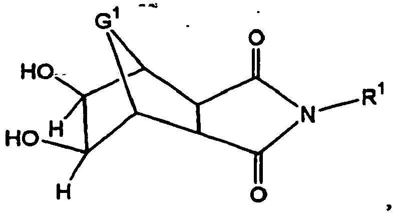 and reacting the imide intermediate with a compound of formula: lg - pg, wherein lg is a leaving group and pg is a protecting group under conditions sufficient to produce the product.

In a further embodiment of the present disclosure is a process of making a product of the formula: 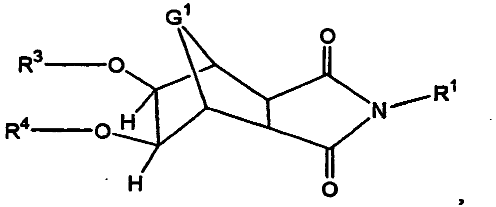 wherein G¹ is O, S or NR², wherein R² is a member of the group consisting of alkyl, cycloalkyl, aryl, heterocyclyl or heteroaryl, wherein R² is optionally further substituted and wherein each R² optionally contains one or more unsaturations; R³ is L¹, wherein L¹ is a linking moiety, R⁴ is pg, wherein pg is a protecting group; R¹ is aryl, cycloalkyl, unsaturated cycloalkyl, alkyl, unsaturated alkyl, heterocyclyl, unsaturated heterocyclyl, heteroaryl or acyl, wherein R¹ is optionally substituted with one or more substituents, the process comprising reacting an intermediate of formula: 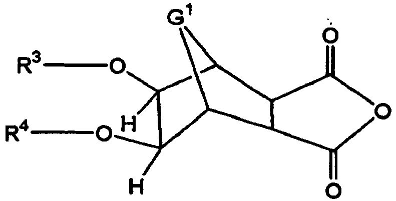 with a reactant of formula: H₂NR¹ under conditions suitable to produce an imide intermediate of formula: 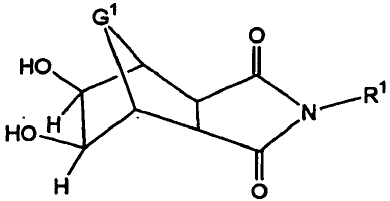 reacting the imide intermediate with a compound of formula lg - pg, wherein 1g is a leaving group and pg is a protecting group, to form a protected intermediate of the formula: 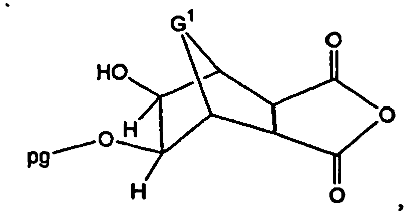 and then reacting the protected intermediate with a reagent suitable to introduce the L¹ moiety, under conditions suitable to produce the product. The process of claim 140, wherein R¹ is optionally substituted aryl or alkyl substituted with optionally substituted aryl.

In a further embodiment of the present disclosure is a process of making a product of the formula: 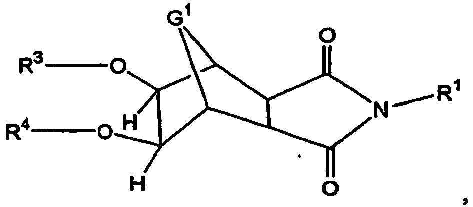 wherein G¹ is **O;** R³ is L¹, wherein L¹ is a linking moiety, R⁴ is pg, wherein pg is a protecting group; R¹ is aryl, cycloalkyl, unsaturated cycloalkyl, alkyl, unsaturated alkyl, heterocyclyl, unsaturated heterocyclyl, heteroaryl or acyl, wherein R¹ is optionally substituted with one or more substituents, the process comprising reacting an intermediate of formula: 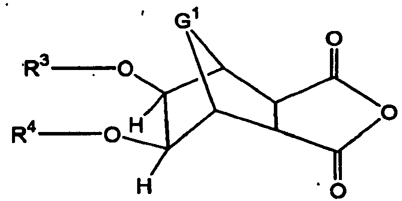 with a reactant of formula: H₂NR¹ under conditions suitable to produce an imide intermediate of formula: 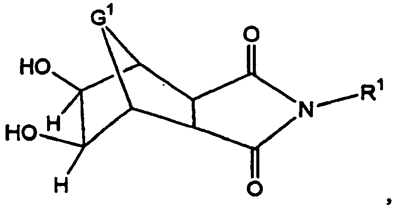 reacting the imide intermediate with a compound of formula Ig - pg, wherein Ig is a leaving group and pg is a protecting group, to form a protected intermediate of the formula: 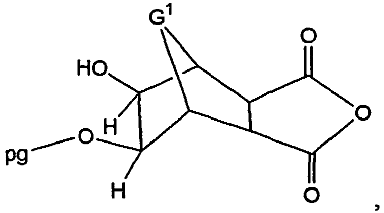 and then reacting the protected intermediate with a reagent suitable to introduce the L¹ moiety, under conditions suitable to produce the product. The process of claim 151, wherein R¹ is optionally substituted aryl or alkyl substituted with optionally substituted aryl.

In a further embodiment of the present invention is a process of making a product of the formula: 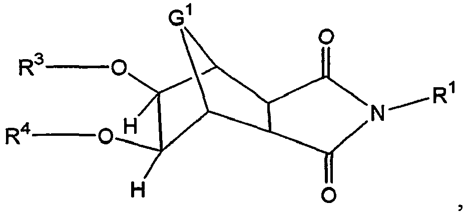 wherein G¹ is O, S or NR², wherein R² is a member of the group consisting of alkyl, cycloalkyl, aryl, heterocyclyl or heteroaryl, wherein R² is optionally further substituted and wherein each R² optionally contains one or more unsaturations; R³ is L¹-SS, wherein L¹ is a linking moiety and SS is a solid support medium, R⁴ is pg, wherein pg is a protecting group; R¹ is aryl, cycloalkyl, unsaturated cycloalkyl, alkyl, unsaturated alkyl, heterocyclyl, unsaturated heterocyclyl, heteroaryl or acyl, wherein R¹ is optionally substituted with one or more substituents, the process comprising reacting an intermediate of formula: 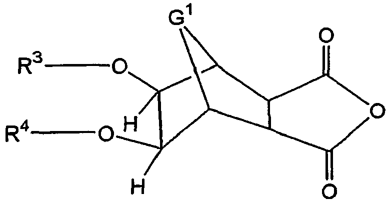 with a reactant of formula: H₂NR¹ under conditions suitable to produce an imide intermediate of formula: 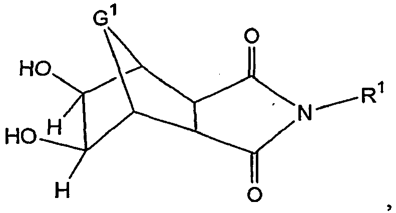 reacting the imide intermediate with a compound of formula lg - pg, wherein lg is a leaving group and pg is a protecting group, to form a protected intermediate of the formula: 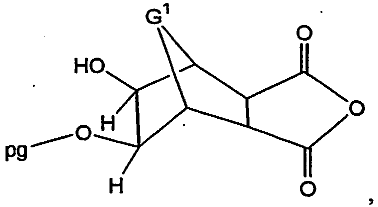 and then reacting the protected intermediate with a reagent suitable to introduce the L¹ moiety, under conditions suitable to produce a support intermdiate of the formula: 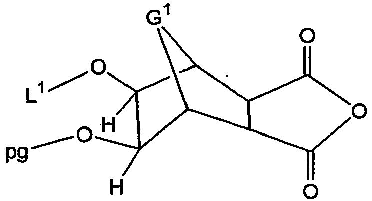 and then reacting the support intermediate with a solid support medium under conditions suitable to produce the product.

In a further embodiment of the present invention is a process of making a product of the formula: 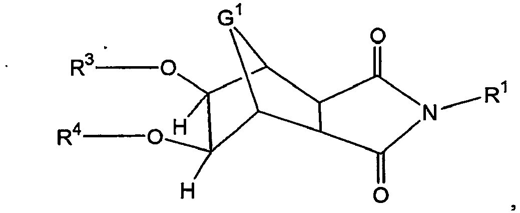 wherein G¹ is O; R³ is L¹-SS, wherein L¹ is a linking moiety and SS is a solid support medium, wherein L¹ is a linking moiety, R⁴ is pg, wherein pg is a protecting group; R¹ is aryl, cycloalkyl, unsaturated cycloalkyl, alkyl, unsaturated alkyl, heterocyclyl, unsaturated heterocyclyl, heteroaryl or acyl, wherein R¹ is optionally substituted with one or more substituents, the process comprising reacting an intermediate of formula: 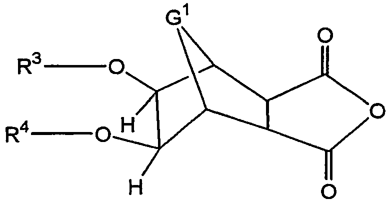 with a reactant of formula: H₂NR¹ under conditions suitable to produce an imide intermediate of formula: 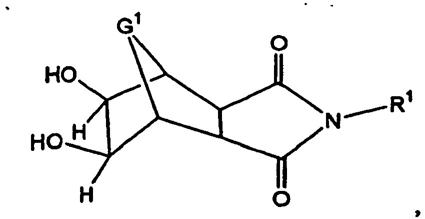 reacting the imide intermediate with a compound of formula lg - pg, wherein lg is a leaving group and pg is a protecting group, to form a protected intermediate of the formula: 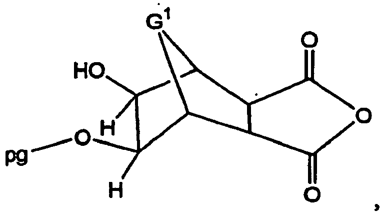 and then reacting the protected intermediate with a reagent suitable to introduce the L¹ moiety, under conditions suitable to produce a support intermediate of formula: 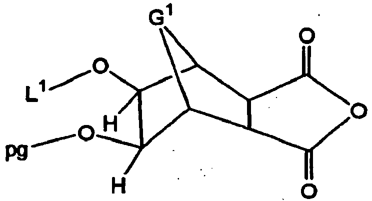 and reacting the support intermediate with a solid support medium having a suitable functional group under conditions suitable to produce the product.

In a further embodiment of the present invention is a process of making a product of the formula: 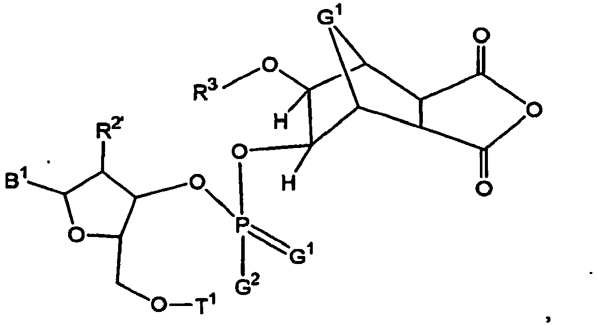 wherein B¹ is a nucleobase, G¹ is O or S, G² is OT², ST² or NR^{N}N^{N'}, R^{2'} is a H, OH, protected OH or a substituent; T¹ is a protecting group, G¹ is O, S or NR², wherein R² is a member of the group consisting of alkyl, cycloalkyl, aryl, heterocyclyl or heteroaryl, wherein R² is optionally further substituted and wherein each R² optionally contains one or more unsaturations; R³ is L¹-SS, wherein L^{T} is a linking moiety and SS is a solid support medium, R⁴ is pg, wherein pg is a protecting group; R¹ is aryl, cycloalkyl unsaturated cycloalkyl, alkyl, unsaturated alkyl, heterocyclyl, unsaturated heterocyclyl, heteroaryl or acyl, wherein R¹ is optionally substituted with one or more substituents, the process comprising reacting an intermediate of formula: 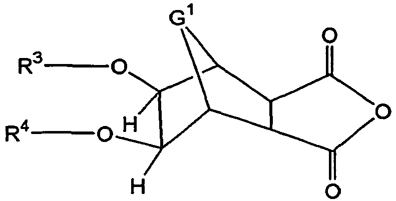 with a reactant of formula: H₂NR¹ under conditions suitable to produce an imide intermediate of formula: 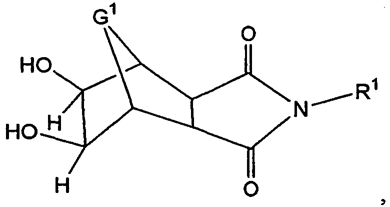 reacting the imide intermediate with a compound of formula lg - pg, wherein lg is a leaving group and pg is a protecting group, to form a protected intermediate of the formula: 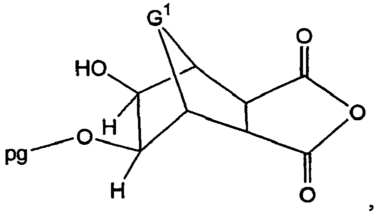 and then reacting the protected intermediate with a reagent suitable to introduce the L¹ moiety, under conditions suitable to produce a support intermediate of the formula: 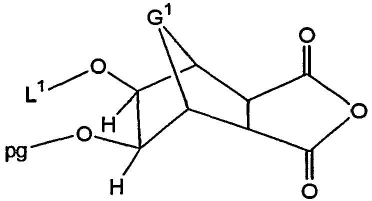 and then reacting the support intermediate with a solid support medium under conditions suitable to produce an oligonucleotide synthesis support of the formula: 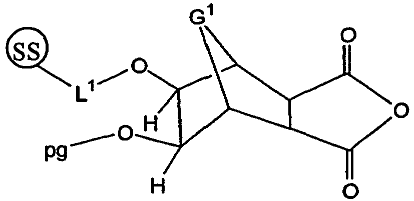 reacting the oligonucleotide synthesis support with an acid to remove pg and form a de-protected oligonucleotide synthesis support, then reacting the deprotected oligonucleotide synthesis support with an activated nucleoside: 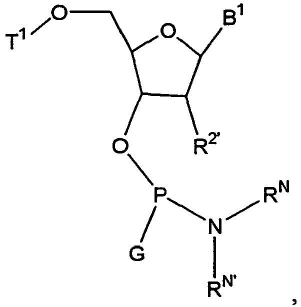 wherein G is OT², ST² or NR^{N}R^{N'}, wherein T² is a blocking group and R^{N} and R^{N'} independently are alkyl or together with the N to which they are attached form a heterocylcic ring structure, under conditions suitable to produce the intermediate: 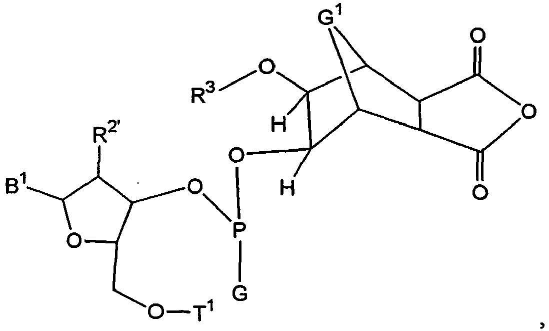 and oxidizing the intermediate to form the product.

A process of making a product of formula: 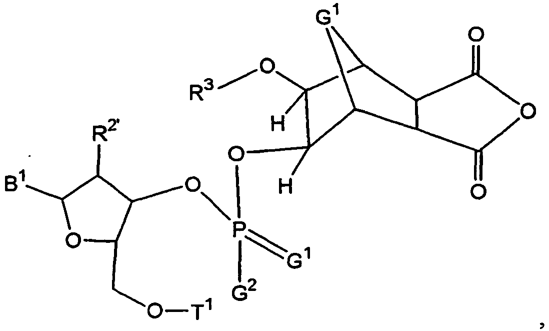 wherein B¹ is a nucleobase, R^{2'} is a H, OH, protected OH or a substituent; T¹ is a protecting group; G¹ is O; R³ is L¹-SS, wherein L¹ is a linking moiety and SS is a solid support medium, wherein L¹ is a linking moiety, R⁴ is pg, wherein pg is a protecting group; R¹ is aryl, cycloalkyl, unsaturated cycloalkyl, alkyl, unsaturated alkyl, heterocyclyl, unsaturated heterocyclyl, heteroaryl or acyl, wherein R¹ is optionally substituted with one or more substituents, the process comprising reacting an intermediate of formula: 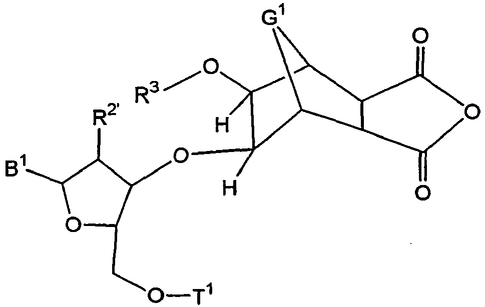 with a reactant of formula: H₂NR¹ under conditions suitable to produce an imide intermediate of formula: 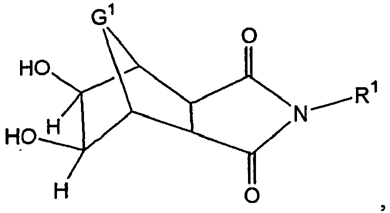 reacting the imide intermediate with a compound of formula lg - pg, wherein lg is a leaving group and pg is a protecting group, to form a protected intermediate of the formula: 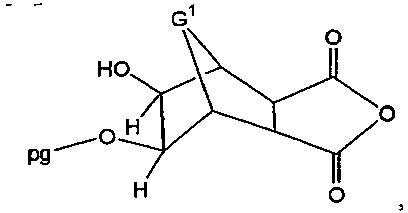 and then reacting the protected intermediate with a reagent suitable to introduce the L¹ moiety, under conditions suitable to produce a support intermediate of formula: 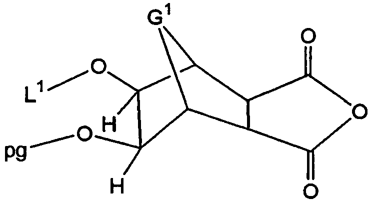 and reacting the support intermediate with a solid support medium having a suitable functional group under conditions suitable to produce an oligonucleotide synthesis support of the formula: 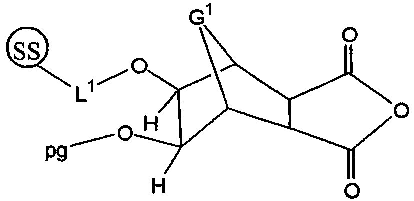 reacting the oligonucleotide synthesis support with an acid to remove pg and form a de-protected oligonucleotide synthesis support, then reacting the deprotected oligonucleotide synthesis support with an activated nucleoside: 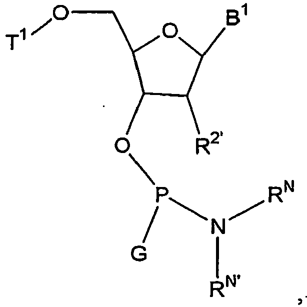 wherein G is OT², ST² or NR^{N}R^{N'}, wherein T² is a blocking group and R^{N} and R^{N'} independently are alkyl or together with the N to which they are attached form a heterocylcic ring structure, under conditions suitable to produce the intermediate: 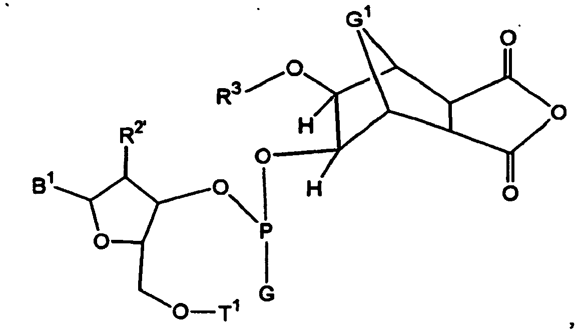 and oxidizing the intermediate to form the product. The process of claim , wherein R¹ is optionally substituted aryl or alkyl substituted with optionally substituted aryl.

In preferred embodiments of the present process, pg is a 4,4'-dimethoxythphenylmethyl group, a 4-methoxytriphenylmethyl group, a pixyl group or a further substituted pixyl group; the reagent suitable to introduce the L¹ moiety is an anhydride, a cyclic anhydride, or more specifically a succinic anhydride; B¹ is a member of the group consisting of thyminyl, cytosinyl, uridinyl, 5-methylcytosinyl, guanyl and adeninyl; R^{2'} is a 2'-substituent selected from H, OH, F, OMe, OCH₂CH₂OMe, OCH₂CH₂CH₂NH₂, or OY, wherein Y is a removable protecting group; T¹ is an acid-labile protecting group selected from 4,4'-dimethoxytriphenylmethyl group, a 4-methoxytriphenylmethyl group, a pixyl group or a further substituted pixyl group.

In a further embodiment of the present disclosure is a process of making a product of the formula: 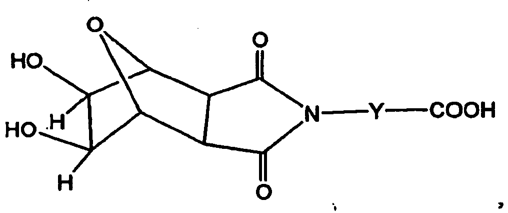 wherein Y is alkylene, cycloalkylene, arylene, heteroarylene, arylalkylene, alkarylene, or alkylenearylalkylene, the process comprising reacting an intermediate of the formula: 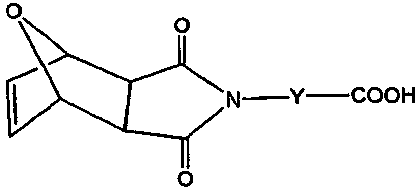 with a suitable oxidizing reagent to form the product, wherein the suitable oxidizing reagent is H₂O₂, optionally in the presence of a catalyst.

In a further embodiment of the present invention is a process of making a product of the formula: 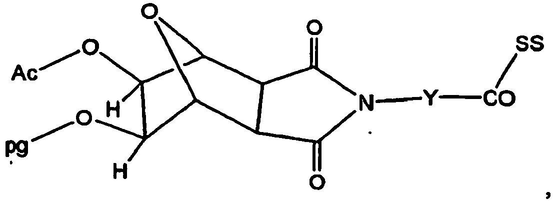 wherein Y is alkylene, cycloalkylene, arylene, heteroarylene, arylalkylene, alkarylene, or alkylenearylalkylene, SS is a solid support medium, Ac is an acyl blocking group and pg is a protecting group the process comprising reacting a compound of formula: 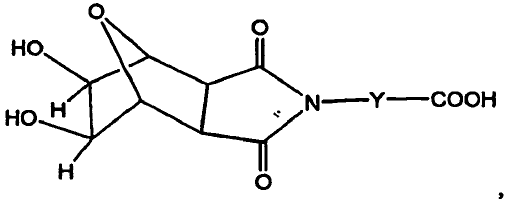 with a protecting reagent of formula lg - pg, wherein lg is a leaving group and pg is a protecting group, to form a protected intermediate of formula: 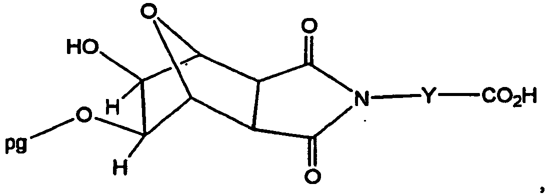 and reacting the protected intermediate with an acylating reagent to form a protected-blocked intermediate of formula: 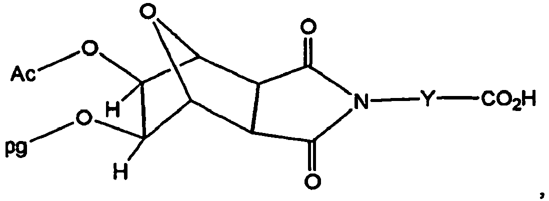 wherein Ac is a blocking group, and reacting the protected-blocked intermediate with a suitable solid support medium to form the product.

In a further embodiment of the present disclosure is a process of making a product of the formula: 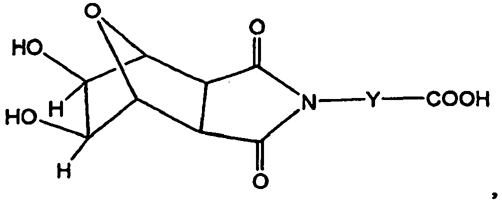 wherein Y is alkylene, cycloalkylene, arylene, heteroarylene, arylalkylene, alkarylene, or alkylenearylalkylene, the process comprising reacting an intermediate of the formula: 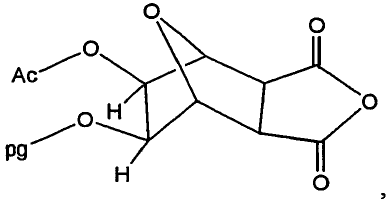 wherein Ac is a blocking group and pg is a protecting group, with a reagent of the formula NH₂-Y-COOH under conditions suitable to produce the product.

In a further embodiment of the present invention is a process of making a product of the formula: 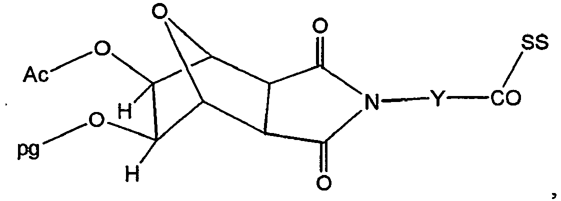 wherein Y is alkylene, cycloalkylene, arylene, heteroarylene, arylalkylene, alkarylene, or alkylenearylalkylene, SS is a solid support medium, Ac is an acyl blocking group and pg is a protecting group the process comprising reacting a compound of formula: 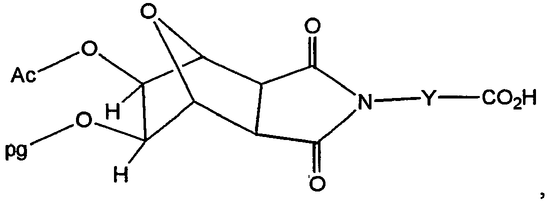 wherein Ac is a blocking group, and reacting the protected-blocked intermediate, with a suitable solid support medium SS to form the product.

### Nucleobases

The nucleobases Bx may be the same or different, and include naturally occurring nucleobases adenine (A), guanine (G), thymine (T), uracil (U) and cytosine (C), as well as modified nucleobases. Modified nucleobases include heterocyclic moieties that are structurally related to the naturally-occurring nucleobases, but which have been chemically modified to impart some property to the modified nucleobase that is not possessed by naturally-occurring nucleobases. The term "nucleobase," as used herein, is intended to by synonymous with "nucleic acid base or mimetic thereof." In general, a nucleobase is any substructure that contains one or more atoms or groups of atoms capable of hydrogen bonding to a base of an oligonucleotide.

As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5 trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine. Further modified nucleobases include tricyclic pyrimidines such as phenoxazine cytidine(1H-pyrimido[5,4-b][1,4]benzoxazin-2(3H)-one), phenothiazine cytidine (1H-pyrimido[5,4-b][1,4]benzothiazin-2(3H)-one), G-clamps such as a substituted phenoxazine cytidine (e.g. 9-(2-aminoethoxy)-H-pyrimido[5,4-b][1,4]benzoxazin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in United States Patent No. 3,687,808, those disclosed in The Concise Encyclopedia Of Polymer Science And Engineering, pages 858-859, Kroschwitz, J.I., ed. John Wiley & Sons, 1990, those disclosed by Englisch et al., Angewandte Chemie, International Edition, 1991, 30, 613, and those disclosed by Sanghvi, Y.S., Chapter 15, Antisense Research and Applications, pages 289-302, Crooke, S.T. and Lebleu, B. , ed., CRC Press, 1993.

Certain of these nucleobases are particularly useful for increasing the binding affinity of the oligomeric compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2°C (Sanghvi, Y.S., Crooke, S.T. and Lebleu, B., eds., Antisense Research and Applications, CRC Press, Boca Raton, 1993, pp. 276-278) and are presently preferred base substitutions, even more particularly when combined with 2'-O-methoxyethyl sugar modifications.

Additional modifications may also be made at other positions on the oligonucleotide, particularly the 3' position of the sugar on the 3' terminal nucleotide and the 5' position of 5' terminal nucleotide. For example, one additional modification of the ligand conjugated oligonucleotides of the present invention involves chemically linking to the oligonucleotide one or more additional non-ligand moieties or conjugates which enhance the activity, cellular distribution or cellular uptake of the oligonucleotide. Such moieties include but are not limited to lipid moieties such as a cholesterol moiety (Letsinger et al., Proc. Natl. Acad. Sci. USA, 1989, 86, 6553), cholic acid (Manoharan et al., Bioorg. Med. Chem. Lett., 1994, 4, 1053), a thioether, e.g., hexyl-S-tritylthiol (Manoharan et al., Ann. N.Y. Acad. Sci., 1992, 660, 306; Manoharan et al., Bioorg. Med. Chem. Let., 1993, 3, 2765), a thiocholesterol (Oberhauser et al., Nucl. Acids Res., 1992, 20, 533), an aliphatic chain, e.g., dodecandiol or undecyl residues (Saison-Behmoaras et al., EMBO J., 1991, 10, 111; Kabanov et al., FEBS Lett., 1990, 259, 327; Svinarchuk et al., Biochimie, 1993, 75, 49), a phospholipid, e.g., di-hexadecyl-rac-glycerol or triethylammonium 1,2-di-O-hexadecyl-rac-glycero-3-H-phosphonate (Manoharan et al., Tetrahedron Lett., 1995, 36, 3651; Shea et al., Nucl. Acids Res., 1990, 18, 3777), a polyamine or a polyethylene glycol chain (Manoharan et al., Nucleosides & Nucleotides, 1995, 14, 969), or adamantane acetic acid (Manoharan et al., Tetrahedron Lett., 1995 36, 3651), a palmityl moiety (Mishra et al., Biochim. Biophys. Acta, 1995, 1264, 229), or an octadecylamine or hexylamino-carbonyl-oxycholesterol moiety (Crooke et al., J. Pharmacol. Exp. Ther., 1996, 277, 923).

Also encompassed within the scope of the present invention are oligonucleotides which comprise at least on nucleotide bearing a 2'-substituent selected from OH, F, O-alkyl (e.g. O-methyl), S-alkyl, N-alkyl, O-alkenyl, S-alkenyl, N-alkenyl; O-alkynyl, S-alkynyl, N-alkynyl; O-alkyl-O-alkyl, wherein the alkyl, alkenyl and alkynyl may be substituted or unsubstituted C₁ to C₁₀ alkyl or C₂ to C₁₀ alkenyl or alkynyl, respectively. Particularly preferred are O[(CH₂)_{g}O]ₕCH₃, O(CH₂)_{g}OCH₃, O(CH₂)_{g}NH₂, O(CH₂)_{g}CH₃, O(CH₂)_{g}ONH₂, and O(CH₂)_{g}ON[(CH₂)_{g}CH₃]₂, where g and h are from 1 to about 10. Other preferred oligonucleotides comprise one of the following at the 2' position: C₁ to C₁₀ lower alkyl, substituted lower alkyl, alkenyl, alkynyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred 2'-modification includes 2'-methoxyethoxy (2'-O-CH₂CH₂OCH₃, also known as 2'-O-(2-methoxyethyl) or 2'-MOE) (Martin et al., Helv. Chim. Acta, 1995, 78, 486-504). A further preferred modification includes 2'-dimethylaminooxyethoxy, i.e., a O(CH₂)₂ON(CH₃)₂ group, also known as 2'-DMAOE, as described in examples hereinbelow, and 2'-dimethylaminoethoxyethoxy (also known in the art as 2'-O-dimethyl-amino-ethoxy-ethyl or 2'-DMAEOE), i.e., 2'-O-CH₂-O-CH₂-N(CH₃)₂, also described in examples hereinbelow.

Other preferred modifications include 2'-methoxy (2'-O-CH₃), 2'-aminopropoxy (2'-OCH₂CH₂CH₂NH₂), 2'-allyl (2'-CH₂-CH=CH₂), 2'-O-allyl (2'-O-CH₂-CH=CH₂) and 2'-fluoro (2'-F). The 2'-modification may be in the arabino (up) position or ribo (down) position. A preferred 2'-arabino modification is 2'-F. Similar modifications may also be made at other positions on the oligonucleotide, particularly the 3' position of the sugar on the 3' terminal nucleotide or in 2'-5' linked oligonucleotides and the 5' position of 5' terminal nucleotide.

Particularly useful sugar substituent groups include O[(CH₂)_{g}O]ₕCH₃, O(CH₂)_{g}OCH₃, O(CH₂)_{g}NH_{2.} O(CH₂)_{g}CH₃, O(CH₂)_{g}ONH₂, and O(CH₂)_{g}ON[(CH₂)_{g}CH₃)]₂, where g and h are from 1 to about 10.

Some particularly useful oligomeric compounds of the invention contain at least one nucleoside having one of the following substituent groups: C₁ to C₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂CH₃, ONO₂ NO_{2.} N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligomeric compound, or a group for improving the pharmacodynamic properties of an oligomeric compound, and other substituents having similar properties. A preferred modification includes 2'-methoxyethoxy [2'-O-CH₂CH₂OCH₃, also known as 2'-O-(2-methoxyethyl) or 2'-MOE] (Martin et al., Helv. Chim. Acta, 1995, 78, 486), i.e., an alkoxyalkoxy group. A further preferred modification is 2'-dimethylaminooxyethoxy, i.e., a O(CH₂)₂ON(CH₃)₂ group, also known as 2'-DMAOE. Representative aminooxy substituent groups are described in co-owned United States Patent Application serial number 09/344,260, filed June 25, 1999, entitled "Aminooxy-Functionalized Oligomers"; and United States Patent Application serial number 09/370,541, filed August 9, 1999, entitled "Aminooxy-Functionalized Oligomers and Methods for Making Same."

Other particularly advantageous 2'-modifications include 2'-methoxy (2'-O-CH₃), 2'-aminopropoxy (2'-OCH₂CH₂CH₂NH₂) and 2'-fluoro (2'-F). Similar modifications may also be made at other positions on nucleosides and oligomers, particularly the 3' position of the sugar on the 3' terminal nucleoside or at a 3'-position of a nucleoside that has a linkage from the 2'-position such as a 2'-5' linked oligomer and at the 5' position of a 5' terminal nucleoside. Oligomers may also have sugar mimetics such as cyclobutyl moieties in place of the pentofuranosyl sugar.

Representative acetamido 2' substituent groups of preferred oligonucleotides which may be produced by the methods of the present invention are disclosed in United States Patent 6,147,200 which is hereby incorporated by reference in its entirety. Representative dimethylaminoethyloxyethyl substituent groups are disclosed in International Patent Application PCT/US99/17895, entitled "2'-O-Dimethylaminoethyloxyethyl-Modified Oligonucleotides", filed August 6, 1999, hereby incorporated by reference in its entirety. For those nucleosides that include a pentofuranosyl sugar, the phosphate group can be linked to either the 2', 3' or 5' hydroxyl moiety of the sugar. In forming oligonucleotides, the phosphate groups covalently link adjacent nucleosides to one another to form a linear polymeric compound. The respective ends of this linear polymeric structure can be joined to form a circular structure by hybridization or by formation of a covalent bond, however, open linear structures are generally preferred. Within the oligonucleotide structure, the phosphate groups are commonly referred to as forming the internucleoside linkages of the oligonucleotide. The normal internucleoside linkage of RNA and DNA is a 3' to 5' phosphodiester linkage.

While the present invention may be adapted to produce oligonucleotides for any desired end use (e.g. as probes for us in the polymerase chain reaction), one preferred use of the oligonucleotides is in antisense therapeutics. One mode of action that is often employed in antisense therapeutics is the so-called RNAse H mechanism, whereby a strand of DNA is introduced into a cell, where the DNA hybridizes to a strand of RNA. The DNA-RNA hybrid is recognized by an endonuclease, RNAse H, which cleaves the RNA strand. In normal cases, the RNA strand is messenger RNA (mRNA), which, after it has been cleaved, cannot be translated into the corresponding peptide or protein sequence in the ribosomes. In this way, DNA may be employed as an agent for modulating the expression of certain genes.

The processes of the present invention are amenable to the production of oligonucleotides which comprise at least one nucleotide having a bicyclic sugar moiety, i.e. an LNA nucleotide (oligonucleotides wherein the 2' and 4' positions are connected by a bridge) also form duplexes with complementary DNA, RNA or LNA with high thermal affinities. Circular dichroism (CD) spectra show that duplexes involving fully modified LNA (esp. LNA:RNA) structurally resemble an A-form RNA:RNA duplex. Nuclear magnetic resonance (NMR) examination of an LNA:DNA duplex confirmed the 3'-endo conformation of an LNA monomer. Recognition of double-stranded DNA has also been demonstrated suggesting strand invasion by LNA. Studies of mismatched sequences show that LNAs obey the Watson-Crick base pairing rules with generally improved selectivity compared to the corresponding unmodified reference strands.

LNAs in which the 2'-hydroxyl group is linked to the 4' carbon atom of the sugar ring thereby forming a 2'-C,4'-C-oxymethylene linkage thereby forming a bicyclic sugar moiety. The linkage may be a methelyne (-CH₂-)ₙ group bridging the 2' oxygen atom and the 4' carbon atom wherein n is 1 or 2 (Singh et al., Chem. Commun., 1998, 4, 455-456). LNA and LNA analogs display very high duplex thermal stabilities with complementary DNA and RNA (Tm = +3 to +10 C), stability towards 3'-exonucleolytic degradation and good solubility properties. Other preferred bridge groups include the 2'-deoxy-2'-CH₂OCH₂-4' bridge.

### Alternative Internucleoside Linkers

In addition to phosphate diester and phosphorothioate diester inter nucleoside linkages, other internucleoside linkers are known in the art. The methods of present invention are amenable to the synthesis of oligonucleotides which comprise full phosphate diester or full phosphorothioate diester backbones, however, chimeric compounds having more than one type of intemucleotide linkage, as well as oligomers having non-phosphate/phosphorothioate diester linkages as described in further detail below, are also contemplated in whole or in part within the context of the present invention.

Exemplary non-phosphate/phosphorothioate diester linkages contemplated within the skill of the art include: phosphorodithioates, phosphotriesters, aminoalkylphosphotriesters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, selenophosphates and boianophosphates. Additional linkages include: thiodiester (-O-C(O)-S-), thionocarbamate (-O-C(O)(NJ)-S-), siloxane (-O-Si(J)₂-O-), carbamate (-O-C(O)-NH- and NH-C(O)-O-), sulfamate (-O-S(O)(O)-N- and N-S(O)(O)-N-, morpholino sulfamide (-O-S(O)(N(morpholino)-), sulfonamide (-O-SO₂-NH-), sulfide (-CH₂-S-CH₂-), sulfonate (-O-SO₂-CH₂-), N,N'-dimethylhydrazine (-CH₂-N(CH₃)-N(CH₃)-), thioformacetal (-S-CH₂-O-), formacetal (-O-CH₂-O-), thioketal (-S-C(J)₂-O-), ketal (-O-C(J)₂-O-), amine (-NH-CH₂-CH₂-), hydroxylamine (-CH₂-N(J)-O-), hydroxylimine (-CH=N-O-), and hydrazinyl (-CH₂-N(H)-N(H)-).

In each of the foregoing substructures relating to internucleoside linkages, J denotes a substituent group which is commonly hydrogen or an alkyl group or a more complicated group that varies from one type of linkage to another.

In addition to linking groups as described above that involve the modification or substitution of the -O-P-O- atoms of a naturally occurring linkage, included within the scope of the present invention are linking groups that include modification of the 5'-methylene group as well as one or more of the -O-P-O-atoms. Linkages of this type are well documented in the prior art and include without limitation the following: amides (-CH₂-CH₂-N(H)-C(O)) and -CH₂-O-N=CH-;and alkylphosphorus (-C(J)₂-P(=O)(OJ)-C(J)₂-C(J)₂-). J is as described above.

### Oligonucleotide Synthesis

Oligonucleotides are generally prepared, as described above, on a solid support medium, e.g. a solid support medium. In general a first synthon (e.g. a monomer, such as a nucleoside) is first attached to a solid support medium, and the oligonucleotide is then synthesized by sequentially coupling monomers to the solid support-bound synthon. This iterative elongation eventually results in a final oligomeric compound or other polymer such as a polypeptide. Suitable solid support media can be soluble or insoluble, or may possess variable solubility in different solvents to allow the growing solid support bound polymer to be either in or out of solution as desired. Traditional support media such as solid support media are for the most part insoluble and are routinely placed in reaction vessels while reagents and solvents react with and/or wash the growing chain until the oligomer has reached the target length, after which it is cleaved from the support and, if necessary further worked up to produce the final polymeric compound. More recent approaches have introduced soluble supports including soluble polymer supports to allow precipitating and dissolving the iteratively synthesized product at desired points in the synthesis (Gravert et al., Chem. Rev., 1997, 97, 489-510).

The term support medium is intended to include all forms of support known to the art skilled for the synthesis of oligomeric compounds and related compounds such as peptides. Some representative support medium that are amenable to the methods of the present invention include but are not limited to the following: controlled pore glass (CPG); oxalyl-controlled pore glass (see, e.g., Alul, et al., Nucleic Acids Research 1991, 19, 1527); silica-containing particles, such as porous glass beads and silica gel such as that formed by the reaction of trichloro-[3-(4-chloromethyl)phenyl]propylsilane and porous glass beads (see Parr and Grohmann, Angew. Chem. Internal. Ed. 1972, 11, 314, sold under the trademark "PORASIL E" by Waters Associates, Framingham, Mass., USA); the mono ester of 1,4-dihydroxymethylbenzene and silica (see Bayer and Jung, Tetrahedron Lett., 1970, 4503, sold under the trademark "BIOPAK" by Waters Associates); TENTAGEL (see, e.g., Wright, et al., Tetrahedron Letters 1993, 34, 3373); cross-linked styrene/divinylbenzene copolymer beaded matrix or POROS, a copolymer of polystyrene/divinylbenzene (available from Perceptive Biosystems); soluble support medium, polyethylene glycol PEG's (see Bonora et al., Organic Process Research & Development, 2000, 4, 225-231).

In particular embodiments of the invention, SM is a solid support medium.

In particular embodiments of the invention, SM is a semi-solid support medium such as polyethylene glycol, chiotosan, etc. One advantage to chitosan as a support medium is that it naturally possesses a terminal amino group, which is convenient for linking to a terminal acid group as described in reference to formula CC, above.

Further support media amenable to the present invention include without limitation PEPS support a polyethylene (PE) film with pendant long-chain polystyrene (PS) grafts (molecular weight on the order of 10⁶, (see Berg, et al., J. Am. Chem. Soc., 1989, 111, 8024 and International Patent Application WO 90/02749),). The loading capacity of the film is as high as that of a beaded matrix with the additional flexibility to accomodate multiple syntheses simultaneously. The PEPS film may be fashioned in the form of discrete, labeled sheets, each serving as an individual compartment. During all the identical steps of the synthetic cycles, the sheets are kept together in a single reaction vessel to permit concurrent preparation of a multitude of peptides at a rate close to that of a single peptide by conventional methods. Also, experiments with other geometries of the PEPS polymer such as, for example, non-woven felt, knitted net, sticks or microwellplates have not indicated any limitations of the synthetic efficacy.

Further support media amenable to the present invention include without limitation particles based upon copolymers of dimethylacrylamide cross-linked with N,N'-bisacryloylethylenediamine, including a known amount of *N*-tertbutoxycarbonyl-beta-alanyl-*N*'-acryloylhexamethylenediamine. Several spacer molecules are typically added via the beta alanyl group, followed thereafter by the amino acid residue subunits. Also, the beta alanyl-containing monomer can be replaced with an acryloyl safcosine monomer during polymerization to form resin beads. The polymerization is followed by reaction of the beads with ethylenediamine to form resin particles that contain primary amines as the covalently linked functionality. The polyacrylamide-based supports are relatively more hydrophilic than are the polystyrene-based supports and are usually used with polar aprotic solvents including dimethylformamide, dimethylacetamide, N-methylpyrrolidone and the like (see Atherton, et al., J. Am. Chem. Soc., 1975, 97, 6584, Bioorg. Chem. 1979, 8, 351, and J. C. S. Perkin 1538 (1981)).

Further support media amenable to the present invention include without limitation a composite of a resin and another material that is also substantially inert to the organic synthesis reaction conditions employed. One exemplary composite (see Scott, et al., J. Chrom. Sci., 1971, 9, 577) utilizes glass particles coated with a hydrophobic, cross-linked styrene polymer containing reactive chloromethyl groups, and is supplied by Northgate Laboratories, Inc., of Hamden, Conn., USA. Another exemplary composite contains a core of fluorinated ethylene polymer onto which has been grafted polystyrene (see Kent and Merrifield, Israel J. Chem. 1978, 17, 243 and van Rietschoten in Peptides 1974, Y. Wolman, Ed., Wiley and Sons, New York, 1975, pp. 113-116). Contiguous solid support media other than PEPS, such as cotton sheets (Lebl and Eichler, Peptide Res. 1989, 2, 232) and hydroxypropylacrylate-coated polypropylene membranes (Daniels, et al., Tetrahedron Lett. 1989, 4345). Acrylic acid-grafted polyethylene-rods and 96-microtiter wells to immobilize the growing peptide chains and to perform the compartmentalized synthesis. (Geysen, et al., Proc. Natl. Acad. Sci. USA, 1984, 81, 3998). A "tea bag" containing traditionally-used polymer beads. (Houghten, Proc. Natl. Acad. Sci. USA, 1985, 82, 5131). Simultaneous use of two different supports with different densities (Tregear, Chemistry and Biology of Peptides, J. Meienhofer, ed., Ann Arbor Sci. Publ., Ann Arbor, 1972 pp. 175-178). Combining of reaction vessels via a manifold (Gorman, Anal. Biochem., 1984, 136, 397). Multicolumn solid-phase synthesis *(e.g.,* Krchnak, et al., Int. J Peptide Protein Res., 1989, 33, 209), and Holm and Meldal, in "Proceedings of the 20th European Peptide Symposium", G. Jung and E. Bayer, eds., Walter de Gruyter & Co., Berlin, 1989 pp. 208-210). Cellulose paper (Eichler, et al., Collect. Czech. Chem. Commun., 1989, 54, 1746). Support mediated synthesis of peptides have also been reported (see, Synthetic Peptides: A User's Guide, Gregory A. Grant, Ed. Oxford University Press 1992; US-A-4,415,732; 4,458,066; 4,500,707; 4,668,777; 4,973,679; 5,132,418; 4,725,677 and Re-34,069.)

It should be understood that the compounds of the present invention depart from the state of the art in that they are useful in solid phase chemical synthesis as traceless linking moieties, i.e. no atoms are imparted to the synthetic product after cleavage from the traceless linker. In a preferred embodiment, the compounds of the present invention are particularly useful as traceless linking moieties for the synthesis of oligonucleotides on solid support medium.

Commercially available equipment routinely used for the support medium based synthesis of oligomeric compounds and related compounds is sold by several vendors including, for example, Applied Biosystems (Foster City, CA). Any other means for such synthesis known in the art may additionally or alternatively be employed. Suitable solid phase techniques, including automated synthesis techniques, are described in F. Eckstein (ed.), Oligonucleotides and Analogues, a Practical Approach, Oxford University Press, New York (1991).

The compounds of the present invention, being particularly useful as traceless linking moieties for the synthesis of oligonucleotides on solid support medium, are amenable to the standard amidite-based synthetic conditions employed in automated synthesizers.

In the context of the present invention, the term "reagent push" has the meaning of a volume of solvent that is substantially free of any active compound (i.e. reagent, activator, by-product, or other substance other than solvent), which volume of solvent is introduced to the column for the purpose, and with the effect, of pushing a reagent solution onto and through the column ahead of a subsequent reagent solution. A reagent push need not be an entire column volume, although in some cases it may include one or more column volumes. In some embodiments, a reagent push comprises at least the minimum volume necessary to substantially clear reagent, by-products and/or activator from a cross-section of the column immediately ahead of the front formed by the reagent solution used for the immediately subsequent synthetic step. An active compound, whether a reagent, by-product or activator, is considered substantially cleared if the concentration of the compound in a cross-section of the column at which the following reagent solution front is located, is low enough that it does not substantially affect the activity of the following reagent solution. The person skilled in the art will recognize that this the volume of solvent required for a "reagent push" will vary depending upon the solvent, the solubility in the solvent of the reagents, activators, by-products, etc., that are on the column, the amounts of reagents, activators, by-products, etc. that are to be cleared from the column, etc. It is considered within the skill of the artisan to select an appropriate volume for each reagent push, especially with an eye toward the Examples, below.

As used herein, unless "column wash" is otherwise modified, it has the same meaning as "reagent push." In some embodiments of the invention, column wash may imply that at least one column volume is permitted to pass through the column before the subsequent reagent solution is applied to the column. Where a column volume (CV) of the column wash is specified, this indicates that a volume of solvent equivalent to the interior volume of the unpacked column is used for the column wash.

In the context of the present invention, a wash solvent is a solvent containing substantially no active compound that is applied to a column between synthetic steps. A "wash step" is a step in which a wash solvent is applied to the column. Both "reagent push" and "column wash" are included within this definition of "wash step".

A wash solvent may be a pure chemical compound or a mixture of chemical compounds, the solvent being capable of dissolving an active compound.

In some embodiments according to the present invention, a wash solvent used in one of the wash steps may comprise some percentage of acetonitrile, not to exceed 50% v/v.

The sequence of capping and oxidation steps may be reversed, if desired. That is, capping may precede or follow oxidation. Also, with selection of a suitable thiation reagent, the oxidation and capping steps may be combined into a single step. For example, it has been surprisingly found that capping with acetic anhydride may be conducted in the presence of N,N'-dimethyldithiuram disulfide.

Various solvents may be used in the oxidation reaction. Suitable solvents are identified in the Caruthers et al. and Köster et al. patents, cited herein. The Cole et al. patent describes acetonitrile as a solvent for phenylacetyldisulfide. Other suitable solvents include toluene, xanthenes, dichloromethane, etc.

Reagents for cleaving an oligonucleotide from a support are set forth, for example, in the Caruthers et al. and Köster et al. patents, as cited herein. It is considered good practice to cleave oligonucleotide containing thymidine (T) nucleotides in the presence of an alkylated amine, such as triethylamine, when the phosphorus protecting group is O-CH₂CH₂CN, because this is now known to avoid the creation if cyano-ethylated thymidine nucleotides (CNET). Avoidance of CNET adducts is described in general in US Patent No. 6,465,628, which is incorporated herein by reference, and especially the Examples in columns 20-30, which are specifically incorporated by reference. In other preferred embodiments, one may remove phosphorus protecting groups in the presence of an alkylated amine, which effectively removes the protecting group under conditions that will not cause CNET formation (e.g. room temperature). This phosphorus deprotection step may then be followed by a wash step. The phosphorus deprotection, or optional wash step, is then followed by the cleaving step, e.g. removal of exocyclic amine protecting groups (e.g. isobutyryl and/or bezoyl groups) and cleavage of the oligonucleotide from the support under standard conditions (strong base and heat).

The oligonucleotide may be worked up by standard procedures known in the art, for example by size exclusion chromatography, high performance liquid chromatography (e.g. reverse-phase HPLC), differential precipitation, etc. In some embodiments according to the present invention, the oligonucleotide is cleaved from a solid support medium while the 5'-OH protecting group is still on the ultimate nucleoside. This so-called DMT-on (or trityl-on) oligonucleotide is then subjected to chromatography, after which the DMT group is removed by treatment in an organic acid, after which the oligonucleotide is de-salted and further purified to form a final product. This procedure will also work well where the acid-labile protecting group is pixyl or substituted pixyl, as the pixyl-on oligonucleotide is conveniently separated from capped shortmer (failure) sequences by reverse phase HPLC.

The 5'-hydroxyl protecting groups may be any groups that are selectively removed under suitable conditions. In particular, the 4,4'-dimethoxytriphenylmethyl (DMT) group is a favored group for protecting at the 5'-position, because it is readily cleaved under acidic conditions (e.g. in the presence of dichloracetic acid (DCA), trichloroacetic acid (TCA), or acetic acid. Removal of DMT from the support-bound oligonucleotide is generally performed with DCA (e.g. about 3 to about 10 percent DCA (v/v) in a suitable solvent. Removal of oligonucleotide after cleavage from the support is generally performed with acetic acid. Where the 5-hydroxyl protecting group is pixyl or substituted pixyl, an acid with a higher pKa than DCA may be used, as pixyl groups are generally labile to higher pKa acids than are trityl groups. In some embodiments, pixyl or substituted pixyl groups may be removed with acetic acid.

As described herein, oligonucleotides can be prepared as chimeras with other oligomeric moieties. In the context of this invention, the term "oligomeric compound" refers to a polymeric structure capable of hybridizing a region of a nucleic acid molecule, and an "oligomeric moiety" a portion of such an oligomeric compound. Oligomeric compounds include oligonucleotides, oligonucleosides, oligonucleotide analogs, modified oligonucleotides and oligonucleotide mimetics. Oligomeric compounds can be linear or circular, and may include branching. They can be single stranded or double stranded, and when double stranded, may include overhangs. In general an oligomeric compound comprises a backbone of linked monomeric subunits where each linked monomeric subunit is directly or indirectly attached to a heterocyclic base moiety. The linkages joining the monomeric subunits, the monomeric subunits and the heterocyclic base moieties can be variable in structure giving rise to a plurality of motifs for the resulting oligomeric compounds including hemimers, gapmers and chimeras. As is known in the art, a nucleoside is a base-sugar combination. The base portion of the nucleoside is normally a heterocyclic base moiety. The two most common classes of such heterocyclic bases are purines and pyrimidines. In the context of this invention, the term " oligonucleoside" refers to nucleosides that are joined by internucleoside linkages that do not have phosphorus atoms. Internucleoside linkages of this type include short chain alkyl, cycloalkyl, mixed heteroatom alkyl, mixed heteroatom cycloalkyl, one or more short chain heteroatomic and one or more short chain heterocyclic. These internucleoside linkages include but are not limited to siloxane, sulfide, sulfoxide, sulfone, acetyl, formacetyl, thioformacetyl, methylene formacetyl, thioformacetyl, alkeneyl, sulfamate; methyleneimino, methylenehydrazino, sulfonate, sulfonamide, amide and others having mixed N, O, S and CH₂ component parts.

Phosphoramidites used in the synthesis of oligonucleotides are available from a variety of commercial sources (included are: Glen Research, Sterling, Virginia; Amersham Pharmacia Biotech Inc., Piscataway, New Jersey; Cruachem Inc., Aston, Pennsylvania; Chemgenes Corporation, Waltham, Massachusetts; Proligo LLC, Boulder, Colorado; PE Biosystems, Foster City California; Beckman Coulter Inc., Fullerton, California). These commercial sources sell high purity phosphoramidites generally having a purity of better than 98%. Those not offering an across the board purity for all amidites sold will in most cases include an assay with each lot purchased giving at least the purity of the particular phosphoramidite purchased. Commercially available phosphoramidites are prepared for the most part for automated DNA synthesis and as such are prepared for immediate use for synthesizing desired sequences of oligonucleotides. Phosphoramidites may be prepared by methods disclosed by e.g. Caruthers et al. (US 4,415,732; 4,458,066; 4,500,707; 4,668,777; 4,973,679; and 5,132,418) and Köster et al. (US RE 34,069).

Double stranded oligonucleotides, such as double-stranded RNA, may be manufactured according to methods according to the present invention, as described herein. In the case of RNA synthesis, it is necessary to protect the 2'-OH of the amidite reagent with a suitable removable protecting groups. Suitable protecting groups for 2'-OH are described in US Patent Nos. 6,008,400, 6,111,086 and 5,889,136. A particularly suitable 2'-protecting group for RNA synthesis is the ACE protecting group as described in US 6,111,086. In some embodiments, it is considered advantageous to use a different 5'-protecting group for amidites used in RNA synthesis. Suitable 5'-protecting groups are set forth in US 6,008,400. A particularly suitable 5'-protecting group is the trimethylsilyloxy (TMSO) group as taught in US 6,008,400. See especially example 1, columns 10-13. The separate strands of the double stranded RNA may be separately synthesized and then annealed to form the double stranded (duplex) oligonucleotide.

### Oligonucleotide Use

Exemplary preferred antisense compounds include DNA or RNA sequences that comprise at least the 8 consecutive nucleobases from the 5'-terminus of one of the illustrative preferred antisense compounds (the remaining nucleobases being a consecutive stretch of the same DNA or RNA beginning immediately upstream of the 5'-terminus of the antisense compound which is specifically hybridizable to the target nucleic acid and continuing until the DNA or RNA contains about 8 to about 80 nucleobases). Similarly preferred antisense compounds are represented by DNA or RNA sequences that comprise at least the 8 consecutive nucleobases from the 3'-terminus of one of the illustrative preferred antisense compounds (the remaining nucleobases being a consecutive stretch of the same DNA or RNA beginning immediately downstream of the 3' terminus of the antisense compound which is specifically hybridizable to the target nucleic acid and continuing until the DNA or RNA contains about 8 to about 80 nucleobases). One having skill in the art, once armed with the empirically-derived preferred antisense compounds illustrated herein will be able, without undue experimentation, to identify further preferred antisense compounds.

Antisense and other compounds of the invention, which hybridize to the target and inhibit expression of the target, are identified through experimentation, and representative sequences of these compounds are herein identified as preferred embodiments of the invention. While specific sequences of the antisense compounds are set forth herein, one of skill in the art will recognize that these serve to illustrate and describe particular embodiments within the scope of the present invention. Additional preferred antisense compounds may be identified by one having ordinary skill.

Specific examples of preferred antisense compounds useful in this invention include oligonucleotides containing modified backbones or non-natural internucleoside linkages. As defined in this specification, oligonucleotides having modified backbones include those that retain a phosphorus atom in the backbone and those that do not have a phosphorus atom in the backbone. For the purposes of this specification, and as sometimes referenced in the art, modified oligonucleotides that do not have a phosphorus atom in their internucleoside backbone can also be considered to be oligonucleosides.

### RNAse H-Dependent Antisense

One method for inhibiting specific gene expression involves using oligonucleotides or oligonucleotide analogs as "antisense" agents. Antisense technology involves directing oligonucleotides, or analogs thereof, to a specific, target messenger RNA (mRNA) sequence. The interaction of exogenous "antisense" molecules and endogenous mRNA modulates transcription by a variety of pathways. Such pathways include transcription arrest, RNAse H recruitment, and RNAi (e.g. siRNA). Antisense technology permits modulation of specific protein activity in a relatively predictable manner.

### EXAMPLES

The present invention may be further understood with reference to the following, no-limiting, illustrative examples, which may be carried out by methods generally described hereinabove. 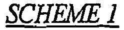 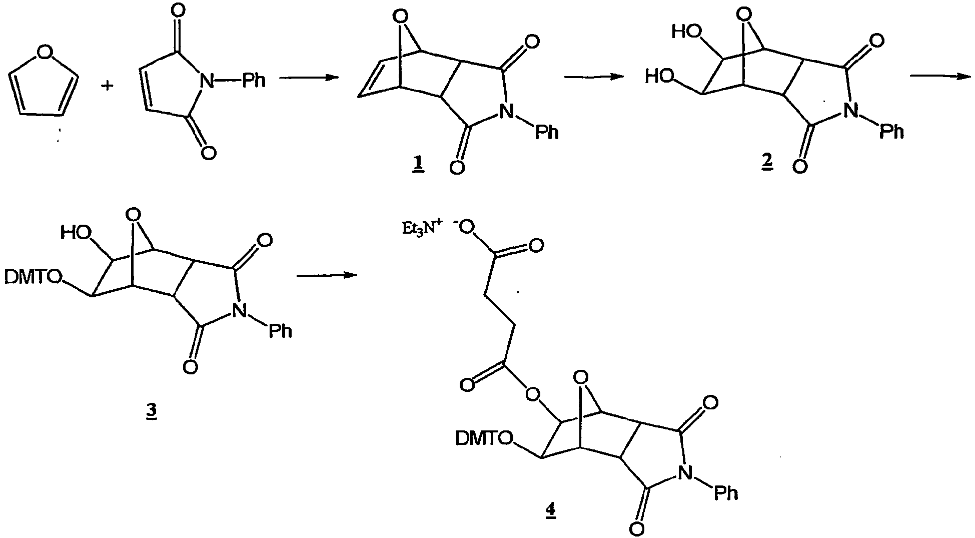 Experiment **1**: Diels-Alder reaction between furan and N-phenylmaleimide to give adduct **1**: *N-*Phenylmaleimide (500 g; 2.887 mole) was taken in acetonitrile (1600 mL) and furan (500 mL) was added and heated under reflux using a heating mantle and ice-water cooling condenser in a 5 L three-necked round bottomed flask provided with magnetic stirring. After refluxing for 5 hours, the reaction mixture was analyzed by HPLC for absence of starting material (viz N-phenyl maleimide). Then the reaction mixture was cooled to room temperature when colorless solid precipitates out. The material was filtered, washed with acetonitrile (500 mL). The filtrate solution was concentrated to afford more of product which was also filtered and washed with acetonitrile (300 mL). The solid **1** was dried under high vacuum at room temperature overnight. Yield: 541 g (78%). ¹H NMR (DMSO-d6): 3.055 (s, 2H), 5.223 (s, 2H), 6.580 (s, 2H), 7-18-7.58 (m, 5H).

Experiment 2: Osmium tetroxide catalyst solution: The content of a lg sealed vial of osmium tetroxide was dissolved in 200 mL of purified t-butyl alcohol. The pale green solution was treated with 3-5 drops of 30% hydrogen peroxide and allowed to remain at room temperature for 1 day. If the solution became dark, the dropwise addition of 30% hydrogen peroxide was repeated until the pale green color persisted. This solution is stabile for at least one year at room temperature. Each mL contains 2 x 10⁻⁵ mole of osmium tetroxide.

Experiment 3: cis-Dihydroxylation of Diels-Alder adduct to give diol **2**: The olefin **1** obtained from above Diels-Alder reaction (225 g, 0.934 mole) was taken in a 5 L three-necked flask fitted with a mechanical stirrer, reflux condenser with ice-water cooling and a heating mantle. Acetone (2500 mL) was added and stirred. A 30% Hydrogen peroxide solution (500 mL) was added followed by osmium tetroxide solution prepared earlier (180 mL). Warning: For larger scales the reaction could be exothermic!! Slow addition (1-2 hour) of osmium tetroxide solution is recommended. Gentle refluxing of reaction mixture with stirring was maintained for 7-8 h. During the period, reaction color changed from brown to pale brown to colorless and solid started crashing out. Vigorous stirring was maintained through out the period. The reaction mixture was analyzed by HPLC for absence of starting material. The reaction mixture was cooled to room temperature and filtered. The solid **2** was washed with ether (2000 mL) and dried in vacuum oven at room temperature overnight. The acetone filtrate solution was concentrated and ether (1000 mL) was added when solid precipitated out which was filtered, washed with ether (300 ml) and dried in over at 45 deg C or two days. Yield of **2** = 179 g (first crop) + 32 g (second crop) = 211 g (82%). ¹H NMR (DMSO-d6): 3.14 (s, 2H), 3.88 (d, 2H), 4.39 (s, 2H), 5.1 (d, 2H), 7-18-7.58 (m, 5H).

Experiment 4: Mono protection of diol with DMT chloride to form **3**: The dihydroxy compound **3** (FW 275; 275 g; 1 mole) was taken in a 5 L round-bottomed flask and co-evaporated with anhydrous pyridine (1200 mL). This step was repeated one more time to render the diol anhydrous. Pyridine (3000 mL) was added and stirred using magnetic stirrer at room temperature. Dimethoxytrityl chloride (FW 338.82; 508.2 g, 1.5 equivalents) was slowly added as solid over a period of 2 hours. Solution was stirred overnight. Tlc indicated almost disappearance of starting material. All volatiles were removed under vacuum using rotavap. Toluene (2000 mL) was added and rotavaped. This step was repeated one more time. The remaining crude material was purified by flash silica gel chromatography using hexane, 20% ethyl acetate-hexane, then 40% ethyl acetate-hexane and finally 100% ethyl acetate. 1% Triethylamine was used through out purification. Yield of **3**: 398 g (71%).

**Experiment 5**: Succinylation of DMT protected compound **3**: DMT protected hydroxy compound **3** (FW 578; 49.13 g; 85 mmole) was dissolved in a mixture ethyl acetate : methylene chloride (600 : 66 = 666 mL). Triethyl amine (FW 101.19; 51.61 g; 71 mL; 0.51 mole; 6 equivalent with respect to starting DMT compound) was added and stirred magnetically at room temperature. To this clear solution, succinic anhydride (FW 100.07; 34.02 g; 0.34 mole, 4 equivalents with respect to starting DMT compound) was added as solid all at once. Stirring was continued overnight. TLC indicated disappearance of starting material. If starting material is seen, more of succinic anhydride is added till completion of reaction. The reaction mixture was diluted with ethyl acetate (300 mL) and washed with water (2 x 200 mL), brine (120 mL) and dried with magnesium sulfate. If the product is colored, the material is passed through a short pad of silica gel eluting with methylene chloride and then 5% methanol:95% methylene chloride to afford the product as a colorless product. Yield of **4**: 60.5 g (91%).

Experiment 6: Synthesis of substituted pixyl alcohol: To a stirred mixture of 4',4'-dimethyldiphenylether (200 g; 1.01 mole), p-methybenzoic acid (154 g; 1.13 mole) and anhydrous zinc chloride (400 g; 2.94 mole) was added phosphorousoxy trichloride (300 ml; 3.27 mole) slowly using an addition funnel. The reaction mixture was then slowly heated to 95 °C when the reaction starts and monitored by tlc. After the reaction is complete, ethyl acetate (500 ml) was added, followed by water (200 ml) slowly. An additional amount of water (2500 ml) was added at a faster rate. Stirred overnight at room temperature when solid comes out. It was filtered and recrystallized from methanol to afford the substituted pixyl alcohol product.

Experiment 7: Synthesis of substituted pixyl chloride: To a stirred solution of substituted pixyl alcohol (310 g; 0.982 mole) in dichloromethane (1000 ml) was added thionyl chloride (102 ml; **1**.1 mole) slowly with cooling. The reaction was monitored by tlc. When complete, the reaction was concentrated, toluene added followed by hexane to afford the desired product as colorless solid.

Experiment 8 Mono protection of diol **2** with substituted pixyl chloride to form the substituted pixyl analog of **3 (3')**: The dihydroxy compound (FW 275; 0.1 mole) was taken in a round-bottomed flask and co-evaporated with anhydrous pyridine. This step was repeated one more time to render the diol anhydrous. Pyridine (200 mL) was added and stirred using magnetic stirrer at room temperature. Trimethyl substituted pixyl chloride (1.5 equivalents) was slowly added as solid over a period of 30 minutes. Solution was stirred overnight. Tlc indicated almost disappearance of starting material. All volatiles were removed under vacuum using rotavap. Toluene was added and rotavaped. This step was repeated one more time. The remaining crude material was purified by flash silica gel chromatography using hexane, 20% ethyl acetate-hexane, then 40% ethyl acetate-hexane and finally 100% ethyl acetate. 1% Triethylamine was used through out purification. The product **3'** was obtained as a colorless solid.

Experiment 9: Succinylation of substituted pixyl protected compound 3' to form the substituted pixyl analog of **4 (4')**: Substituted pixyl protected hydroxy compound **3'** (85 mmole) was dissolved in a mixture ethyl acetate : methylene chloride (600 : 66 = 666 mL). Triethyl amine (FW 101.19; 51.61 g; 71 mL; 0.51 mole; 6 equivalent with respect to starting compound) was added and stirred magnetically at room temperature. To this clear solution, succinic anhydride (FW 100.07; 34.02 g; 0.34 mole, 4 equivalents with respect to starting compound) was added as solid all at once. Stirring was continued overnight. TLC indicated disappearance of starting material. If starting material is seen, more of succinic anhydride is added till completion of reaction. The reaction mixture was diluted with ethyl acetate (300 mL) and washed with water (2 x 200 mL), brine (120 mL) and dried with magnesium sulfate. If the product is colored, the material is passed through a short pad of silica gel eluting with methylene chloride and then 5% methanol:95% methylene chloride to afford the product as a colorless product. Yield of **4'**: 93%.

Experiment 10: Loading of DMT protected succinate **4** to controlled pore glass: Loading of the succinate molecule was performed similar to nucleoside succinate using HBTU as activator and Hunig's base in acetonitrile as solvent. The unreacted sites were capped with acetic anhydride in pyridine in presence of DMAP as catalyst. Loading was then checked using the standard UV method. Loading = 40 micromole/gram.

Experiment 11: Loading of substituted pixyl protected succinate **4'** to controlled pore glass: Loading of the succinate molecule was performed similar to nucleoside succinate using HBTU as activator and Hunig's base in acetonitrile as solvent. The unreacted sites were capped with acetic anhydride in pyridine in presence of DMAP as catalyst. Loading was then checked using the standard UV method. Loading = 38 micromole/gram.

Experiment 12: Loading of DMT protected succinate **4** to HL30 amino-derivatized primer support: Loading of the succinate molecule was performed similar to nucleoside succinate using HBTU as activator and Hunig's base in acetonitrile as solvent. The unreacted sites were capped with acetic anhydride in pyridine in presence of DMAP as catalyst. Loading was then checked using the standard UV method. Loading = 90 micromole/gram.

Experiment 13: Loading of substituted pixyl protected succinate **4'** to HL30 amino-derivatized primer support: Loading of the succinate molecule was performed similar to nucleoside succinate using HBTU as activator and Hunig's base in acetonitrile as solvent. The unreacted sites were capped with acetic anhydride in pyridine in presence of DMAP as catalyst. Loading was then checked using the standard UV method. Loading = 93 micromole/gram.

Experiment 14: Loading of DMT protected succinate **4** to OligoPrep: Loading of the succinate molecule was performed similar to nucleoside succinate using HBTU as activator and Hunig's base in acetonitrile as solvent. The unreacted sites were capped with acetic anhydride in pyridine in presence of DMAP as catalyst. Loading was then checked using the standard UV method. Loading = 244 micromole/gram.

Experiment 15: Loading of substituted pixyl protected succinate **4'** to OligoPrep: Loading of the succinate molecule was performed similar to nucleoside succinate using HBTU as activator and Hunig's base in acetonitrile as solvent. The unreacted sites were capped with acetic anhydride in pyridine in presence of DMAP as catalyst. Loading was then checked using the standard UV method. Loading = 253 micromole/gram.

Experiment 16: Loading of DMT protected succinate **4** to Nittomar 250 solid support: Loading of the succinate molecule was performed similar to nucleoside succinate using HBTU as activator and Hunig's base in acetonitrile as solvent. The unreacted sites were capped with acetic anhydride in pyridine in presence of DMAP as catalyst. Loading was then checked using the standard UV method. Loading = 240 micromole/gram.

Experiment 17: Loading of substituted pixyl protected succinate **4'** to Nittomar 250 solid support: Loading of the succinate molecule was performed similar to nucleoside succinate using HBTU as activator and Hunig's base in acetonitrile as solvent. The unreacted sites were capped with acetic anhydride in pyridine in presence of DMAP as catalyst. Loading was then checked using the standard UV method. Loading = 250 micromole/gram.

Experiment 18: Synthesis of fully-modified 5'-d(TCC-CGC-CTG-TGA-CAT-GCA-TT)-3' phosphorothioate 20-mer using **4**: Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

### Experiment 19: Synthesis of fully-modified 5'-d(TCC-CGC-CTG-TGA-CAT-GCA-TT)-3' phosphorothioate 20-mer using 4':

Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 20: Synthesis of fully-modified 5'-d(GCC-CAA-GCT-GGC-ATC-CGT-CA)-3' phosphorothioate 20-mer using **4**: Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 21: Synthesis of fully-modified 5'-d(GCC-CAA-GCT-GGC-ATC-CGT-CA)-3' phosphorothioate 20-mer using **4'**: Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 22: Synthesis of 5'-d(TCC-CGC-CTG-TGA-CAT-GCA-TT)-3' DNA 20-mer using **4**: Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Oxidation was performed using a solution of iodine in THF/water/pyridine as recommended by ABI manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

### Experiment 23: Synthesis of 5'-d(TCC-CGC-CTG-TGA-CAT-GCA-TT)-3' DNA 20-mer using 4':

Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Oxidation was performed using a solution of iodine in THF/water/pyridine as recommended by ABI manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 24: Synthesis of 5'-d(GCC-CAA-GCT-GGC-ATC-CGT-CA)-3' DNA 20-mer using **4**: Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Oxidation was performed using a solution of iodine in THF/water/pyridine as recommended by ABI manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 25: Synthesis of 5'-d(GCC-CAA-GCT-GGC-ATC-CGT-CA)-3' DNA 20-mer using **4'**: Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Oxidation was performed using a solution of iodine in THF/water/pyridine as recommended by ABI manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 26: Synthesis of fully-modified 5'-d(TCC-CGC-CTG-TGA-CAT-GCA-TT)-3' phosphorothioate 20-mer using **4**: Synthesis of above sequence was performed on an Amersham Biosciences' Akta OligoPilot DNA/RNA Synthesizer on a 172 micromole scale using cyanoethyl phosphoramidites and the above prepared HL30 Primer solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 27: Synthesis of fully-modified 5'-d(TCC-CGC-CTG-TGA-CAT-GCA-TT)-3' phosphorothioate 20-mer using **4'**: Synthesis of above sequence was performed on an Amersham Biosciences' Akta OligoPilot DNA/RNA Synthesizer on a 175 micromole scale using cyanoethyl phosphoramidites and the above prepared HL30 Primer solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 28: Synthesis of fully-modified 5'-d(GCC-CAA-GCT-GGC-ATC-CGT-CA)-3' phosphorothioate 20-mer using **4**: Synthesis of above sequence was performed on an Amersham Biosciences' Akta OligoPilot DNA/RNA Synthesizer on a 178 micromole scale using cyanoethyl phosphoramidites and the above prepared HL30 Primer solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 29: Synthesis of fully-modified 5'-d(GCC-CAA-GCT-GGC-ATC-CGT-CA)-3' phosphorothioate 20-mer using **4'**: Synthesis of above sequence was performed on an Amersham Biosciences' Akta OligoPilot DNA/RNA Synthesizer on a 181 micromole scale using cyanoethyl phosphoramidites and the above prepared HL30 Primer solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 30: Synthesis of 5'-d(TCC-CGC-CTG-TGA-CAT-GCA-TT)-3' DNA 20-mer using **4**: Synthesis of above sequence was performed on an Amersham Biosciences' Akta OligoPilot DNA/RNA Synthesizer on a 184 micromole scale using cyanoethyl phosphoramidites and the above prepared HL30 Primer solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 31: Synthesis of 5'-d(TCC-CGC-CTG-TGA-CAT-GCA-TT)-3' DNA 20-mer using **4'**: Synthesis of above sequence was performed on an Amersham Biosciences' Akta OligoPilot DNA/RNA Synthesizer on a 179 micromole scale using cyanoethyl phosphoramidites and the above prepared HL30 Primer solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 32: Synthesis of 5'-d(GCC-CAA-GCT-GGC-ATC-CGT-CA)-3' DNA 20-mer using 4: Synthesis of above sequence was performed on an Amersham Biosciences' Akta OligoPilot DNA/RNA Synthesizer on a 180 micromole scale using cyanoethyl phosphoramidites and the above prepared HL30 Primer solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 33: Synthesis of 5'-d(GCC-CAA-GCT-GGC-ATC-CGT-CA)-3' DNA 20-mer using **4'**: Synthesis of above sequence was performed on an Amersham Biosciences' Akta OligoPilot DNA/RNA Synthesizer on a 169 micromole scale using cyanoethyl phosphoramidites and the above prepared HL30 Primer solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 34: Synthesis of fully-modified 5'-d(TCC-CGC-CTG-TGA-CAT-GCA-TT)-3' phosphorothioate 20-mer using **4**: Synthesis of above sequence was performed on an Amersham Biosciences' Akta OligoPilot DNA/RNA Synthesizer on a 172 micromole scale using cyanoethyl phosphoramidites and the above prepared OligoPrep solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 35: Synthesis of fully-modified 5'-d(TCC-CGC-CTG-TGA-CAT-GCA-TT)-3' phosphorothioate 20-mer using **4'**: Synthesis of above sequence was performed on an Amersham Biosciences' Akta OligoPilot DNA/RNA Synthesizer on a 175 micromole scale using cyanoethyl phosphoramidites and the above prepared OligoPrep solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 36: Synthesis of fully-modified 5'-d(GCC-CAA-GC'T-GGC-ATC-CGT-CA)-3' phosphorothioate 20-mer using **4**: Synthesis of above sequence was performed on an Amersham Biosciences' Akta OligoPilot DNA/RNA Synthesizer on a 178 micromole scale using cyanoethyl phosphoramidites and the above prepared OligoPrep solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 37: Synthesis of fully-modified 5'-d(GCC-CAA-GCT-GGC-ATC-CGT-CA)-3' phosphorothioate 20-mer using **4'**: Synthesis of above sequence was performed on an Amersham Biosciences' Akta OligoPilot DNA/RNA Synthesizer on a 181 micromole scale using cyanoethyl phosphoramidites and the above prepared OligoPrep solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 38: Synthesis of 5'-d(TCC-CGC-CTG-TGA-CAT-GCA-TT)-3' DNA 20-mer using **4**: Synthesis of above sequence was performed on an Amersham Biosciences' Akta OligoPilot DNA/RNA Synthesizer on a 184 micromole scale using cyanoethyl phosphoramidites and the above prepared OligoPrep solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

### Experiment 39: Synthesis of 5'-d(TCC-CGC-CTG-TGA-CAT-GCA-TT)-3' DNA 20-mer using 4':

Synthesis of above sequence was performed on an Amersham Biosciences' Akta OligoPilot DNA/RNA Synthesizer on a 179 micromole scale using cyanoethyl phosphoramidites and the above prepared OligoPrep solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 40: Synthesis of 5'-d(GCC-CAA-GCT-GGC-ATC-CGT-CA)-3' DNA 20-mer using **4**: Synthesis of above sequence was performed on an Amersham Biosciences' Akta OligoPilot DNA/RNA Synthesizer on a 180 micromole scale using cyanoethyl phosphoramidites and the above prepared OligoPrep solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

### Experiment 41: Synthesis of 5'-d(GCC-CAA-GCT-GGC-ATC-CGT-CA)-3' DNA 20-mer using 4':

Synthesis of above sequence was performed on an Amersham Biosciences' Akta OligoPilot DNA/RNA Synthesizer on a 169 micromole scale using cyanoethyl phosphoramidites and the above prepared OligoPrep solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 42: Synthesis of fully-modified 5'-d(TCC-CGC-CTG-TGA-CAT-GCA-TT)-3' phosphorothioate 20-mer using **4**: Synthesis of above sequence was performed on an Amersham Biosciences' Akta OligoPilot DNA/RNA Synthesizer on a 172 micromole scale using cyanoethyl phosphoramidites and the above prepared Nittomar 250 solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 43: Synthesis of fully-modified 5'-d(TCC-CGC-CTG-TGA-CAT-GCA-TT)-3' phosphorothioate 20-mer using **4'**: Synthesis of above sequence was performed on an Amersham Biosciences' Akta OligoPilot DNA/RNA Synthesizer on a 175 micromole scale using cyanoethyl phosphoramidites and the above prepared Nittomar 250 solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 44: Synthesis of fully-modified 5'-d(GCC-CAA-GCT-GGC-ATC-CGT-CA)-3' phosphorothioate 20-mer using **4**: Synthesis of above sequence was performed on an Amersham Biosciences' Akta OligoPilot DNA/RNA Synthesizer on a 178 micromole scale using cyanoethyl phosphoramidites and the above prepared Nittomar 250 solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 45: Synthesis of fully-modified 5'-d(GCC-CAA-GCT-GGC-ATC-CGT-CA)-3' phosphorothioate 20-mer using **4'**: Synthesis of above sequence was performed on an Amersham Biosciences' Akta OligoPilot DNA/RNA Synthesizer on a 181 micromole scale using cyanoethyl phosphoramidites and the above prepared Nittomar 250 solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 46: Synthesis of 5'-d(TCC-CGC-CTG-TGA-CAT-GCA-TT)-3' DNA 20-mer using **4**: Synthesis of above sequence was performed on an Amersham Biosciences' Akta OligoPilot DNA/RNA Synthesizer on a 184 micromole scale using cyanoethyl phosphoramidites and the above prepared Nittomar 250 solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 47: Synthesis of 5'-d(TCC-CGC-CTG-TGA-CAT-GCA-TT)-3' DNA 20-mer using **4'**: Synthesis of above sequence was performed on an Amersham Biosciences' Akta OligoPilot DNA/RNA Synthesizer on a 179 micromole scale using cyanoethyl phosphoramidites and the above prepared Nittomar 250 solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 48: Synthesis of 5'-d(GCC-CAA-GCT-GGC-ATC-CGT-CA)-3' DNA 20-mer using **4**: Synthesis of above sequence was performed on an Amersham Biosciences' Akta OligoPilot DNA/RNA Synthesizer on a 180 micromole scale using cyanoethyl phosphoramidites and the above prepared Nittomar 250 solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 49: Synthesis of 5'-d(GCC-CAA-GCT-GGC-ATC-CGT-CA)-3' DNA 20-mer using **4'**: Synthesis of above sequence was performed on an Amersham Biosciences' Akta OligoPilot DNA/RNA Synthesizer on a 169 micromole scale using cyanoethyl phosphoramidites and the above prepared Nittomar 250 solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 50: Synthesis of fully-modified 5'-[2'-*O*-methoxyethyl-(TGTG]-d(CTA-TTC-TGT-G-)-[2'-O-methoxyethyl-(AATT]-3' phosphorothioate 18-mer using **4**: Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 51: Synthesis of fully-modified 5'-[2'-*O*-methoxyethyl-(TGTG]-d(CTA-TTC-TGT-G-)-[2'-O-methoxyethyl-(AATT]-3' phosphorothioate 18-mer using **4'**: Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 52: Synthesis of fully-modified 5'-[2'-*O*-methoxyethyl-(GCCTC]-d(AGT-CTG-CTT-C-)-[2'-O-methoxyethyl-(GCACC]-3' phosphorothioate 20-mer using **4**: Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 53: Synthesis of fully-modified 5'-[2'-*O*-methoxyethyl-(GCCTC]-d(AGT-CTG-CTT-C-)-[2'-O-methoxyethyl-(GCACC]-3' phosphorothioate 20-mer using **4'**: Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 54: Synthesis of 5'-[2'-*O*-methoxyethyl-(TGTG]-d(CTA-TTC-TGT-G-)-[2'-O-methoxyethyl-(AATT]-3' 18-mer phosphate diester using **4**: Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Oxidation was performed using a solution of iodine in THF/water/pyridine as recommended by ABI manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 55: Synthesis of 5'-[2'-*O*-methoxyethyl-(TGTG]-d(CTA-TTC-TGT-G-)-[2'-O-methoxyethyl-(AATT]-3' 18-mer phosphate diester using **4'**: Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Oxidation was performed using a solution of iodine in THF/water/pyridine as recommended by ABI manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 56: Synthesis of 5'-[2'-*O*-methoxyethyl-(GCCTC]-d(AGT-CTG-CTT-C-)-[2'-O-methoxyethyl-(GCACC]-3' 20-mer phosphate diester using **4**: Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Oxidation was performed using a solution of iodine in THF/water/pyridine as recommended by ABI manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 57: Synthesis of 5'-[2'-*O*-methoxyethyl-(GCCTC]-d(AGT-CTG-CTT-C-)-[2'-O-methoxyethyl-(GCACC]-3' 20-mer phosphate diester using **4'**: Synthesis of above sequence was performed on an ABI 390Z DNA/RNA Synthesizer on a 15 micromole scale using cyanoethyl phosphoramidites and the above prepared CPG solid support. Detritylation was performed using 3% dichloroacetic acid in toluene (volume/volume). Oxidation was performed using a solution of iodine in THF/water/pyridine as recommended by ABI manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 58: Synthesis of fully-modified 5'-[2'-*O*-methoxyethyl-(TGTG]-d(CTA-TTC-TGT-G-)-[2'-O-methoxyethyl-(AATT]-3' phosphorothioate 18-mer using **4**: Synthesis of above sequence was performed on an Amersham Biosciences' Akta OligoPilot DNA/RNA Synthesizer on a 172 micromole scale using cyanoethyl phosphoramidites and the above prepared HL30 Primer solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 59: Synthesis of fully-modified 5'-[2'-*O*-methoxyethyl-(TGTG]-d(CTA-TTC-TGT-G-)-[2'-O-methoxyethyl-(AATT]-3' phosphorothioate 18-mer using **4'**: Synthesis of above sequence was performed on an Amersham Biosciences' Akta OligoPilot DNA/RNA Synthesizer on a 175 micromole scale using cyanoethyl phosphoramidites and the above prepared HL30 Primer solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 60: Synthesis of fully modified 5'-[2'-*O*-methoxyethyl-(GCCTC]-d(AGT-CTG-CTT-C-)-[2'-O-methoxyethyl-(GCACC]-3' phosphorothioate 20-mer using **4**: Synthesis of above sequence was performed on an Amersham Biosciences' Akta OligoPilot DNA/RNA Synthesizer on a 178 micromole scale using cyanoethyl phosphoramidites and the above prepared HL30 Primer solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 61: Synthesis of fully-modified 5'-[2'-*O*-methoxyethyl-(GCCTC]-d(AGT-CTG-CTT-C-)-[2'-O-methoxyethyl-(GCACC]-3' phosphorothioate 20-mer using **4'**: Synthesis of above sequence was performed on an Amersham Biosciences' Akta OligoPilot DNA/RNA Synthesizer on a 181 micromole scale using cyanoethyl phosphoramidites and the above prepared HL30 Primer solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 62: Synthesis of 5-[2'-*O*-methoxyethyl-(TGTG]-d(CTA-TrC-TGT-G-)-[2'-O-methoxyethyl-(AATT]-3' 18-mer phosphate diester using **4**: Synthesis of above sequence was performed on an Amersham Biosciences' Akta OligoPilot DNA/RNA Synthesizer on a 184 micromole scale using cyanoethyl phosphoramidites and the above prepared HL30 Primer solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 63: Synthesis of 5'-[2'-*O*-methoxyethyl-(TGTG]-d(CTA-TTC-TGT-G-)-[2'-O-methoxyethyl-(AATT]-3' 18-mer phosphate diester using **4'**: Synthesis of above sequence was performed on an Amersham Biosciences' Akta OligoPilot DNA/RNA Synthesizer on a 179 micromole scale using cyanoethyl phosphoramidites and the above prepared HL30 Primer solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 64: Synthesis of 5'-[2'-*O*-methoxyethyl-(GCCTC]-d(AGT-CTG-CTT-C-)-[2'-O-methoxyethyl-(GCACC]-3' 20-mer phosphate diester using **4**: Synthesis of above sequence was performed on an Amersham Biosciences' Akta OligoPilot DNA/RNA Synthesizer on a 180 micromole scale using cyanoethyl phosphoramidites and the above prepared HL30 Primer solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 65: Synthesis of 5'-[2'-*O*-methoxyethyl-(GCCTC]-d(AGT-CTG-CTT-C-)-[2'-O-methoxyethyl-(GCACC]-3' 20-mer phosphate diester using **4'**: Synthesis of above sequence was performed on an Amersham Biosciences' Akta OligoPilot DNA/RNA Synthesizer on a 169 micromole scale using cyanoethyl phosphoramidites and the above prepared HL30 Primer solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 66: Synthesis of fully-modified 5'-[2'-*O*-methoxyethyl-(TGTG]-d(CTA-TTC-TGT-G-)-[2'-O-methoxyethyl-(AATT]-3' phosphorothioate 18-mer using **4**: Synthesis of above sequence was performed on an Amersham Biosciences' Akta OligoPilot DNA/RNA Synthesizer on a 172 micromole scale using cyanoethyl phosphoramidites and the above prepared OligoPrep solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 67: Synthesis of fully-modified 5'-[2'-*O*-methoxyethyl-(TGTG]-d(CTA-TTC-TGT-G-)-[2'-O-methoxyethyl-(AATT]-3' phosphorothioate 18-mer using **4'**: Synthesis of above sequence was performed on an Amersham Biosciences' Akta OligoPilot DNA/RNA Synthesizer on a 175 micromole scale using cyanoethyl phosphoramidites and the above prepared OligoPrep solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 68: Synthesis of fully modified 5'-[2'-*O*-methoxyethyl-(GCCTC]-d(AGT-CTG-CTT-C-)-[2'-O-methoxyethyl-(GCACC]-3' phosphorothioate 20-mer using **4**: Synthesis of above sequence was performed on an Amersham Biosciences' Akta OligoPilot DNA/RNA Synthesizer on a 178 micromole scale using cyanoethyl phosphoramidites and the above prepared OligoPrep solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 69: Synthesis of fully modified 5'-[2'-*O*-methoxyethyl-(GCCTC]-d(AGT-CTG-CTT-C-)-[2'-O-methoxyethyl-(GCACC]-3' phosphorothioate 20-mer using **4'**: Synthesis of above sequence was performed on an Amersham Biosciences' Akta OligoPilot DNA/RNA Synthesizer on a 181 micromole scale using cyanoethyl phosphoramidites and the above prepared OligoPrep solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 70: Synthesis of 5'-[2'-*O*-methoxyethyl-(TGTG]-d(CTA-TTC-TGT-G-)-[2'-O-methoxyethyl-(AATT]-3' 18-mer phosphate diester using **4**: Synthesis of above sequence was performed on an Amersham Biosciences' Akta OligoPilot DNA/RNA Synthesizer on a 184 micromole scale using cyanoethyl phosphoramidites and the above prepared OligoPrep solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 71: Synthesis of 5'-[2'-*O*-methoxyethyl-(TGTG]-d(CTA-TTC-TGT-G-)-[2'-O-methoxyethyl-(AATT]-3' 18-mer phosphate diester using **4'**: Synthesis of above sequence was performed on an Amersham Biosciences' Akta OligoPilot DNA/RNA Synthesizer on a 179 micromole scale using cyanoethyl phosphoramidites and the above prepared OligoPrep solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 72: Synthesis of 5'-[2'-*O*-methoxyethyl-(GCCTC]-d(AGT-CTG-CTT-C-)-[2'-O-methoxyethyl-(GCACC]-3' 20-mer phosphate diester using **4**: Synthesis of above sequence was performed on an Amersham Biosciences' Akta OligoPilot DNA/RNA Synthesizer on a 180 micromole scale using cyanoethyl phosphoramidites and the above prepared OligoPrep solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 73: Synthesis of 5'-[2'-*O*-methoxyethyl-(GCCTC]-d(AGT-CTG-CTT-C-)-[2'-O-methoxyethyl-(GCACC]-3' 20-mer phosphate diester using **4'**: Synthesis of above sequence was performed on an Amersham Biosciences' Akta OligoPilot DNA/RNA Synthesizer on a 169 micromole scale using cyanoethyl phosphoramidites and the above prepared OligoPrep solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 74: Synthesis of fully-modified 5'-[2'-*O*-methoxyethyl-(TGTG]-d(CTA-TTC-TGT-G-)-[2'-O-methoxyethyl-(AATT]-3' phosphorothioate 18-mer using **4**: Synthesis of above sequence was performed on an Amersham Biosciences' Akta OligoPilot DNA/RNA Synthesizer on a 172 micromole scale using cyanoethyl phosphoramidites and the above prepared Nittomar 250 solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 75: Synthesis of fully-modified 5'-[2'-*O*-methoxyethyl-(TGTG]-d(CTA-TTC-TGT-G-)-[2'-O-methoxyethyl-(AATT]-3' phosphorothioate 18-mer using **4'**: Synthesis of above sequence was performed on an Amersham Biosciences' Akta OligoPilot DNA/RNA Synthesizer on a 175 micromole scale using cyanoethyl phosphoramidites and the above prepared Nittomar 250 solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 76: Synthesis of fully-modified 5'-[2'-*O*-methoxyethyl-(GCCTC]-d(AGT-CTG-CTT-C-)-[2'-O-methoxyethyl-(GCACC]-3' phosphorothioate 20-mer using **4**: Synthesis of above sequence was performed on an Amersham Biosciences' Akta OligoPilot DNA/RNA Synthesizer on a 178 micromole scale using cyanoethyl phosphoramidites and the above prepared Nittomar 250 solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 77: Synthesis of fully-modified 5'-[2'-*O*-methoxyethyl-(GCCTC]-d(AGT-CTG-CTT-C-)-[2'-O-methoxyethyl-(GCACC]-3' phosphorothioate 20-mer using **4'**: Synthesis of above sequence was performed on an Amersham Biosciences' Akta OligoPilot DNA/RNA Synthesizer on a 181 micromole scale using cyanoethyl phosphoramidites and the above prepared Nittomar 250 solid support. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 78: Synthesis of 5'-[2'-*O*-methoxyethyl-(TGTG]-d(CTA-TTC-TGT-G-)-[2'-O-methoxyethyl-(AATT]-3' 18-mer phosphate diester using **4**: Synthesis of above sequence was performed on an Amersham Biosciences' Akta OligoPilot DNA/RNA Synthesizer on a 184 micromole scale using cyanoethyl phosphoramidites and the above prepared Nittomar 250 solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 79: Synthesis of 5-[2'-*O*-methoxyethyl-(TGTG]-d(CTA-TTC-TGT-G-)-[2'-O-methoxyethyl-(AATT]-3' 18-mer phosphate diester using **4'**: Synthesis of above sequence was performed on an Amersham Biosciences' Akta OligoPilot DNA/RNA Synthesizer on a 179 micromole scale using cyanoethyl phosphoramidites and the above prepared Nittomar 250 solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 80: Synthesis of 5'-[2'-*O*-methoxyethyl-(GCCTC]-d(AGT-CTG-CTT-C-)-[2'-O-methoxyethyl-(GCACC]-3' 20-mer phosphate diester using **4**: Synthesis of above sequence was performed on an Amersham Biosciences' Akta OligoPilot DNA/RNA Synthesizer on a 180 micromole scale using cyanoethyl phosphoramidites and the above prepared Nittomar 250 solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 81: Synthesis of 5'-[2'-*O*-methoxyethyl-(GCCTC]-d(AGT-CTG-CTT-C-)-[2'-O-methoxyethyl-(GCACC]-3' 20-mer phosphate diester using **4'**: Synthesis of above sequence was performed on an Amersham Biosciences' Akta OligoPilot DNA/RNA Synthesizer on a 169 micromole scale using cyanoethyl phosphoramidites and the above prepared Nittomar 250 solid support. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 82: Reaction of DMT protected compound **3** with sebacic anhydride: DMT protected hydroxy compound 3 (FW 578; 49.13 g; 85 mmole) was dissolved in a mixture ethyl acetate : methylene chloride (600 : 66 = 666 mL). Triethyl amine (FW 101.19; 51.61 g; 71 mL; 0.51 mole; 6 equivalent with respect to starting DMT compound) was added and stirred magnetically at room temperature. To this clear solution, sebacic anhydride (0.34 mole, 4 equivalents with respect to starting DMT compound) was added as solid all at once. Stirring was continued overnight. TLC indicated disappearance of starting material. If starting material is seen, more of succinic anhydride is added till completion of reaction. The reaction mixture was diluted with ethyl acetate (300 mL) and washed with water (2 x 200 mL), brine (120 mL) and dried with magnesium sulfate. If the product is colored, the material is passed through a short pad of silica gel eluting with methylene chloride and then 5% methanol:95% methylene chloride to afford the product as a colorless product. Yield of 4a: 60.5 g (91%).

Experiment 83: Loading of DMT protected sebaciate **4a** to controlled pore glass: Loading of the sebaciate molecule was performed similar to nucleoside succinate using HBTU as activator and Hunig's base in acetonitrile as solvent. The unreacted sites were capped with acetic anhydride in pyridine in presence of DMAP as catalyst. Loading was then checked using the standard UV method. Loading = 40 micromole/gram.

Experiment 84: Loading of DMT protected sebaciate **4a** to HL30 amino-derivatized primer support: Loading of the sebaciate molecule was performed similar to nucleoside succinate using HBTU as activator and Hunig's base in acetonitrile as solvent. The unreacted sites were capped with acetic anhydride in pyridine in presence of DMAP as catalyst. Loading was then checked using the standard UV method. Loading = 90 micromole/gram.

Experiment 85: Loading of DMT protected succinate **4a** to OligoPrep: Loading of the succinate molecule was performed similar to nucleoside succinate using HBTU as activator and Hunig's base in acetonitrile as solvent. The unreacted sites were capped with acetic anhydride in pyridine in presence of DMAP as catalyst. Loading was then checked using the standard UV method. Loading = 244 micromole/gram.

Experiment 86: Loading of DMT protected sebaciate **4a** to Nittomar 200 solid support: Loading of the sebaciate molecule was performed similar to nucleoside succinate using HBTU as activator and Hunig's base in acetonitrile as solvent. The unreacted sites were capped with acetic anhydride in pyridine in presence of DMAP as catalyst. Loading was then checked using the standard UV method. Loading = 200 micromole/gram.

Experiment 87: Synthesis of fully-modified 5'-d(TCC-CGC-CTG-TGA-CAT-GCA-TT)-3' phosphorothioate 20-mer: Synthesis of above sequence was performed on an ABI 390Z DNA/RNA Synthesizer on a 15 micromole scale using cyanoethyl phosphoramidites and the above prepared CPG solid support derivatized with **4a**. Detritylation was performed using 3% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 88: Synthesis of fully-modified 5'-d(GCC-CAA-GCT-GGC-ATC-CGT-CA)-3' phosphorothioate 20-mer: Synthesis of above sequence was performed on an ABI 390Z DNA/RNA Synthesizer on a 15 micromole scale using cyanoethyl phosphoramidites and the above prepared CPG solid support derivatized with **4a**. Detritylation was performed using 3% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 89: Synthesis of 5'-d(TCC-CGC-CTG-TGA-CAT-GCA-TT)-3' DNA 20-mer: Synthesis of above sequence was performed on an ABI 390Z DNA/RNA Synthesizer on a 15 micromole scale using cyanoethyl phosphoramidites and the above prepared CPG solid support derivatized with **4a**. Detritylation was performed using 3% dichloroacetic acid in toluene (volume/volume). Oxidation was performed using a solution of iodine in THF/water/pyridine as recommended by ABI manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 90: Synthesis of 5'-d(GCC-CAA-GCT-GGC-ATC-CGT-CA)-3' DNA 20-mer: Synthesis of above sequence was performed on an ABI 390Z DNA/RNA Synthesizer on a 15 micromole scale using cyanoethyl phosphoramidites and the above prepared CPG solid support derivatized with 4a. Detritylation was performed using 3% dichloroacetic acid in toluene (volume/volume). Oxidation was performed using a solution of iodine in THF/water/pyridine as recommended by ABI manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 91: Synthesis of fully-modified 5'-d(TCC-CGC-CTG-TGA-CAT-GCA-TT)-3' phosphorothioate 20-mer: Synthesis of above sequence was performed on an Amersham Biosciences' Akta OligoPilot DNA/RNA Synthesizer on a 172 micromole scale using cyanoethyl phosphoramidites and the above prepared HL30 Primer solid support derivatized with **4a**. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 92: Synthesis of fully-modified 5'-d(GCC-CAA-GCT-GGC-ATC-CGT-CA)-3' phosphorothioate 20-mer: Synthesis of above sequence was performed on an Amersham Biosciences' Akta OligoPilot DNA/RNA Synthesizer on a 178 micromole scale using cyanoethyl phosphoramidites and the above prepared HL30 Primer solid support derivatized with **4a**. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 93: Synthesis of 5'-d(TCC-CGC-CTG-TGA-CAT-GCA-TT)-3' DNA 20-mer: Synthesis of above sequence was performed on an Amersham Biosciences' Akta OligoPilot DNA/RNA Synthesizer on a 184 micromole scale using cyanoethyl phosphoramidites and the above prepared HL30 Primer solid support derivatized with **4a**. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 94: Synthesis of 5'-d(GCC-CAA-GCT-GGC-ATC-CGT-CA)-3' DNA 20-mer: Synthesis of above sequence was performed on an Amersham Biosciences' Akta OligoPilot DNA/RNA Synthesizer on a 180 micromole scale using cyanoethyl phosphoramidites and the above prepared HL30 Primer solid support derivatized with **4a**. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 95: Synthesis of fully-modified 5'-d(TCC-CGC-CTG-TGA-CAT-GCA-TT)-3' phosphorothioate 20-mer: Synthesis of above sequence was performed on an Amersham Biosciences' Akta OligoPilot DNA/RNA Synthesizer on a 172 micromole scale using cyanoethyl phosphoramidites and the above prepared OligoPrep solid support derivatized with **4a**. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 96: Synthesis of fully-modified 5'-d(GCC-CAA-GCT-GGC-ATC-CGT-CA)-3' phosphorothioate 20-mer: Synthesis of above sequence was performed on an Amersham Biosciences' Akta OligoPilot DNA/RNA Synthesizer on a 178 micromole scale using cyanoethyl phosphoramidites and the above prepared OligoPrep solid support derivatized with **4a**. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 97: Synthesis of fully-modified 5'-d(GCC-CAA-GCT-GGC-ATC-CGT-CA)-3' phosphorothioate 20-mer: Synthesis of above sequence was performed on an Amersham Biosciences' Akta OligoPilot DNA/RNA Synthesizer on a 178 micromole scale using cyanoethyl phosphoramidites and the above prepared OligoPrep solid support derivatized with **4a**. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 98: Synthesis of 5'-d(GCC-CAA-GCT-GGC-ATC-CGT-CA)-3' DNA 20-mer: Synthesis of above sequence was performed on an Amersham Biosciences' Akta OligoPilot DNA/RNA Synthesizer on a 180 micromole scale using cyanoethyl phosphoramidites and the above prepared OligoPrep solid support derivatized with **4a**. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 99: Synthesis of fully-modified 5'-d(TCC-CGC-CTG-TGA-CAT-GCA-TT)-3' phosphorothioate 20-mer: Synthesis of above sequence was performed on an Amersham Biosciences' Akta OligoPilot DNA/RNA Synthesizer on a 172 micromole scale using cyanoethyl phosphoramidites and the above prepared Nittomar 200 solid support derivatized with 4a. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 100: Synthesis of fully-modified 5'-d(GCC-CAA-GCT-GGC-ATC-CGT-CA)-3' phosphorothioate 20-mer: Synthesis of above sequence was performed on an Amersham Biosciences' Akta OligoPilot DNA/RNA Synthesizer on a 178 micromole scale using cyanoethyl phosphoramidites and the above prepared Nittomar 2-0 solid support derivatized with **4a**. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 101: Synthesis of 5'-d(TCC-CGC-CTG-TGA-CAT-GCA-TT)-3' DNA 20-mer: Synthesis of above sequence was performed on an Amersham Biosciences' Akta OligoPilot DNA/RNA Synthesizer on a 184 micromole scale using cyanoethyl phosphoramidites and the above prepared Nittomar 200 solid support derivatized with **4a**. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 102: Synthesis of 5'-d(GCC-CAA-GCT-GGC-ATC-CGT-CA)-3' DNA 20-mer: Synthesis of above sequence was performed on an Amersham Biosciences' Akta OligoPilot DNA/RNA Synthesizer on a 180 micromole scale using cyanoethyl phosphoramidites and the above prepared Nittomar 200 solid support derivatized with **4a**. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 103: Synthesis of fully-modified 5'-[2'-*O*-methoxyethyl-(TGTG]-d(CTA-TTC-TGT-G-)-[2'-O-methoxyethyl-(AATT]-3' phosphorothioate 18-mer: Synthesis of above sequence was performed on an ABI 390Z DNA/RNA Synthesizer on a 15 micromole scale using cyanoethyl phosphoramidites and the above prepared CPG solid support derivatized with **4a**. Detritylation was performed using 3% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 104: Synthesis of fully-modified 5'-[2'-*O*-methoxyethyl-(GCCTC]-d(AGT-CTG-CTT-C-)-[2'-O-methoxyethyl-(GCACC]-3' phosphorothioate 20-mer: Synthesis of above sequence was performed on an ABI 390Z DNA/RNA Synthesizer on a 15 micromole scale using cyanoethyl phosphoramidites and the above prepared CPG solid support derivatized with **4a**. Detritylation was performed using 3% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 105: Synthesis of 5'-[2'-*O*-methoxyethyl-(TGTG]-d(CTA-TTC-TGT-G-)-[2'-O-methoxyethyl-(AATT]-3' 18-mer phosphate diester: Synthesis of above sequence was performed on an ABI 390Z DNA/RNA Synthesizer on a 15 micromole scale using cyanoethyl phosphoramidites and the above prepared CPG solid support derivatized with **4a**. Detritylation was performed using 3% dichloroacetic acid in toluene (volume/volume). Oxidation was performed using a solution of iodine in THF/water/pyridine as recommended by ABI manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 106: Synthesis of 5'-[2'-*O*-methoxyethyl-(GCCTC]-d(AGT-CTG-CTT-C-)-[2'-O-methoxyethyl-(GCACC]-3' 20-mer phosphate diester: Synthesis of above sequence was performed on an ABI 390Z DNA/RNA Synthesizer on a 15 micromole scale using cyanoethyl phosphoramidites and the above prepared CPG solid support derivatized with **4a**. Detritylation was performed using 3% dichloroacetic acid in toluene (volume/volume). Oxidation was performed using a solution of iodine in THF/water/pyridine as recommended by ABI manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 107: Synthesis of fully-modified 5'-[2'-*O*-methoxyethyl-(TGTG]-d(CTA-TTC-TGT-G-)-[2'-O-methoxyethyl-(AATT]-3' phosphorothioate 18-mer: Synthesis of above sequence was performed on an Amersham Biosciences' Akta OligoPilot DNA/RNA Synthesizer on a 172 micromole scale using cyanoethyl phosphoramidites and the above prepared HL30 Primer solid support derivatized with 4a. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 108: Synthesis of fully modified 5'-[2'-*O*-methoxyethyl-(GCCTC]-d(AGT-CTG-CTT-C-)-[2'-O-methoxyethyl-(GCACC]-3' phosphorothioate 20-mer: Synthesis of above sequence was performed on an Amersham Biosciences' Akta OligoPilot DNA/RNA Synthesizer on a 178 micromole scale using cyanoethyl phosphoramidites and the above prepared HL30 Primer solid support derivatized with 4a. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 109: Synthesis of 5'-[2'-*O*-methoxyethyl-(TGTG]-d(CTA-TTC-TGT-G-)-[2'-O-methoxyethyl-(AATT]-3' 18-mer phosphate diester: Synthesis of above sequence was performed on an Amersham Biosciences' Akta OligoPilot DNA/RNA Synthesizer on a 184 micromole scale using cyanoethyl phosphoramidites and the above prepared HL30 Primer solid support derivatized with 4a. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 110: Synthesis of 5'-[2'-*O*-methoxyethyl-(GCCTC]-d(AGT-CTG-CTT-C-)-[2'-O-methoxyethyl-(GCACC]-3' 20-mer phosphate diester: Synthesis of above sequence was performed on an Amersham Biosciences' Akta OligoPilot DNA/RNA Synthesizer on a 180 micromole scale using cyanoethyl phosphoramidites and the above prepared HL30 Primer solid support derivatized with **4a**. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 111: Synthesis of fully-modified 5'-[2'-*O*-methoxyethyl-(TGTG]-d(CTA-TTC-TGT-G-)-[2'-O-methoxyethyl-(AATT]-3' phosphorothioate 18-mer: Synthesis of above sequence was performed on an Amersham Biosciences' Akta OligoPilot DNA/RNA Synthesizer on a 172 micromole scale using cyanoethyl phosphoramidites and the above prepared OligoPrep solid support derivatized with **4a**. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 112: Synthesis of fully modified 5'-[2'-*O*-methoxyethyl-(GCCTC]-d(AGT-CTG-CTT-C-)-[2'-O-methoxyethyl-(GCACC]-3' phosphorothioate 20-mer: Synthesis of above sequence was performed on an Amersham Biosciences' Akta OligoPilot DNA/RNA Synthesizer on a 178 micromole scale using cyanoethyl phosphoramidites and the above prepared OligoPrep solid support derivatized with **4a**. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 113: Synthesis of 5'-[2'-*O*-methoxyethyl-(TGTG]-d(CTA-TTC-TGT-G-)-[2'-O-methoxyethyl-(AATT]-3' 18-mer phosphate diester: Synthesis of above sequence was performed on an Amersham Biosciences' Akta OligoPilot DNA/RNA Synthesizer on a 184 micromole scale using cyanoethyl phosphoramidites and the above prepared OligoPrep solid support derivatized with **4a**. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 114: Synthesis of 5'-[2'-*O*-methoxyethyl-(GCCTC]-d(AGT-CTG-CTT-C-)-[2'-O-methoxyethyl-(GCACC]-3' 20-mer phosphate diester: Synthesis of above sequence was performed on an Amersham Biosciences' Akta OligoPilot DNA/RNA Synthesizer on a 180 micromole scale using cyanoethyl phosphoramidites and the above prepared OligoPrep solid support derivatized with **4a**. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 115: Synthesis of fully-modified 5'-[2'-*O*-methoxyethyl-(TGTG]-d(CTA-TTC-TGT-G-)-[2'-O-methoxyethyl-(AATT]-3' phosphorothioate 18-mer: Synthesis of above sequence was performed on an Amersham Biosciences' Akta OligoPilot DNA/RNA Synthesizer on a 172 micromole scale using cyanoethyl phosphoramidites and the above prepared Nittomar 200 solid support derivatized with **4a**. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 116: Synthesis of fully-modified 5'-[2'-*O*-methoxyethyl-(GCCTC]-d(AGT-CTG-CTT-C-)-[2'-O-methoxyethyl-(GCACC]-3' phosphorothioate 20-mer: Synthesis of above sequence was performed on an Amersham Biosciences' Akta OligoPilot DNA/RNA Synthesizer on a 178 micromole scale using cyanoethyl phosphoramidites and the above prepared Nittomar 200 solid support derivatized with **4a**. Detritylation was performed using 10% dichloroacetic acid in toluene (volume/volume). Sulfurization was performed using a 0.2 M solution of phenylacetyl disulfide in acetonitrile:3-picoline (1:1 v/v) for 2 minutes. At the end of synthesis, the support was washed with acetonitrile, cleaved, deprotected using ammonium hydroxide at 55 deg C for 12 hours. The crude oligo was purified in the usual manner to afford the desired phosphorothioate oligonucleotide.

Experiment 117: Synthesis of 5'-[2'-*O*-methoxyethyl-(TGTG]-d(CTA-TTC-TGT-G-)-[2'-O-methoxyethyl-(AATT]-3' 18-mer phosphate diester: Synthesis of above sequence was performed on an Amersham Biosciences' Akta OligoPilot DNA/RNA Synthesizer on a 184 micromole scale using cyanoethyl phosphoramidites and the above prepared Nittomar 200 solid support derivatized with **4a**. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

Experiment 118: Synthesis of 5'-(2'-*O*-methoxyethyl-(GCCTC]-d(AGT-CTG-CTT-C-)-[2'-O-methoxyethyl-(GCACC]-3' 20-mer phosphate diester: Synthesis of above sequence was performed on an Amersham Biosciences' Akta OligoPilot DNA/RNA Synthesizer on a 180 micromole scale using cyanoethyl phosphoramidites and the above prepared Nittomar 200 solid support derivatized with **4a**. Detritylation was performed using 10% dichloroacetic acid in toluene (volume/volume). Oxidation was performed using a solution of iodine in THF/water/pyridine as recommended by instrument manual protocol. At the end of synthesis, the support was washed with acetonitrile, cleaved, deprotected using ammonium hydroxide at 55 deg C for 12 hours. The crude oligo was purified in the usual manner to afford the desired oligonucleotide.

## Claims

1. A compound of Formula I: 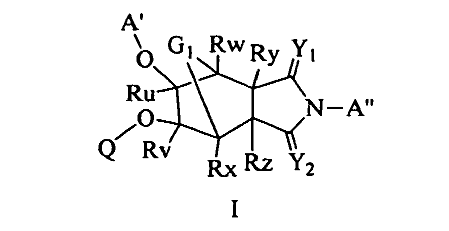 wherein:
one of A' and A" is SM or L-SM,
SM is a support medium;
L is a linking moiety,
the other of A' and A" is H or a substituent;
each of Ru, Rv, Rw, Rx, Ry and Rz is H, alkyl, substituted alkyl, alkenyl, substituted alkenyl, alkynyl or substituted alkynyl;
Y₁ and Y₂ are each independently of one another O, S, NR₁ CH₂ or CR₁R₁: R₁ is H, alkyl, substituted alkyl, alkenyl, substituted alkenyl, alkynyl or substituted alkynyl;
G₁ is O, S, or NR';
wherein R' is H or a substituent;
Q is H, a nucleosidyl moiety, a protected nucleosidyl moiety, a nucleosidyl moiety linked through a linker, a protected oligonucleotidyl or an oligonucleotidyl moiety, or a protecting group; and
said linker is a phosphitidyl triester, phosphodiester, phosphorothioate diester, or phosphotriester moiety.

2. The compound of claim 1, wherein each of Y₁ and Y₂ is O.

3. The compound of claim 1, wherein at least one of Y₁ and Y₂ is O.

4. The compound of claim 1, wherein A" is phenyl.

5. The compound of claim 1, wherein A" is L-SM.

6. The compound of claim 1, wherein A' is L-SM.

7. The compound of claim 1, having the formula: 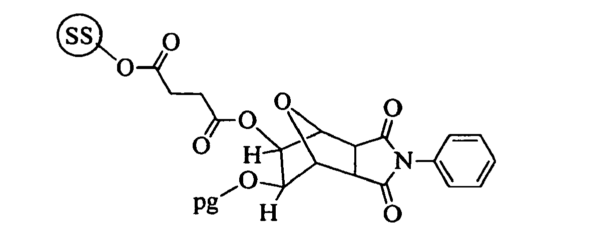 wherein SS is a solid support medium and pg is a 4,4'-dimethoxytriphenylmethyl group or an optionally further substituted pixyl group.

8. The compound of claim 1, having the formula: 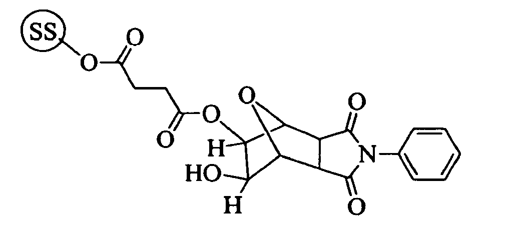 wherein SS is a solid support medium.

9. The compound of claim 1, having the formula: 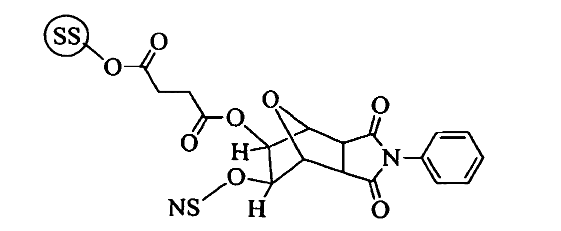 wherein NS is a residue of an optionally protected nucleoside and SS is a solid support medium.

10. The compound of claim 1 having the formula: 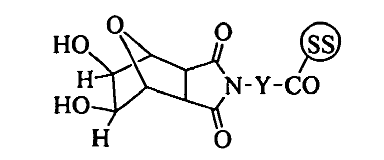 wherein Y is alkylene, cycloalkylene, arylene, heteroarylene, arylalkylene, alkarylene, or alkylenearylalkylene and SS is a solid support medium.

11. A compound of claim 1 of formula: 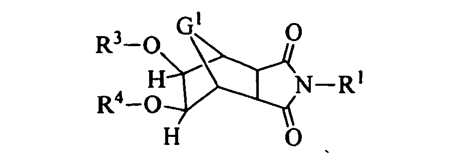 wherein G¹ is O,
R¹ is phenyl;
R³ is a linking moiety connected to a solid support medium; and
R⁴ is a member of the group consisting of H, a protecting group, and a nucleoside linked through a phosphodiester, phosphorothioate or phosphoramidate bond, which nucleoside is optionally protected at the 5'-position or linked to one or more further nucleosides through an internucleoside linker.

12. A compound of claim 1 of formula: 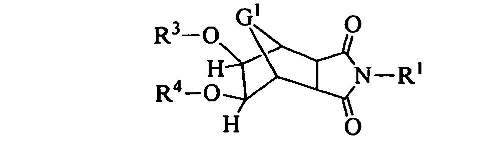 wherein G¹ is O, S or NR²,
R² is a substituent; and
R¹ is aryl, cycloalkyl, unsaturated cycloalkyl, alkyl, unsaturated alkyl, heterocyclyl, unsaturated heterocyclyl, heteroaryl or acyl, wherein R¹ is optionally substituted with one or more substituents;
R³ is a linking moiety connected to a solid support medium; and
R⁴ is a member of the group consisting of H, a protecting group, and a nucleoside linked through a phosphodiester, phosphorothioate or phosphoramidate linker, which nucleoside is optionally protected at the 5'-position or linked to one or more further nucleosides through an internucleoside linker.

13. The compound of claim 11, wherein R⁴ is H.

14. The compound of claim 11, wherein R⁴ is a nucleoside linked through a phosphodiester, phosphorothioate or phosphoramidate linker, wherein said nucleoside is optionally protected with a protecting group.

15. The compound of claim 11, wherein R⁴ is a nucleoside linked through a phosphodiester, phosphorothioate or phosphoramidate linker, wherein said nucleoside has a free 5'-OH group.

16. The compound of claim 11, wherein R⁴ is a 5'-protected nucleoside linked through a phosphodiester, phosphorothioate or phosphoramidate linker.

17. The compound of claim 11, wherein R⁴ is a nucleoside linked through a phosphodiester, phosphorothioate or phosphoramidate linker, wherein said nucleoside is connected to one or more further nucleosides through a suitable internucleoside linker.

18. A compound of claim 1 having the formula: 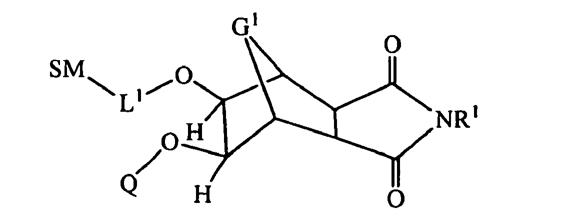 wherein:
SM is a support medium;
L¹ is a linking moiety;
G¹ is O, S, NR²;
R² is other than H;
Q is H or pg;
pg is a protecting group; and
R¹ is a blocking group.

19. The compound of claim 18 wherein SM is a solid support material.

20. The compound of any one of claims 18 or 19 wherein L¹ is C(=O)-(CH₂)₂-C(=O).

21. The compound of any one of claims 18 to 20 wherein G¹ is O.

22. The compound of any one of claims 18 to 21 wherein Q is H.

23. The compound of any one of claims 18 to 21 wherein Q is a protecting group.

24. The compound of any one of claims 18 to 23 wherein G¹ is O, and R¹ is an optionally further substituted alkyl, aryl, acyl, aralkyl, arylacyl, cycloalkyl, heteroaryl or heteroarylacyl group.

25. The compound of any one of claims 18 to 24 wherein R¹ is aryl wherein said aryl group is optionally substituted with one or more substituents selected from F, Cl, Br, I, O-C₁-C₁₂ alkyl O-C₁-C₁₂ acyl, NO₂, heteroaryl, alkyl and acyl.

26. The compound of claim 25 wherein R¹ is phenyl or substituted phenyl.

27. The compound of any one of claims 1 to 24 wherein R¹ is an alkyl group selected from methyl, ethyl, n-propyl, isopropyl, n-butyl, s-butyl, t-butyl, i-butyl, n-pentanyl, isooctanyl and dodecanyl wherein said alkyl group is optionally substituted with one or more substituents selected from F, Cl, Br, I, O-C₁-C₁₂ alkyl, O-C₁-C₁₂ acyl, NO₂, aryl and heteroaryl.

28. The compound of any one of claims 1 to 7 wherein R¹ is an acyl group selected from formyl, acetyl, propanoyl, butanoyl and octanoyl wherein said acyl group is optionally substituted with one or more substituents selected from F, Cl, Br, I, O-C₁-C₁₂ alkyl, NO₂, aryl or heteroaryl.

29. The compound of claim 1 having the formula:

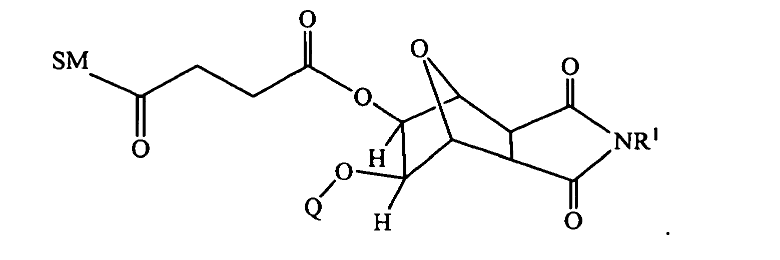

30. A process of making the compound of claim 1 having Formula I comprising reacting a compound of Formula II with a compound of Formula III: 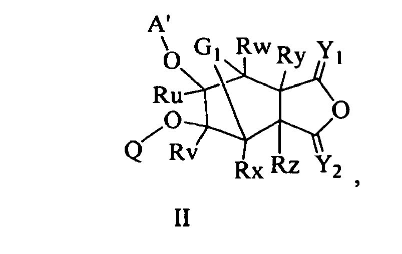 NH₂A" III.

31. The process of claim 30, further comprising synthesising a compound of Formula II by Diels-Alder reaction from dienophile IV and diene V: 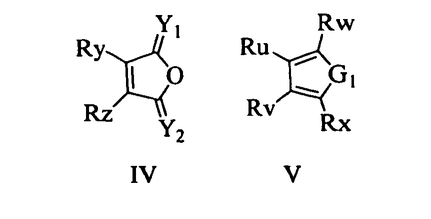 to form intermediate A: 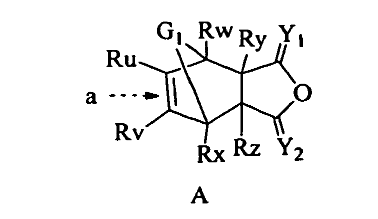 adding hydroxyl groups across the double bond to form intermediate B: 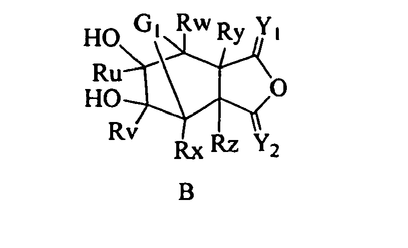 and derivatising intermediate B to form a compound of Formula II.

32. A process of making the compound of claim 1 having Formula I comprising
reacting dienophile IVa with diene V to form intermediate AA: 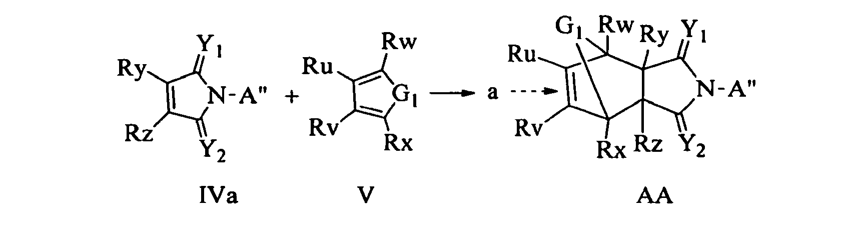 adding hydroxyl groups across double bond a to form intermediate BB: 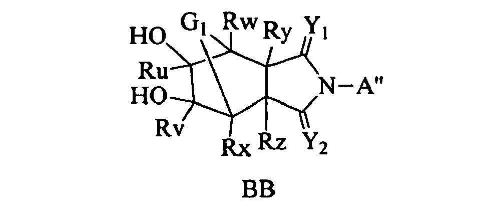 and derivatising intermediate BB to form a compound of Formula I.

## Patentansprüche

1. Verbindung der Formel I: 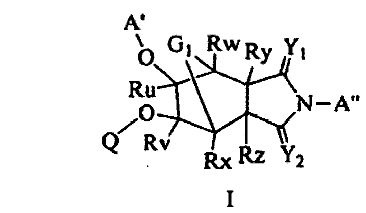 wobei:
einer der Reste A' und A" für SM oder L-SM steht,
SM ein Trägermedium bedeutet;
L für eine Verknüpfungsgruppierung steht,
der jeweils andere Rest A' bzw. A" für H oder einen Substituenten steht;
Ru, Rv, Rw, Rx, Ry bzw. Rz jeweils für H, Alkyl, substituiertes Alkyl, Alkenyl, substituiertes Alkenyl, Alkinyl oder substituiertes Alkinyl steht;
Y₁ und Y₂ jeweils unabhängig voneinander für 0, S, NR₁CH₂ oder CR₁R₁ stehen:
R₁ für H, Alkyl, substituiertes Alkyl, Alkenyl, substituiertes Alkenyl, Alkinyl oder substituiertes Alkinyl steht;
G₁ für 0, S oder NR' steht;
wobei R' für H oder einen Substituenten steht;
Q für H, eine Nukleosidylgruppierung, eine geschützte Nukleosidylgruppierung, eine über einen Linker verknüpfte Nukleosidylgruppierung,
eine geschützte Oligonukleotidyl- oder eine Oligonukleotidylgruppierung oder eine Schutzgruppe steht; und
es sich bei dem Linker um eine Phosphitidyltriester-, Phosphodiester-, Phosphorthioatdiester- oder Phosphotriestergruppierung handelt.

2. Verbindung nach Anspruch 1, wobei Y₁ bzw. Y₂ jeweils für O steht.

3. Verbindung nach Anspruch 1, wobei wenigstens einer der Reste Y₁ und Y₂ für 0 steht.

4. Verbindung nach Anspruch 1, wobei A" für Phenyl steht.

5. Verbindung nach Anspruch 1, wobei A" für L-SM steht.

6. Verbindung nach Anspruch 1, wobei A' für L-SM steht.

7. Verbindung nach Anspruch 1, mit der Formel: 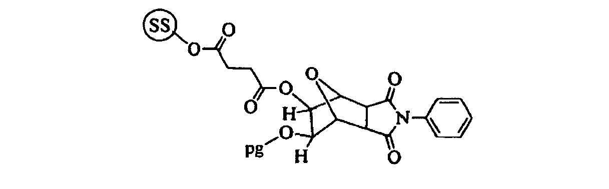 wobei SS ein festes Trägermedium bedeutet und pg für eine 4,4'-Dimethoxytriphenylmethylgruppe oder eine gegebenenfalls weiter substituierte Pixylgruppe steht.

8. Verbindung nach Anspruch 1, mit der Formel: 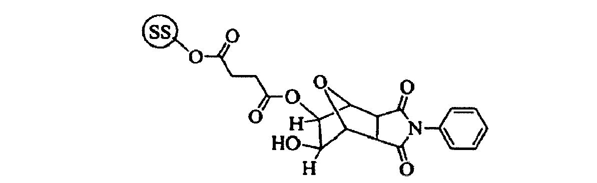 wobei SS ein festes Trägermedium bedeutet.

9. Verbindung nach Anspruch 1, mit der Formel: 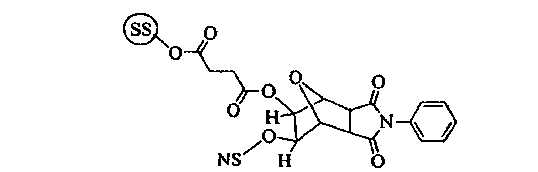 wobei NS für einen Rest eines gegebenenfalls geschützten Nukleosids steht und SS ein festes Trägermedium bedeutet.

10. Verbindung nach Anspruch 1 mit der Formel: 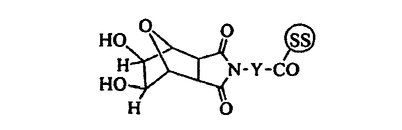 wobei Y für Alkylen, Cycloalkylen, Arylen, Heteroarylen, Arylalkylen, Alkarylen oder Alkylenarylalkylen steht und SS ein festes Trägermedium bedeutet.

11. Verbindung nach Anspruch 1 der Formel: 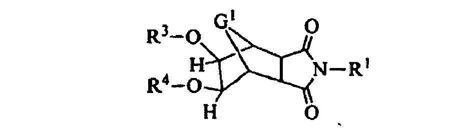 wobei G¹ für 0 steht,
R¹ für Phenyl steht;
R³ für eine mit einem festen Trägermedium verbundene Verknüpfungsgruppierung steht; und
R⁴ für ein Mitglied der aus H, einer Schutzgruppe und einem über eine Phosphodiester-, Phosphorthioat- oder Phosphoramidatbindung verknüpften Nukleosid bestehenden Gruppe steht, wobei das Nukleosid gegebenenfalls in 5'-Stellung geschützt oder über einen Internukleosid-Linker mit einem oder mehreren weiteren Nukleosiden verknüpft ist.

12. Verbindung nach Anspruch 1 der Formel: 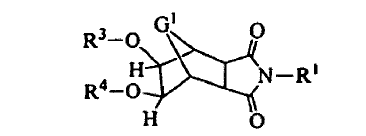 wobei G¹ für 0, S oder NR² steht,
R² für einen Substituenten steht; und
R¹ für Aryl, Cycloalkyl, ungesättigtes Cycloalkyl, Alkyl, ungesättigtes Alkyl, ungesättigtes Heterocyclyl, Heterocyclyl, Heteroaryl oder Acyl steht, wobei R¹ gegebenenfalls mit einem oder mehreren Substituenten substituiert ist;
R³ für eine mit einem festen Trägermedium verbundene Verknüpfungsgruppierung steht; und
R⁴ für ein Mitglied der aus H, einer Schutzgruppe und einem über einen Phosphodiester-, Phosphorthioat- oder Phosphoramidat-Linker verknüpften Nukleosid bestehenden Gruppe steht, wobei das Nukleosid gegebenenfalls in 5'-Stellung geschützt oder über einen Internukleosid-Linker mit einem oder mehreren weiteren Nukleosiden verknüpft ist.

13. Verbindung nach Anspruch 11, wobei R⁴ für H steht.

14. Verbindung nach Anspruch 11, wobei R⁴ für ein über einen Phosphodiester-, Phosphorthioat- oder Phosphoramidat-Linker verknüpftes Nukleosid steht, wobei das Nukleosid gegebenenfalls mit einer Schutzgruppe geschützt ist.

15. Verbindung nach Anspruch 11, wobei R⁴ für ein über einen Phosphodiester-, Phosphorthioat- oder Phosphoramidat-Linker verknüpftes Nukleosid steht, wobei das Nukleosid eine freie 5'-OH-Gruppe aufweist.

16. Verbindung nach Anspruch 11, wobei R⁴ für ein über einen Phosphodiester-, Phosphorthioat- oder Phosphoramidat-Linker verknüpftes 5'-geschütztes Nukleosid steht.

17. Verbindung nach Anspruch 11, wobei R⁴ für ein über einen Phosphodiester-, Phosphorthioat- oder Phosphoramidat-Linker verknüpftes Nukleosid steht, wobei das Nukleosid über einen geeigneten Internukleosid-Linker mit einem oder mehreren weiteren Nukleosiden verbunden ist.

18. Verbindung nach Anspruch 1 mit der Formel: 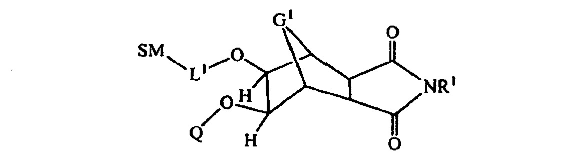 wobei:
SM ein festes Trägermedium bedeutet;
L¹ für eine Verknüpfungsgruppierung steht;
G¹ für 0, S, NR² steht;
R² für einen anderen Rest als H steht;
Q für H oder pg steht;
pg für eine Schutzgruppe steht; und
R¹ für eine Blockierungsgruppe steht.

19. Verbindung nach Anspruch 18, wobei SM ein festes Trägermaterial bedeutet.

20. Verbindung nach einem der Ansprüche 18 oder 19, wobei L¹ für C(=O)-(CH₂)₂-C(=O) steht.

21. Verbindung nach einem der Ansprüche 18 bis 20, wobei G¹ für 0 steht.

22. Verbindung nach einem der Ansprüche 18 bis 21, wobei Q für H steht.

23. Verbindung nach einem der Ansprüche 18 bis 21, wobei Q für eine Schutzgruppe steht.

24. Verbindung nach einem der Ansprüche 18 bis 23, wobei G¹ für 0 und R¹ für eine gegebenenfalls weiter substituierte Alkyl-, Aryl-, Acyl-, Aralkyl-, Arylacyl-, Cycloalkyl-, Heteroalkyl - oder Heteroarylacylgruppe steht.

25. Verbindung nach einem der Ansprüche 18 bis 24, wobei R¹ für Aryl steht, wobei die Arylgruppe gegebenenfalls mit einem oder mehreren aus F, Cl, Br, I, O-C₁-C₁₂Alkyl, O-C₁-C₁₂Acyl, NO₂, Heteroaryl, Alkyl und Acyl ausgewählten Substituenten substituiert ist.

26. Verbindung nach Anspruch 25, wobei R¹ für Phenyl oder substituiertes Phenyl steht.

27. Verbindung nach einem der Ansprüche 1 bis 24, wobei R¹ für eine aus Methyl, Ethyl, n-Propyl, Isopropyl, n-Butyl, s-Butyl, t-Butyl, i-Butyl, n-Pentanyl, Isooctanyl und Dodecanyl ausgewählte Alkylgruppe steht, wobei die Alkylgruppe gegebenenfalls mit einem oder mehreren aus F, Cl, Br, I, O-C₁-C₁₂Alkyl, O-C₁-C₁₂Acyl, NO₂, Aryl und Heteroaryl ausgewählten Substituenten substituiert ist.

28. Verbindung nach einem der Ansprüche 1 bis 7, wobei R¹ für eine aus Formyl, Acetyl, Propanoyl, Butanoyl und Octanoyl ausgewählte Acylgruppe steht, wobei die Acylgruppe gegebenenfalls mit einem oder mehreren aus F, Cl, Br, I, O-C₁-C₁₂Alkyl, NO₂, Aryl oder Heteroaryl ausgewählten Substituenten substituiert ist.

29. Verbindung nach Anspruch 1 mit der Formel :

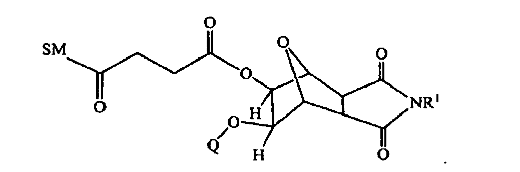

30. Verfahren zur Herstellung der Verbindung nach Anspruch 1 mit Formel I, bei dem man eine Verbindung der Formel II mit einer Verbindung der Formel III umsetzt: 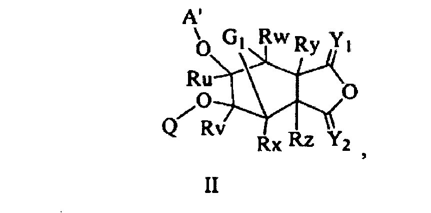
NH₂A" III.

31. Verfahren nach Anspruch 30, bei dem man ferner eine Verbindung der Formel II in einer Diels-Alder-Reaktion aus Dienophil IV und Dien V: 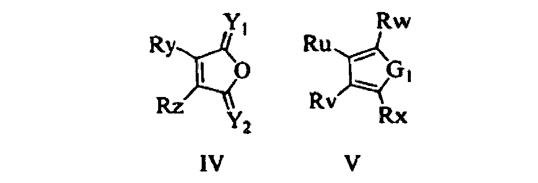 unter Bildung der Zwischenstufe A: 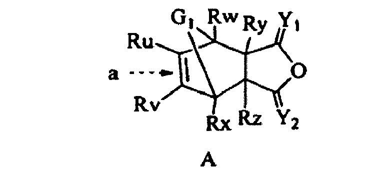 synthetisiert, wobei man Hydroxylgruppen an die Doppelbindung unter Bildung der Zwischenstufe B: 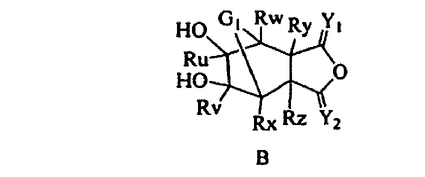 addiert und die Zwischenstufe B unter Bildung einer Verbindung der Formel II derivatisiert.

32. Verfahren zur Herstellung der Verbindung nach Anspruch 1 mit Formel I, bei dem man das Dienophil IVa mit dem Dien V unter Bildung der Zwischenstufe AA umsetzt: 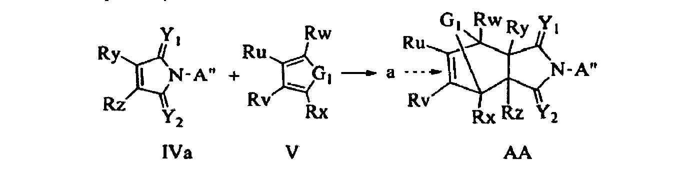 Hydroxylgruppen an die Doppelbindung a unter Bildung der Zwischenstufe BB: 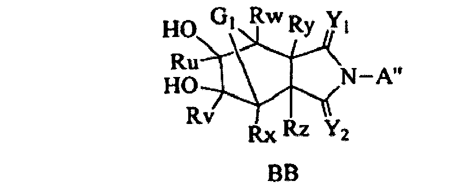 addiert und die Zwischenstufe BB unter Bildung einer Verbindung der Formel I derivatisiert.

## Revendications

1. Composé de formule I : 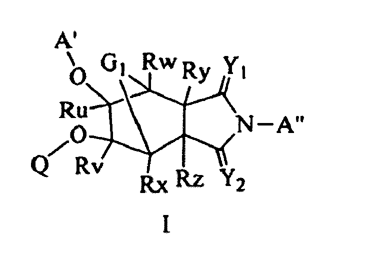 dans laquelle :
l'un de A' et A" est SM ou L-SM,
SM est un milieu de support ;
L est un fragment de liaison,
l'autre de A' et A" est H ou un substituant ;
chacun de Ru, Rv, Rw, Rx, Ry et Rz est H, alkyle,
alkyle substitué, alcényle, alcényle substitué,
alcynyle ou alcynyle substitué ;
Y₁ et Y₂ sont chacun indépendamment l'un de l'autre 0, S, NR₁CH₂ ou CR₁R₁ :
R₁ est H, alkyle, alkyle substitué, alcényle, alcényle substitué, alcynyle ou alcynyle substitué ;
G₁ est O, S, ou NR' ;
R' étant H ou un substituant ;
Q est H, un fragment nucléosidyle, un fragment nucléosidyle protégé, un fragment nucléosidyle lié par l'intermédiaire d'un lieur, un oligonucléotidyle protégé ou un fragment oligonucléotidyle, ou un groupe protecteur ; et
ledit lieur est un triester de phosphitidyle, un phosphodiester, un diester de phosphorothioate, ou un fragment phosphotriester.

2. Composé de la revendication 1, dans lequel chacun de Y₁ et Y₂ est O.

3. Composé de la revendication 1, dans lequel au moins l'un de Y₁ et Y₂ est O.

4. Composé de la revendication 1, dans lequel A" est phényle.

5. Composé de la revendication 1, dans lequel A" est L-SM.

6. Composé de la revendication 1, dans lequel A' est L-SM.

7. Composé de la revendication 1, ayant la formule 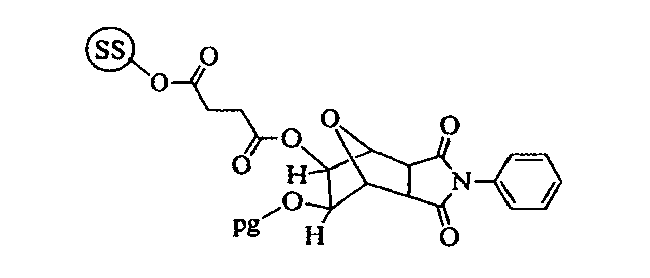 dans laquelle SS est un milieu de support solide et pg est un groupe 4,4'-diméthoxytriphénylméthyle ou un groupe pixyle facultativement substitué plus avant.

8. Composé de la revendication 1, ayant la formule : 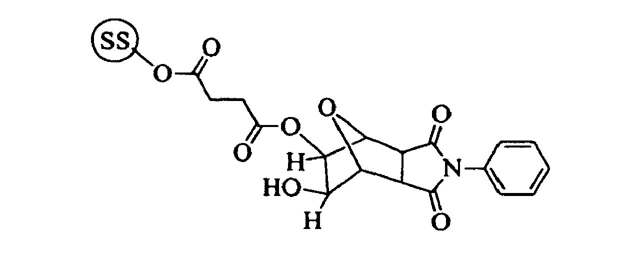 dans laquelle SS est un milieu de support solide.

9. Composé de la revendication 1, ayant la formule : 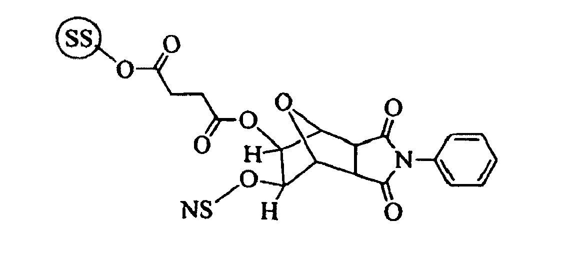 dans laquelle NS est un résidu d'un nucléoside facultativement protégé et SS est un milieu de support solide.

10. Composé de la revendication 1 ayant la formule : 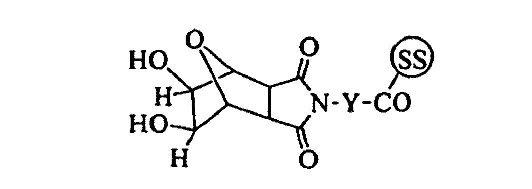 dans laquelle Y est alkylène, cycloalkylène, arylène, hétéroarylène, arylalkylène, alkarylène, ou alkylènearylalkylène et SS est un milieu de support solide.

11. Composé de la revendication 1 de formule : 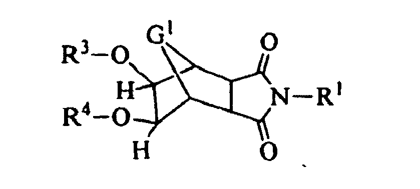 dans laquelle G¹ est O,
R¹ est phényle ;
R³ est un fragment de liaison relié à un milieu de support solide ; et
R⁴ est un membre du groupe constitué de H, un groupe protecteur, et un nucléoside lié par l'intermédiaire d'une liaison phosphodiester, phosphorothioate ou phosphoramidate, ledit nucléoside étant facultativement protégé à la position 5' ou lié à un ou plusieurs autres nucléosides par l'intermédiaire d'un lieur internucléoside.

12. Composé de la revendication 1 de formule : 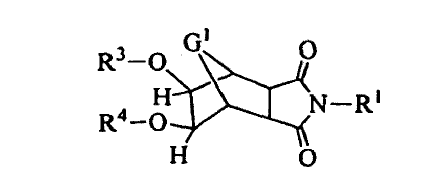 dans laquelle G¹ est 0, S ou NR²,
R² est un substituant ; et
R¹ est aryle, cycloalkyle, cycloalkyle insaturé, alkyle, alkyle insaturé, hétérocyclyle, hétérocyclyle insaturé, hétéroaryle ou acyle, R¹ étant facultativement substitué par un ou plusieurs substituants ;
R³ est un fragment de liaison relié à un milieu de support solide ; et
R⁴ est un membre du groupe constitué de H, un groupe protecteur, et un nucléoside lié par l'intermédiaire d'un lieur phosphodiester, phosphorothioate ou phosphoramidate, ledit nucléoside étant facultativement protégé à la position 5' ou lié à un ou plusieurs autres nucléosides par l'intermédiaire d'un lieur internucléoside.

13. Composé de la revendication 11, dans lequel R⁴ est H.

14. Composé de la revendication 11, dans lequel R⁴ est un nucléoside lié par l'intermédiaire d'un lieur phosphodiester, phosphorothioate ou phosphoramidate, ledit nucléoside étant facultativement protégé avec un groupe protecteur.

15. Composé de la revendication 11, dans lequel R⁴ est un nucléoside lié par l'intermédiaire d'un lieur phosphodiester, phosphorothioate ou phosphoramidate, ledit nucléoside ayant un groupe 5'-OH libre.

16. Composé de la revendication 11, dans lequel R⁴ est un nucléoside 5'-protégé lié par l'intermédiaire d'un lieur phosphodiester, phosphorothioate ou phosphoramidate.

17. Composé de la revendication 11, dans lequel R⁴ est un nucléoside lié par l'intermédiaire d'un lieur phosphodiester, phosphorothioate ou phosphoramidate, ledit nucléoside étant relié à un ou plusieurs autres nucléosides par l'intermédiaire d'un lieur internucléoside adapté.

18. Composé de la revendication 1 ayant la formule : 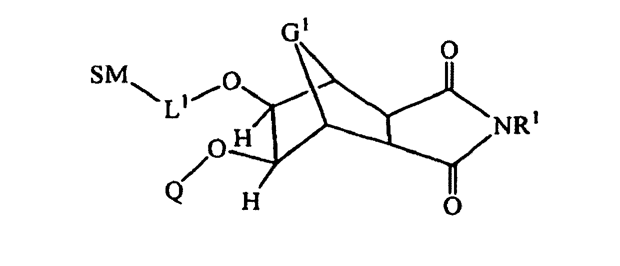 dans laquelle :
SM est un milieu de support ;
L¹ est un fragment de liaison ;
G¹ est 0, S, NR² ;
R² est autre que H ;
Q est H ou pg ;
pg est un groupe protecteur ; et
R¹ est un groupe de blocage.

19. Composé de la revendication 18 dans lequel SM est un matériau de support solide.

20. Composé de l'une quelconque des revendications 18 ou 19 dans lequel L¹ est C(=O)-(CH₂)₂-C(=O).

21. Composé de l'une quelconque des revendications 18 à 20 dans lequel G¹ est O.

22. Composé de l'une quelconque des revendications 18 à 21 dans lequel Q est H.

23. Composé de l'une quelconque des revendications 18 à 21 dans lequel Q est un groupe protecteur.

24. Composé de l'une quelconque des revendications 18 à 23 dans lequel G¹ est 0, et R¹ est un groupe alkyle, aryle, acyle, aralkyle, arylacyle, cycloalkyle, hétéroaryle hétéroarylacyle facultativement substitué plus avant.

25. Composé de l'une quelconque des revendications 18 à 24 dans lequel R¹ est aryle, ledit groupe aryle étant facultativement substitué par un ou plusieurs substituants choisis parmi F, Cl, Br, I, O-(alkyle en C₁-C₁₂), O-(acyle en C₁-C₁₂), NO₂, hétéroaryle, alkyle et acyle.

26. Composé de la revendication 25 dans lequel R¹ est phényle ou phényle substitué.

27. Composé de l'une quelconque des revendications 1 à 24 dans lequel R¹ est un groupe alkyle choisi parmi méthyle, éthyle, n-propyle, isopropyle, n-butyle, s-butyle, t-butyle, i-butyle, n-pentanyle, isooctanyle et dodécanyle, ledit groupe alkyle étant facultativement substitué par un ou plusieurs substituants choisis parmi F, Cl, Br, I, O-(alkyle en C₁-C₁₂), O-(acyle en C₁-C₁₂), NO₂, aryle et hétéroaryle.

28. Composé de l'une quelconque des revendications 1 à 7 dans lequel R¹ est un groupe acyle choisi parmi un groupe formyle, acétyle, propanoyle, butanoyle et octanoyle, ledit groupe acyle étant facultativement substitué par un ou plusieurs substituants choisis parmi F, Cl, Br, I, 0-(alkyle en C₁-C₁₂), NO₂, aryle ou hétéroaryle.

29. Composé de la revendication 1 ayant la formule :

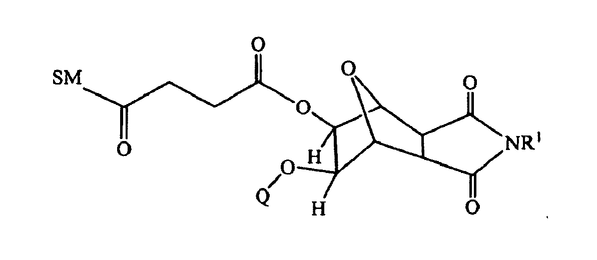

30. Procédé de fabrication du composé de la revendication 1 ayant la formule I comprenant la réaction d'un composé de formule II avec un composé de formule III : 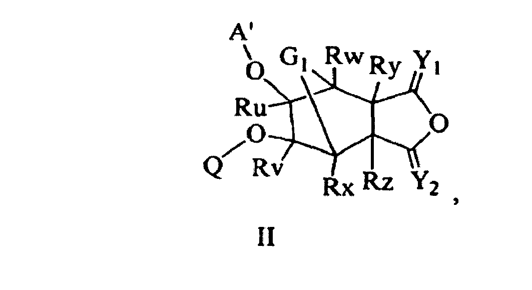 NH₂A" III.

31. Procédé de la revendication 30, comprenant en outre la synthèse d'un composé de formule II par réaction de Diels-Alder à partir de diénophile IV et de diène V : 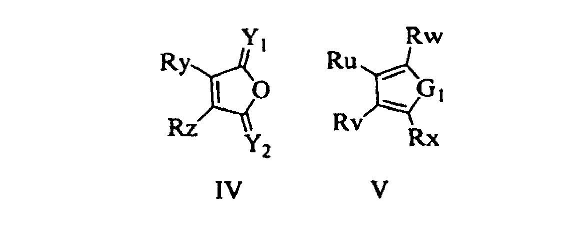 pour former l'intermédiaire A : 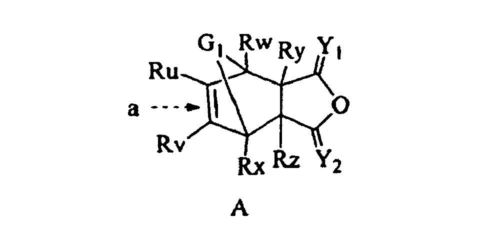 l'ajout de groupes hydroxyle de part et d'autre de la double liaison pour former l'intermédiaire B : 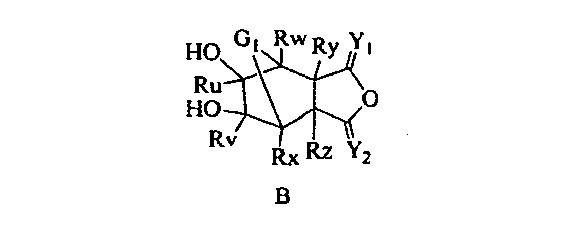 et la dérivation de l'intermédiaire B pour former un composé de formule II.

32. Procédé de fabrication du composé de la revendication 1 ayant la formule I comprenant la réaction du diénophile IVa avec le diène V pour former l'intermédiaire AA : 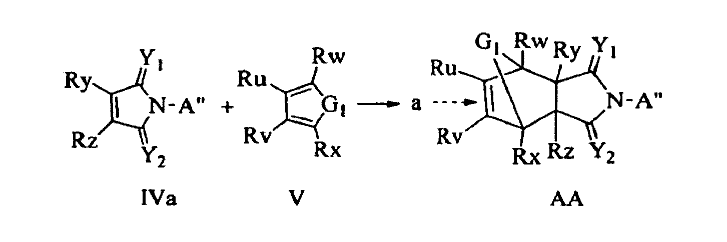 l'ajout de groupes hydroxyle de part et d'autre de la double liaison pour former l'intermédiaire BB : 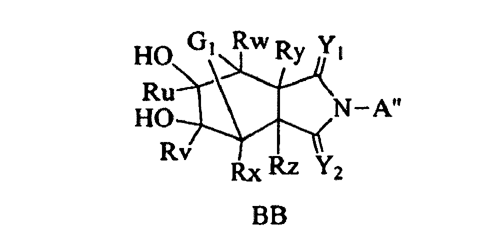 et la dérivation de l'intermédiaire BB pour former un composé de formule I.
